(19) Europäisches Patentamt
European Patent Office
Office européen des brevets

(11) EP 3 038 652 B1

(12) **EUROPEAN PATENT SPECIFICATION**

(45) Date of publication and mention of the grant of the patent:
**21.03.2018 Bulletin 2018/12**

(21) Application number: **14766301.7**

(22) Date of filing: **28.08.2014**

(51) Int Cl.:
*A61K 31/506* (2006.01) *A61K 31/519* (2006.01)
*A61K 45/06* (2006.01) *A61P 35/00* (2006.01)

(86) International application number:
**PCT/US2014/053244**

(87) International publication number:
**WO 2015/031666 (05.03.2015 Gazette 2015/09)**

(54) **COMBINATION OF AN ALK INHIBITOR AND A CDK INHIBITOR FOR THE TREATMENT OF CELL PROLIFERATIVE DISEASES**

KOMBINATION VON ALK- UND CDK-HEMMERN ZUR BEHANDLUNG ZELLPROLIFERATIVER ERKRANKUNGEN

COMBINAISON D'UN INHIBITEUR D'ALK ET D'UN INHIBITEUR DE CDK POUR LE TRAITEMENT DE MALADIES PROLIFÉRATIVES CELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.08.2013 US 201361871275 P**

(43) Date of publication of application:
**06.07.2016 Bulletin 2016/27**

(73) Proprietor: **Novartis AG**
**4056 Basel (CH)**

(72) Inventors:
- **HARRIS, Jennifer Leslie**
  **San Diego, California 92121 (US)**
- **LI, Nanxin**
  **San Diego, California 92121 (US)**
- **SMITH, Timothy R.**
  **San Diego, California 92121 (US)**

(74) Representative: **Kristl, Jernej et al**
**Novartis Pharma AG**
**IP Patent Department**
**Forum 1**
**Novartis Campus**
**4056 Basel (CH)**

(56) References cited:
**WO-A1-2010/020675 WO-A1-2012/106540**
**WO-A2-2008/073687**

- **P. BONVINI ET AL: "The effect of the cyclin-dependent kinase inhibitor flavopiridol on anaplastic large cell lymphoma cells and relationship with NPM-ALK kinase expression and activity", HAEMATOLOGICA, vol. 94, no. 7, 16 June 2009 (2009-06-16), pages 944-955, XP055144707, ISSN: 0390-6078, DOI: 10.3324/haematol.2008.004861**
- **THOMAS H. MARSILJE ET AL: "Synthesis, Structure-Activity Relationships, and in Vivo Efficacy of the Novel Potent and Selective Anaplastic Lymphoma Kinase (ALK) Inhibitor 5-Chloro- N 2-(2-isopropoxy-5-methyl-4-(piperidin-4-yl )phenyl)- N 4-(2-(isopropylsulfonyl)phenyl)pyrimidine-2,4-diamine (LDK378) Currently in Phase 1 and Pha", JOURNAL OF MEDICINAL CHEMISTRY, vol. 56, no. 14, 25 July 2013 (2013-07-25) , pages 5675-5690, XP055145299, ISSN: 0022-2623, DOI: 10.1021/jm400402q**
- **ROBERT ROSKOSKI: "Anaplastic lymphoma kinase (ALK): Structure, oncogenic activation, and pharmacological inhibition", PHARMACOLOGICAL RESEARCH, vol. 68, no. 1, 1 February 2013 (2013-02-01), pages 68-94, XP055145296, ISSN: 1043-6618, DOI: 10.1016/j.phrs.2012.11.007**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 038 652 B1

- BENGT HALLBERG ET AL: "Mechanistic insight into ALK receptor tyrosine kinase in human cancer biology", NATURE REVIEWS CANCER, vol. 13, no. 10, 24 September 2013 (2013-09-24), pages 685-700, XP55086717, ISSN: 1474-175X, DOI: 10.1038/nrc3580

## Description

### FIELD OF THE INVENTION

The present invention relates to a pharmaceutical combination comprising an ALK inhibitor and a CDK inhibitor, as defined in the claims, for use in the treatment of neuroblastoma.

### BACKGROUND OF THE INVENTION

ALK inhibitors

Anaplastic lymphoma kinase (ALK) is a member of the insulin receptor superfamily of receptor tyrosine kinases. This protein comprises an extracellular domain, a hydrophobic stretch corresponding to a single pass transmembrane region, and an intracellular kinase domain. It plays an important role in the development of the brain and exerts its effects on specific neurons in the nervous system, and is normally expressed in the developing nervous tissue. Genetic alterations of ALK have been implicated in oncogenesis in hematopoietic and non-hematopoietic tumors. The gene has been found to be rearranged, mutated, or amplified in a series of tumours including anaplastic large cell lymphomas, neuroblastoma, and non-small cell lung cancer. The aberrant expression of full-length ALK receptor proteins has been reported in neuroblastomas and glioblastomas; and ALK fusion proteins have occurred in anaplastic large cell lymphoma. While the chromosomal rearrangements are the most common genetic alterations in the ALK gene, ALK amplification has been shown in breast cancers and oesophageal cancers. The development of compounds that selectively target ALK in the treatment of ALK-positive tumors is therefore potentially highly desirable. A few small-molecule inhibitors of ALK kinase activity have been described in the recent years, e. g., in WO 2008/073687 A1; some of which are currently undergoing clinical evaluation. Crizotinib, a tyrosine kinase inhibitor of cMET and ALK has been approved for patients with ALK-positive advanced non-small cell lung cancer; more potent ALK inhibitors might shortly follow.

CDK inhibitors

The cyclin-dependent kinases (CDK) is a large family of protein kinases. CDKs regulate initiation, progression, and completion of the mammalian cell cycle. The function of CDKs is to phosphorylate and thus activate or deactivate certain proteins, including e.g. retinoblastoma proteins, lamins, histone H1, and components of the mitotic spindle. The catalytic step mediated by CDKs involves a phospho-transfer reaction from ATP to the macromolecular enzyme substrate.

Tumor development is closely associated with genetic alteration and deregulation of CDKs and their regulators, suggesting that inhibitors of CDKs may be useful anti-cancer therapeutics. Indeed, early results suggest that transformed and normal cells differ in their requirement for, e.g., cyclin D/CDK4/6 and that it may be possible to develop novel antineoplastic agents devoid of the general host toxicity observed with conventional cytotoxic and cytostatic drugs. Several groups of compounds (reviewed in e.g. Fischer, P. M. Curr. Opin. Drug Discovery Dev. 2001, 4, 623-634) have been found to possess anti-proliferative properties by virtue of CDK-specific ATP antagonism. The development of monotherapies for the treatment of proliferative disorders, such as cancers, using therapeutics targeted generically at CDKs, or at specific CDKs, is therefore potentially highly desirable. Inhibitors of CDKs are known and patent applications have been filed on such inhibitors; for examples, WO2007/140222, WO2010/020675, and WO2011/101409.

Bonvini et al. disclose in Haematologica, 2009, 84(7), 944-955 that the combination of the CDK inhibitor flavopiridol and the ALK small molecule inhibitor WHI-154 induces rapid apoptosis in anaplastic large cell lymphoma cells (ALCL).

Attempts have been made to prepare compounds that inhibit either the ALK or CDK4/6, and a number of such compounds have been disclosed in the art. However, in view of the number of pathological responses that are mediated by ALK and CDK4/6, there remains a continuing need for effective and safe therapeutic agents and a need for their preferential use in combination therapy. Surprisingly, it has been found that an ALK inhibitor provokes strong anti-proliferative activity and an *in vivo* antitumor response in combination with a CDK 4/6 inhibitor. The present invention is related to specific combinations therapy for treatment of proliferative diseases, which are neuroblastomas.

### SUMMARY OF THE INVENTION

The present disclosure relates to a pharmaceutical combination, separately or together, comprising (1) a first agent which is an ALK inhibitor or a pharmaceutically acceptable salt thereof, and (2) a second agent which is a CDK inhibitor or a pharmaceutically acceptable salt thereof. The disclosure also relates to this pharmaceutical combination for use in treating a proliferative disease.

In one aspect, the present invention relates to a pharmaceutical combination, separately or together, comprising

(1) a first agent which is an ALK inhibitor which is Compound A1, or a pharmaceutically acceptable salt thereof; or Compound A2, or a pharmaceutically acceptable salt thereof, and (2) a second agent which is a CDK inhibitor which is Compound B, or a pharmaceutically acceptable salt thereof, wherein Compound A1, Compound A2 and Compound B are as defined in the claims, for use in the treatment of neuroblastoma.

In a second aspect, the invention relates to a pharmaceutical composition comprising the pharmaceutical combination of the first aspect and at least one excipient for use in the treatment of neuroblastoma.

The present invention also provides further embodiments as described in the claims.

The disclosure also relates to a method of treating a proliferative disease, which method comprises administering to a patient in need thereof, a therapeutically effective amount of a first agent which is an ALK inhibitor and a therapeutically effective amount of a second agent which is a CDK inhibitor, wherein the first and the second agent are administered simultaneously, separately or sequentially.

## DETAILED DESCRIPTION OF THE FIGURES

Figure 1 illustrates with hypothetical data how potential synergistic interactions in compound combinations can be assessed from the output of CHALICE software based on the Loewe Additivity model. The figure on the left is a Dose Matrix plot, where each individual block of the 7x7 matrix reports the percent of inhibition (cell death) by the drug treatment. The inhibition by the single compound treatment alone is reported in the far left hand column for Agent A, and the bottom row for Agent B; the data is normalized to inhibition by the vehicle control, which is set to 0 (the value where both Agent A and Agent B concentrations are 0). The figure on the right is a Loewe Excess Matrix plot, where each individual block reports the excess inhibition comparing the experimental data in the dose matrix to an expected inhibition value generated by the Loewe additivity model. In this view, synergy is defined as values >0, additive is defined as values = 0 and antagonism is defined as values <0. The highlighted blocks identify combinations that synergy is observed in the experimental data.

Figure 2 shows the CHALICE matrix plots demonstrating the dose effects (% inhibition) from co-treatment with Compound A1 and Compound B (top row), Compound A1 self-cross (middle row) and Compound B self-cross (bottom row) on the inhibition of LAN-1 human neuroblastoma cells.

Figure 3 shows a boxplot providing a visual summary of the synergy scores of drug combinations in 15 disease (ALK mutant) and normal (wide type) neuroblastoma cell lines (see data in Tables 2 and 3). In this plot, synergy scores generated from the ALK inhibitors, Compounds A1 and A2, but not A3, were included. As used herein, an ALK inhibitor refers to Compound A1 or Compound A2, and the CDK4/6 inhibitor refers to Compound B. Each box represents the range of synergy scores of a particular treatment regimen (ALK x CDK, ALK self-cross, or CDK self-cross); the horizontal white line within the box represents the group median and the vertical solid line represents the group standard deviation. The solid circles represent outliers.

Figures 4A, 4B and 4C show scatter plots which provide visual identification of "hit" synergistic combinations. Maximum combination efficacy values were plotted against synergy scores of combinations of an ALK inhibitor and a CDK4/6 inhibitor, ALK inhibitors self-crosses, and CDK4/6 inhibitor self-cross in 15 disease (ALK mutant) and normal (wide type) neuroblastoma cell lines (see data in Tables 2 and 3). In these plots, only data generated with Compounds A1 and A2 were plotted; that is, as used herein, the ALK inhibitor refers to Compound A1 or Compound A2, and the CDK4/6 inhibitor refers to Compound B. Figure 4A is a scatter plot of data from the self-cross of the two ALK inhibitors, Compounds A1 and A2 in the 15 cell lines. The plot shows preferential single agent efficacy for the ALK Disease. Figure 4B is the scatter plot of data from the self-cross of the CDK inhibitor, Compound B. The plot shows minimal single agent efficacy or synergy. Figure 4C is the scatter plot of data from combinations of either one the ALK inhibitors, Compounds A1 and A2, with the CDK inhibitor, Compound B. The plot shows interaction leading both to synergy and increased efficacy in four Disease (Lan-5, Kelly, Lan-1, NB-1643, and two Normal (NB-1, SK-N-BE) cell lines.

Figures 5A, 5B, 5C, and 5D show typical examples of drug combination plots and their interpretations based on the Chou and Talalay combination index theorem. Figure 5A is a Fa-CI plot for constant combination ratio. CI as defined by Chou according to the following equation: $CI = (D)_1/(D_X)_1 + (D)_2/(D_x)_2$ where $(D_x)_1$ and $(D_x)_2$ are the concentrations of compounds $D_1$ and $D_2$ needed to produce a given level of anti-proliferative effect when used individually, whereas $(D)_1$ and $(D)_2$ are their concentrations that produce the same anti-proliferative effect when used in combination. The combination index is a quantitative measure of drug interaction defined as an additive effect (CI =1), antagonism (CI >1), or synergy (CI <1). $F_a$ means fraction affected is defined as the fraction of cells affected by the given concentration of compounds alone or in combination. $F_a = 0$ is determined based on DMSO control by the dose, and $F_a = 1$ is a full response (no viable cells left). Typically, a Fa-CI plot, as used herein, is used to assess synergy. Figure 5B is a classic isobolograms at $ED_{50}$, $ED_{75}$, and $ED_{90}$. $(D)_1$ and $(D)_2$ mean concentration of drug 1 and drug

2, respectively. $ED_x$ means dose at X% effect, 100% effect means no viable cells left. Figure 5C is a normalized isobologram for combination at different ratios. The terms are as defined in Figure 5B. Figure 5D is a Fa-DRI plot (Chou and Chou, 1988; Chou and Martin, 2005) where DRI means dose-reduction index and is related to CI according to the following equation: $CI = (D)_1/(D_x)_1 + (D)_2/(D_x)_2 = 1/(DRI)_1 + 1/(DRI)_2$. DRI estimates how much the dose of each drug can be reduced when synergistic drugs are given in combination, while still achieving the same effect size as each drug administered individually

Figures 6A, 6B, 6C, 6D, 6E and 6F show the drug combination plots for the combinations of Compounds A1 and B, Compound A and Compound B in NB-1643 cells (Disease): (6A) Median-effect plot; (6B) dose-effect curves; classic isobologram at $ED_{50}$, $ED_{75}$, and $ED_{90}$; (6C) Fa-CI plot; (6D) Fa-logCI plot; (6E) classical isobologram; and (6F) conservative isobologram. The plots jointly demonstrating the combination was synergistic across the tested concentration range.

Figures 7A, 7B, 7C, 7D, 7E and 7F show the drug combination plots for the combinations of Compounds A1 and B, Compound A1 alone and Compound B alone in SH-SY5Y (Disease) cells: (7A) Median-effect plot; (7B) dose-effect curves; (7C) Fa-CI plot; (7D) Fa-log(CI) plot; (7E) classic isobologram at ED50, ED75, and ED90; and (7F) conservative isobologram. Figures 7C to 7F show that the combination was moderate synergistic at low dose and additive or slightly antagonistic at high dose.

Figures 8A, 8B, 8C, 8D, 8E and 8F show the drug combination plots for the combinations of Compounds A1 and B, Compound A, and Compound B in NB1691 (Normal) cells: (7A) Median-effect plot; (7B) dose-effect curves; (7C) Fa-CI plot; (7D) Fa-log(CI) plot; (7E) classic isobologram at $ED_{50}$, $ED_{75}$, and $ED_{90}$; and (7F) conservative isobologram. Figures 8C to 8F demonstrate that the combination was strongly synergetic at low dose and additive at higher doses, and antagonistic at high Compound A1 doses.

Figures 9A, 9B, 9C, 9D, 9E and 9F show the drug combination plots for the combinations of Compounds A1 and B, Compound A and Compound B in EDC1 (Normal)cells: (9A) Median-effect plot; (9B) dose-effect curves; (9C) Fa-CI plot; (9D) Fa-log(CI) plot; (9E) classic isobologram at ED50, ED75, and ED90; and (9F) conservative isobologram. Figures 9C to 9F demonstrate that the combination was synergetic across the tested concentration range.

Figures 10A, 10B, 10C and 10D show the morphology of SH-SY5Y cells in response to treatments by Compound A1, B1 or the combination of Compounds A1 and B, each at IC 50 of the respective compounds and 72 hours post treatment: (a) vehicle; (b) treated with Compound A1 alone; (c) treated with Compound B alone, and (d) treated with combination of Compounds A1 and B.

Figures 11A, 11B and 11C compare cell viability with apoptosis of NB1643 cells, analyzed by ApoTox-GloTM triplex assay, at 72 hours post treatment with: (a) Compound A1 alone; (b) Compound B alone, and (c) combination of Compounds A1 and B combined at equipotent ratio (0, ¼. ½, 1, 2, and 4 times the IC50 of each of the compounds). The results show that drug treatment enhances cell death, but same level of apoptosis is observed with Compound A1 alone as with the combination with Compound B.

Figures 12A, 12B and 12C show viability of NB1643 cells, analyzed by CTG assay, at 72 hours post treatment with: (a) Compound A1 alone; (b) Compound B alone, and (c) combination of Compounds A and B combined at equipotent ratio. The results confirm that combination treatment enhances cell death.

Figures 13A, 13B and 13C compare cell viability with apoptosis of SH-SY5Y cells, analyzed by ApoTox-GloTM triplex assay, at 72 hours post treatment with: (a) Compound A1 alone; (b) Compound B alone, and (c) combination of Compounds A1 and B combined at the equipotent ratio (0, 1/4. 1/2, 1, 2, and 4 times the IC50 of each of the compounds). The results show co-treatment enhances cell death. The cell were dying earlier at the highest concentration, such that the apoptosis was not detectable at those concentrations.

Figures 14A, 14B and 14C compare cell viability with apoptosis of EBC1 cells, analyzed by ApoTox-GloTM triplex assay, at 72 hours post treatment with: (a) Compound A1 alone; (b) Compound B alone, and (c) combination of Compounds A1 and B combined at equipotent ratio (0, 1/4. 1/2, 1, 2, and 4 times the IC50 of each of the compounds). The data show little or no enhancement of cell death or apoptosis with co-treatment.

Figure 15 shows the Western blot of total and pALK expression in NB1643 cells at 20 hour post treatment with: vehicle; Compound A1 at 1/16, 1/8, 1/4 and 4 times the IC50 dose; Compound B at 1/16, 1/8, ¼ and 4 times the IC50 dose; and combination of Compounds A1 and B at 1/16, 1/8, 1/4 and 4 times the IC50 dose of each of the compounds. The result shows co-treatment greatly reduces pALK protein expression in NB1643 cells starting at 1/16x of IC50 doses

Figure 16 shows the Western blot of total Rb, phospho-Rb S780, and phospho-Rb S795, expression in NB1643 cells at 20 hour post treatment with: vehicle; Compound A1 at 1/16, 1/8, 1/4 and 4 times the IC50 dose; Compound B at 1/16, 1/8, 1/4 and 4 times the IC50 dose; and combination of Compounds A1 and B at 1/16, 1/8, 1/4 and 4 times the IC50 dose of each of the compounds. The result shows co-treatment reduces pRb expression in NB1643 cells starting at 1/16x of IC50 doses. The combination is more effective in reducing pRb S780 expression than pRb S795 expression.

Figure 17 shows the Western blot of ALK, pALK, total Rb, and phospho-Rb S795, expression in NBEBC1 cells at

20 hour post treatment with: vehicle; Compound A1 at 1/4, 1/2, 1 and 4 times the IC50 dose; Compound B at 1/4, 1/2, 1 and 4 times the IC50 dose; and combination of Compounds A1 and B at 1/4, 1/2, 1 and 4 times the IC50 dose of each of the compounds. The results show co-treatment is more effective in reducing pALK and pRb protein expression.

Figure 18 shows the relative tumor volume of human neuroblastoma SH-SY5Y xenografts in CB17 SCID mice with time for treatment groups (1) vehicle control, (2) Compound A1 at 50 mg/kg, (3) Compound B at 187.5 to 250 mg/kg, and (4) combination of Compound A1 at 50 mg/kg and Compound B at 187.5 to 250 mg/kg. The dose for Compound B started at 250 mg/kg and was reduced to 187.5 mg/kg at day 5. The results shows treatment with Compound A1 alone resulted in only a slight tumor growth delay compared to vehicle control. Treatment with Compound B alone resulted slower tumor growth. Co-treatment effectively shrunk the existing tumor and achieved total tumor remission.

Figures 19A, 19B, 19C and 19D show the variability of tumor volume with the duration of treatment (in weeks) for individual mice in each of the treatment groups described in Figure 18 above.

Figure 20 shows the survival of the mice (in percentage) versus the duration of treatment (in weeks) in each of the treatment groups described in Figure 18 above. On day 7, two of the mice from Group 4 died, and on day 14, one mouse from the Compound B group died. The mice in the Control group and the Compound A1 group were euthanized due to the size of their tumor.

Figure 21 are scatter plots of combinatorial drug effects (efficacy vs synergy score) from combinations of Compound A1 and Compound B, and their respective self-crosses in 16 Disease (ALK mutant) and Normal (wide-type) neuroblastoma cell lines (see data in Table 10). Synergistic combination hits were identified as having both a synergy score >2 and a maximum efficacy >100 (see Figures 4A, 4B and 4C for interpretation). The plot at top is the self-cross of Compound A1 (an ALK inhibitor) which shows preferential single agent efficacy for the ALK Disease. The plot in the middle is the self-cross of Compound B (a CDK inhibitor) which shows minimal single agent efficacy or synergy. The plot at the bottom is the combinations of Compounds A1 and B, which shows interaction leading both to synergy and increased efficacy in two Disease (NB-1691, Lan-5) and one Normal (NB-1691) cell lines.

Figures 22A, 22B show the dose effects of co-treatment with an ALK inhibitor and a CDK4/6 inhibitor on the proliferation of Kelly human neuroblastoma cells. Figure 22A shows the dose matrix and isobologram demonstrating the dose effects of co-treatment with Compound A1 (an ALK inhibitor) and Compound B (a CDK4/6 inhibitor). The combination was moderately synergistic with a synergy score of 1.75 and the isobologram indicated a very strong interaction. Figure 22B shows the dose matrix and isobologram demonstrating the dose effects of co-treatment with Compound A2 (an ALK inhibitor) and Compound B. The combination was moderately synergistic with a synergy score of 1.48 and the isobologram indicated a very strong interaction.

Figures 23A, 23B, 23C and 23D show the dose effect of co-treatment with an ALK inhibitor and a CDK4/6 inhibitor on the proliferation of Kelly and NB-1 neuroblastoma cells. Figure 23A shows the dose matrix and Loewe excess matrix demonstrating the dose effects of co-treatment with Compound A1 (an ALK inhibitor) and Compound B (a CDK4/6 inhibitor) in Kelly cells. The combination was synergistic with a calculated synergy score of 2.51. Figure 23B shows the dose matrix and Loewe excess matrix demonstrating the dose effects of co-treatment with Compound A2 (an ALK inhibitor) and Compound B on Kelly cells. The combination was synergistic with a synergy score of 2.29. Figure 23C shows the dose matrix and Loewe excess matrix demonstrating the dose effects of co-treatment with Compound A1 and Compound B on the proliferation of NB-1 human neuroblastoma cells. The combination was not synergistic. Figure 23D shows the dose matrix and Loewe excess matrix demonstrating the dose effects of co-treatment with Compound A2 and Compound B on the proliferation of human NB-1 neuroblastoma cells. The combination was not synergistic.

Figures 24A, 24B, 24C, 24D, 24E and 24F show the dose effects of co-treatment with an ALK inhibitor and a CDK4/6 inhibitor in Kelly, NB-1 and SH-SY5Y neuroblastoma cells. Figure 24A shows the dose matrix and Loewe excess matrix demonstrating the dose effects of co-treatment with Compound A1 (an ALK inhibitor) and Compound B on the proliferation of Kelly cells; the synergy score was 0.820. Figure 24B shows the dose matrix and Loewe excess matrix demonstrating the dose effect of co-treatment with Compound A2 (an ALK inhibitor) and Compound B in Kelly human neuroblastoma cells; the synergy score was 1.52. Figure 24C shows the dose matrix and Loewe excess matrix demonstrating the dose effects of co-treatment with Compound A1 and Compound B in NB-1 human neuroblastoma cells; the combination is not synergistic. Figure 24D shows the dose matrix and Loewe excess matrix demonstrating the dose effect of co-treatment with Compound A2 and Compound B in NB-1 human neuroblastoma cells; the combination is not synergistic. Figure 24E shows the dose matrix and Loewe excess matrix demonstrating the dose effect of co-treatment with Compound A1 and Compound B in SH-SY5Y human neuroblastoma cells. Figure 24F shows the dose matrix and Loewe excess matrix demonstrating the dose effect of co-treatment with Compounds A2 and B in SH-SY5Y human neuroblastoma cells.

## DETAILED DESCRIPTION OF THE INVENTION

Definition

The following general definitions are provided to better understand the invention:

The terms "about" or "approximately" usually means within 20%, more preferably within 10%, and most preferably still within 5% of a given value or range. Alternatively, especially in biological systems, the term "about" means within about a log (*i.e.,* an order of magnitude) preferably within a factor of two of a given value.

"Anaplastic lymphoma kinase (ALK) inhibitors" used herein relates to compounds which inhibit the kinase activity of the enzyme. Such compounds will be referred to as "ALK inhibitors".

"ALK resistant tumor or cancer" refers to a cancer or tumor that either fails to respond favorably to treatment with prior ALK inhibitors, or alternatively, recurs or relapses after responding favorably to ALK inhibitors. The cancer or tumor may be resistant or refractory at the beginning of treatment or it may become resistant or refractory during treatment.

"Co-administer", "co-administration" or "combined administration" or the like are meant to encompass administration of the selected therapeutic agents to a single patient, and are intended to include treatment regimens in which the agents are not necessarily administered by the same route of administration or at the same time.

"Combination" refers to either a fixed combination in one dosage unit form, or a non-fixed combination (or kit of parts) for the combined administration where a compound and a combination partner (e.g. another drug as explained below, also referred to as "therapeutic agent" , "agent" or "co-agent") may be administered independently at the same time or separately within time intervals, especially where these time intervals allow that the combination partners show a cooperative, e.g. synergistic effect. The term "combined administration" or the like as utilized herein are meant to encompass administration of the selected combination partner to a single subject in need thereof (e.g. a patient), and are intended to include treatment regimens in which the agents are not necessarily administered by the same route of administration or at the same time. The term "fixed combination" means that the active ingredients, e.g. a compound of formula A1 and a combination partner, are both administered to a patient simultaneously in the form of a single entity or dosage. The terms "non-fixed combination" or "kit of parts" mean that the active ingredients, e.g. a compound of formula A1 and a combination partner, are both administered to a patient as separate entities either simultaneously, concurrently or sequentially with no specific time limits, wherein such administration provides therapeutically effective levels of the two compounds in the body of the patient.

"Cyclin dependent kinase (CDK) inhibitor" as defined herein refers to a small molecule that interacts with a cyclin-CDK complex to block kinase activity.

"Dose range" refers to an upper and a lower limit of an acceptable variation of the amount of therapeutic agent specified. Typically, a dose of the agent in any amount within the specified range can be administered to patients undergoing treatment.

"Jointly therapeutically effective amount" in reference to combination therapy means that amount of each of the combination partners, which may be administered, together, independently at the same time or separately within appropriate time intervals that the combination partners exert cooperatively, beneficial/ therapeutic effects in alleviating, delaying progression of or inhibiting the symptoms of a disease in a patient in need thereof.

"Pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

"Pharmaceutical preparation" or "pharmaceutical composition" refers to a mixture or solution containing at least one therapeutic agent to be administered to a warm-blooded mammal, e.g., a human in order to prevent, treat or control a particular disease or condition affecting the mammal.

"Salts" (which, what is meant by "or salts thereof" or "or a salt thereof"), can be present alone or in mixture with free compound, e.g. the compound of the formula (I), and are preferably pharmaceutically acceptable salts. Such salts of the compounds of formula (I) are formed, for example, as acid addition salts, preferably with organic or inorganic acids, from compounds of formula (I) with a basic nitrogen atom. Suitable inorganic acids are, for example, halogen acids, such as hydrochloric acid, sulfuric acid, or phosphoric acid. Suitable organic acids are, e.g., carboxylic acids or sulfonic acids, such as fumaric acid or methanesulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred. In view of the close relationship between the novel compounds in free form and those in the form of their salts, including those salts that can be used as intermediates, for example in the purification or identification of the novel compounds, any reference to the free compounds hereinbefore and

hereinafter is to be understood as referring also to the corresponding salts, as appropriate and expedient. The salts of compounds of formula (I) are preferably pharmaceutically acceptable salts; suitable counter-ions forming pharmaceutically acceptable salts are known in the field.

"Single pharmaceutical composition" refers to a single carrier or vehicle formulated to deliver effective amounts of both therapeutic agents to a patient. The single vehicle is designed to deliver an effective amount of each of the agents, along with any pharmaceutically acceptable carriers or excipients. In some embodiments, the vehicle is a tablet, capsule, pill, or a patch. In other embodiments, the vehicle is a solution or a suspension.

"Subject", "patient", or "warm-blooded animal" is intended to include animals. Examples of subjects include mammals, e.g., humans, dogs, cows, horses, pigs, sheep, goats, cats, mice, rabbits, rats, and transgenic non-human animals. In certain embodiments, the subject is a human, e.g., a human suffering from, at risk of suffering from, or potentially capable of suffering from a brain tumor disease. Particularly preferred, the subject or warm-blooded animal is human.

"Therapeutically effective" preferably relates to an amount of a therapeutic agent that is therapeutically or in a broader sense also prophylactically effective against the progression of a proliferative disease.

"Treatment" includes prophylactic and therapeutic treatment (including but not limited to palliative, curing, symptom-alleviating, symptom-reducing) as well as the delay of progression of a cancer disease or disorder. The term "prophylactic" means the prevention of the onset or recurrence of a cancer. The term "delay of progression" as used herein means administration of the combination to patients being in a pre-stage or in an early phase of the cancer to be treated, a pre-form of the corresponding cancer is diagnosed and/or in a patient diagnosed with a condition under which it is likely that a corresponding cancer will develop.

Description of the Preferred Embodiments

The present invention relates to a pharmaceutical combination comprising, separately or together, (a) a first agent which is an anaplastic lymphoma kinase (ALK) inhibitor which is Compound A1 (or a pharmaceutically acceptable salt thereof), or Compound A2 (or a pharmaceutically acceptable salt thereof) and (b) a second agent which is a cyclin-dependent kinase (CDK) inhibitor which is compound B, or a pharmaceutically acceptable salt thereof, for use in the treatment of neuroblastoma. Such combination may be for simultaneous, separate or sequential use for the treatment of neuroblastoma.

In one preferred embodiment of the combination of the invention, the ALK inhibitor is Compound A1, which is 5-chloro-N2-(2-isopropoxy-5-methyl-4-(piperidin-4-yl)phenyl)-N4-[2-(propane-2-sulfonyl)-phenyl]-pyrimidine-2,4-diamine, below:

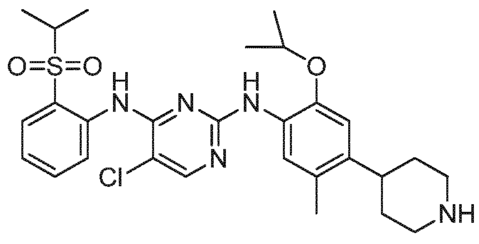

(A1),

or pharmaceutically acceptable salts thereof.

In another preferred embodiment, the ALK inhibitor is Compound A2 which is N6-(2-isopropoxy-5-methyl-4-(1-methylpiperidin-4-yl)phenyl)-N4-(2-(isopropylsulfonyl)phenyl)-1H-pyrazolo[3,4-d]pyrimidine-4,6-diamine, below:

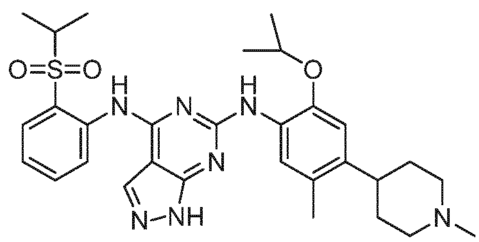

(A2),

or pharmaceutically acceptable salts thereof.

In another aspect of the present disclosure, the ALK inhibitor is Compound A3 which is (R)-3-(1-(2,6-dichloro-3-fluorophenyl)ethoxy)-5-(1-(piperidin-4-yl)-1H-pyrazol-4-yl)pyridin-2-amine, commonly known as Crizotinib and trade name XALKORI®) below:

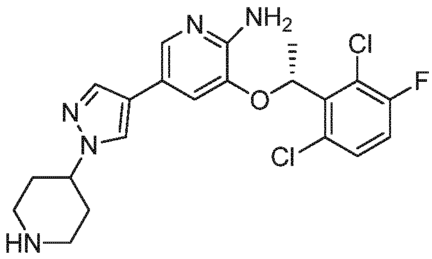

(A3)

or a pharmaceutically acceptable salt thereof.

In one aspect of the combination of the disclosure, the CDK inhibitor is a CDK4 or a CDK6 inhibitor. In one variation, the CDK inhibitor is a CDK4 inhibitor. In another variation, the CDK inhibitor is a CDK6 inhibitor. In still another variation, the CDK inhibitor is a CDK4 and CDK6 dual inhibitor.

In one embodiment, the CDK inhibitor is Compound B1 which is 7-cyclopentyl-N,N-dimethyl-2-((5-(piperazin-1-yl)pyridin-2-yl)amino)-7H-pyrrolo[2,3-d]pyrimidine-6-carboxamide, described by the formula below:

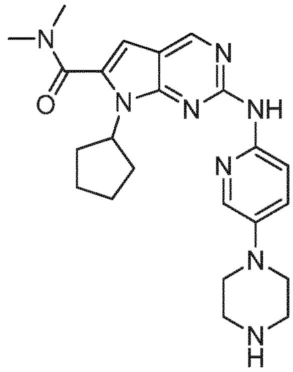

(B1).

Specific embodiments of the pharmaceutical combinations of the present invention include the following:

(1) Combination comprising Compound A1 and Compound B1;
(2) Combination comprising Compound A2 and Compound B1.

Also described is the combination of more than two separate active ingredients as set forth above, i.e., a pharmaceutical combination within the scope of this invention could include three active ingredients or more.

Unless indicated otherwise, if there is a discrepancy between the structure of a compound and its chemical name, the structure of the compound prevails.

The compounds A1, A2, and B, may be incorporated in the combination of the present invention in either the form of its free base or any salt thereof. Salts can be present alone or in mixture with free compound, and are preferably pharmaceutically acceptable salts. Such salts of the compounds are formed, for example, as acid addition salts, preferably with organic or inorganic acids, with a basic nitrogen atom. Suitable inorganic acids are, for example, halogen acids, such as hydrochloric acid, sulfuric acid, or phosphoric acid. Suitable organic acids are, e.g., succinic acid, carboxylic acids or sulfonic acids, such as fumaric acid or methansulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

Comprised are likewise the pharmaceutically acceptable salts thereof, the corresponding racemates, diastereoisomers, enantiomers, tautomers, as well as the corresponding crystal modifications of above disclosed compounds where present, e.g. solvates, hydrates and polymorphs, which are disclosed therein. The compounds used as active ingredients in the combinations of the present invention can be prepared and administered as described in the cited documents, respectively.

**Pharmacology and Utility**

It is noted that the individual partners of the combination of the present invention are compounds that are known

to have the inhibitory activity. It has now been surprisingly found that the combination(s) of the present invention and their pharmaceutically acceptable salts exhibit beneficial cooperative (e.g., synergistic) therapeutic properties when tested in vitro in cell-free kinase assays and in cellular assays, and in vivo in a cancer mouse model, and are therefore useful for the treatment of proliferative diseases, particularly cancers. The term "proliferative disease" includes, but not restricted to, cancer, tumor, hyperplasia, restenosis, cardiac hypertrophy, immune disorder and inflammation.

In one aspect, the present invention relates to a pharmaceutical combination comprising, separately or together, (a) a first agent which is an anaplastic lymphoma kinase (ALK) inhibitor which is (i) compound A1, or a pharmaceutically acceptable salt thereof, or (ii) compound A2, or a pharmaceutically acceptable salt thereof, and (b) a second agent which is a cyclin-dependent kinase (CDK) inhibitor which is compound (B) or a pharmaceutically acceptable salt thereof, for use in the treatment of neuroblastoma. In another aspect, the present disclosure provides the use of a pharmaceutical combination, separately or together, (a) a first agent which is an anaplastic lymphoma kinase (ALK) inhibitor or a pharmaceutically acceptable salt thereof and (b) a second agent which is a cyclin-dependent kinases (CDK) inhibitor or a pharmaceutically acceptable salt thereof, for the preparation of a medicament for the treatment of a proliferative disease, particularly cancer.

In one aspect, the present disclosure further relates to a method for treating a proliferative disease in a subject in need thereof, comprising administering to said subject a jointly therapeutically effective amount of a pharmaceutical combination or a pharmaceutical composition, comprising: (a) a first agent which is an anaplastic lymphoma kinase (ALK) inhibitor or a pharmaceutically acceptable salt thereof and (b) a second agent which is a cyclin-dependent kinases (CDK) inhibitor or a pharmaceutically acceptable salt thereof. In accordance with the present invention, the first agent and the second agent may be administered either together in a single pharmaceutical composition, independently in separate pharmaceutical compositions, or sequentially.

Preferably, the present invention is useful for treating a mammal, especially humans, suffering from neuroblastoma.

Examples of a proliferative disease the can be treated with the combination of the present disclosure are for instance cancers, including, but are not limited to, sarcoma, neutroblastoma, lymphomas, cancer of the lung, bronchus, prostate, breast (including sporadic breast cancers and sufferers of Cowden disease), pancreas, gastrointestine, colon, rectum, colon, colorectal adenoma, thyroid, liver, intrahepatic bile duct, hepatocellular, adrenal gland, stomach, gastric, glioma, glioblastoma, endometrial, melanoma, kidney, renal pelvis, urinary bladder, uterine corpus, cervix, vagina, ovary, multiple myeloma, esophagus, a leukemia, acute myelogenous leukemia, chronic myelogenous leukemia, lymphocytic leukemia, myeloid leukemia, brain, a carcinoma of the brain, oral cavity and pharynx, larynx, small intestine, non-Hodgkin lymphoma, melanoma, villous colon adenoma, a neoplasia, a neoplasia of epithelial character, a mammary carcinoma, basal cell carcinoma, anaplastic large cell lymphoma, non-small cell lung carcinoma, squamous cell carcinoma, actinic keratosis, tumor diseases (including solid tumors), a tumor of the neck or head, polycythemia vera, essential thrombocythemia, myelofibrosis with myeloid metaplasia, inflammatory breast cancer, and Waldenstroem disease.

Further examples include polycythemia vera, essential thrombocythemia, myelofibrosis with myeloid metaplasia, asthma, COPD, ARDS, Loffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma, eosinophil-related disorders affecting the airways occasioned by drug-reaction, psoriasis, contact dermatitis, atopic dermatitis, alopecia areata, erythema multiforme, dermatitis herpetiformis, scleroderma, vitiligo, hypersensitivity angiitis, urticaria, bullous pemphigoid, lupus erythematosus, pemphisus, epidermolysis bullosa acquisita, autoimmune haematogical disorders (e.g. haemolytic anaemia, aplastic anaemia, pure red cell anaemia and idiopathic thrombocytopenia), systemic lupus erythematosus, polychondritis, scleroderma, Wegener granulomatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary biliary cirrhosis, uveitis (anterior and posterior), interstitial lung fibrosis, psoriatic arthritis, glomerulonephritis, cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, and coronary artery disease, reperfusion injuries, retinopathy, such as diabetic retinopathy or hyperbaric oxygen-induced retinopathy, and conditions characterized by elevated intraocular pressure or secretion of ocular aqueous humor, such as glaucoma.

Where a cancer, a tumor, a tumor disease, sarcoma, or a cancer is mentioned, also metastasis in the original organ or tissue and/or in any other location are implied alternatively or in addition, whatever the location of the tumor and/or metastasis.

The combination of ALK and CDK4/6 inhibitors of the present invention is particularly useful for the treatment of ALK positive cancers, i.e. a cancer mediated by/depending on anaplastic lymphoma kinase (ALK). The data shown herein that combinations of the present invention are effective in treating cancer, particularly neuroblastoma, showing overexpression or amplification and/or somatic mutation of ALK gene and/or protein.

The combination of ALK and CDK4/6 inhibitors of the present invention may also be useful in treating ALK

resistant tumors or cancers. One mechanism for tumor resistance when treated with ALK inhibitors is for mutations to appear in the ALK gene. This mechanism has been demonstrated in a clinical trial in Crizotinib treated patients with ALK positive tumors (mostly non-small cell lung carcinoma). Some of these resistance mutations are similar to the mutations found in neuroblastoma. While not wished to be bound by theory, it is hypothesized that these resistance mutations lead to activation of ALK to further drive the proliferation of the tumor. For example, mutations in the T1151/L1152/C1156 area and the 11171/F1174 area of ALK are similar to neuroblastoma mutations. Since the combinations of ALK inhibitor and CDK inhibitor the present invention are effective in neuroblastoma tumors that have amplifying mutations, the combinations would be effective in these ALK resistant tumors.

Accordingly, in one embodiment, the combination of the present invention is useful in treating a proliferative disease that is dependent on the amplification of the ALK gene. In another embodiment, the combination of the present invention is useful in treating a proliferative disease that is dependent on a mutation of the ALK gene. In yet another embodiment, the combination of the present invention is useful in treating a proliferative disease that is dependent on a mutation and amplification of the ALK gene.

In one aspect of the disclosure, the cell proliferative disease is selected from lymphoma, osteosarcoma, melanoma, a tumor of breast, renal, prostate, colorectal, thyroid, ovarian, pancreatic, neuronal, lung, uterine or gastrointestinal tumor, ALK resistant tumor, inflammatory breast cancer, anaplastic large cell lymphoma, non-small cell lung carcinoma and neuroblastoma..

In a preferred aspect of the disclosure, the cell proliferative disease is anaplastic large cell lymphoma. In another preferred aspect of the disclosure, the cell proliferative disease is non-small cell lung carcinoma. In yet another preferred aspect of the disclosure, the cell proliferative disease is anaplastic large cell lymphoma. According to the invention, the cell proliferative disease is neuroblastoma. In a preferred embodiment, the cell proliferative disease is an ALK resistant tumor.

*In vitro* and *in vivo* studies demonstrated that the administration of a pharmaceutical combination of the invention resulted in a beneficial effect, e.g., a synergistic therapeutic effect, with regard to alleviating, delaying progression of or inhibiting the symptoms, compared with a monotherapy applying only one of agents (a) or agents (b) used in the combination of the invention. The benefit that small amounts of the active ingredients can be used, e.g., that the dosages may be smaller and/or administered less frequently, could diminish the incidence or severity of side effects, which may lead to an improved quality of life or a decreased morbidity. This is in accordance with the desires and requirements of the patients to be treated. The result of the *in vitro* and *in vivo* studies is reported in the Example section, *infra.*

To demonstrate that the combination of an ALK inhibitor and a CDK inhibitor of the present invention is particularly suitable for the effective treatment of proliferative diseases with good therapeutic margin and other advantages, clinical trials can be carried out in a manner known to the skilled person.

Suitable clinical studies are, e.g., open label, dose escalation studies in patients with proliferative diseases. Such studies prove in particular the synergism of the active ingredients of the combination of the invention. The beneficial effects can be determined directly through the results of these studies which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) is administered in a fixed dose and the dose of agent (b) is escalated. Each patient receives doses of the agent (a) either daily or intermittent. The efficacy of the treatment can be determined in such studies, e.g., after 12, 18 or 24 weeks by evaluation of symptom scores every 6 weeks.

### Pharmaceutical Composition, Administration and Dosage

It is one objective of this invention to provide a pharmaceutical composition comprising a quantity, which is jointly therapeutically effective at targeting or preventing proliferative diseases, of each combination partner agent (a) and (b) of the invention.

In one aspect, the present invention relates to a pharmaceutical composition which comprises a pharmaceutical combination comprising, separately or together, (a) a first agent which is an anaplastic lymphoma kinase (ALK) inhibitor selected from compound A1 or a pharmaceutically acceptable salt thereof, or compound A2 or a pharmaceutically acceptable salt thereof, and (b) a second agent which is a cyclin-dependent kinases (CDK) inhibitor which is compound (B) or a pharmaceutically acceptable salt thereof, and at least one excipient for use in the treatment of neuroblastoma. In one reference embodiment, such pharmaceutical composition of the present invention is for use in the treatment of a proliferative disease. In accordance with the present invention, agent (a) and agent (b) may be administered together in a single pharmaceutical composition, separately in one combined unit dosage form or in two separate unit dosage forms, or sequentially. The unit dosage form may also be a fixed combination.

The pharmaceutical compositions for separate administration of agents or for the administration in a fixed combination (i.e., a single galenical composition comprising at least two combination partners (a) and (b)) according to

the invention may be prepared in a manner known *per se* and are those suitable for enteral, such as oral or rectal, topical, and parenteral administration to subjects, including mammals (warm-blooded animals) such as humans, comprising a therapeutically effective amount of at least one pharmacologically active combination partner alone, e.g., as indicated above, or in combination with one or more pharmaceutically acceptable carriers or diluents, especially suitable for enteral or parenteral application. Suitable pharmaceutical compositions contain, e.g., from about 0.1 % to about 99.9%, preferably from about 1% to about 60%, of the active ingredient(s).

Pharmaceutical compositions for the combination therapy for enteral or parenteral administration are, e.g., those in unit dosage forms, such as sugar-coated tablets, tablets, capsules or suppositories, ampoules, injectable solutions or injectable suspensions. Topical administration is e.g. to the skin or the eye, e.g. in the form of lotions, gels, ointments or creams, or in a nasal or a suppository form. If not indicated otherwise, these are prepared in a manner known *per se,* e.g., by means of conventional mixing, granulating, sugar-coating, dissolving or lyophilizing processes. It will be appreciated that the unit content of each agent contained in an individual dose of each dosage form need not in itself constitute an effective amount since the necessary effective amount can be reached by administration of a plurality of dosage units.

Pharmaceutical compositions may comprise one or more pharmaceutical acceptable carriers or diluents and may be manufactured in conventional manner by mixing one or both combination partners with a pharmaceutically acceptable carrier or diluent. Examples of pharmaceutically acceptable diluents include, but are not limited to, lactose, dextrose, mannitol, and/or glycerol, and/or lubricants and/or polyethylene glycol. Examples of pharmaceutically acceptable binders include, but are not limited to, magnesium aluminum silicate, starches, such as corn, wheat or rice starch, gelatin, methylcellulose, sodium carboxymethylcellulose and/or polyvinylpyrrolidone, and, if desired, pharmaceutically acceptable disintegrators include, but are not limited to, starches, agar, alginic acid or a salt thereof, such as sodium alginate, and/or effervescent mixtures, or adsorbents, dyes, flavorings and sweeteners. It is also possible to use the compounds of the present invention in the form of parenterally administrable compositions or in the form of infusion solutions. The pharmaceutical compositions may be sterilized and/or may comprise excipients, for example preservatives, stabilizers, wetting compounds and/or emulsifiers, solubilisers, salts for regulating the osmotic pressure and/or buffers.

In particular, a therapeutically effective amount of each of the combination partner of the combination of the invention may be administered simultaneously or sequentially and in any order, and the components may be administered separately or as a fixed combination. For example, the method of preventing or treating a cancer according to the invention may comprise: (i) administration of the first agent in free or pharmaceutically acceptable salt form; and (ii) administration of a second agent in free or pharmaceutically acceptable salt form, simultaneously or sequentially in any order, in jointly therapeutically effective amounts, preferably in synergistically effective amounts, e.g., in daily or intermittently dosages corresponding to the amounts described herein. The individual combination partners of the combination of the invention may be administered separately at different times during the course of therapy or concurrently in divided or single combination forms. The instant invention is therefore to be understood as embracing all such regimens of simultaneous or alternating treatment and the term "administering" is to be interpreted accordingly.

The effective dosage of each of combination partner agents employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

For purposes of the present invention, a therapeutically effective dose will generally be a total daily dose administered to a host in single or divided doses. The compound of formula (I) may be administered to a host in a daily dosage range of, for example, from about 0.05 to about 50 mg/ kg body weight of the recipient, preferably about 0.1-25 mg/kg body weight of the recipient, more preferably from about 0.5 to 10 mg/kg body weight of the recipient. Agent (b) may be administered to a host in a daily dosage range of, for example, from about 0.001 to 1000 mg/kg body weight of the recipient, preferably from 1.0 to 100 mg/kg body weight of the recipient, and most preferably from 1.0 to 50 mg/kg body weight of the recipient. Dosage unit compositions may contain such amounts of submultiples thereof to make up the daily dose.

The ALKi and CDKi combination of the invention can be used alone or combined with at least one other pharmaceutically active compound for use in these pathologies. These active compounds can be combined in the same pharmaceutical preparation or in the form of combined preparations "kit of parts" in the sense that the combination partners can be dosed independently or by use of different fixed combinations with distinguished amounts of the combination partners, i.e., simultaneously or at different time points. The parts of the kit of parts can then, e.g., be administered

simultaneously or chronologically staggered, that is at different time points and with equal or different time intervals for any part of the kit of parts. Non-limiting examples of compounds which can be cited for use in combination with the ALKi and CDKi combination of the invention include cytotoxic chemotherapy drugs, such as anastrozole, doxorubicin hydrochloride, flutamide, dexamethaxone, docetaxel, cisplatin, paclitaxel, etc.

**KITS**

The present disclosure further relates to a kit comprising a first compound selected from the group consisting of Compounds A1 to A3 or pharmaceutically acceptable salts thereof, and Compound B or pharmaceutically acceptable salts thereof, and a package insert or other labeling including directions for treating a proliferative disease.

The present disclosure further relates to a kit comprising a first compound selected from Compounds A1-A3 or pharmaceutically acceptable salts thereof, and a package insert or other labeling including directions for treating a proliferative disease by co-administering with Compound B or a pharmaceutically acceptable salt thereof.

**EXAMPLES**

The following examples illustrate the invention described herein. The combination of Compound A1 and Compound B and the combination of Compound A2 and Compound B are part of the claimed invention. The other combinations in the examples are reference examples of the combination of the disclosure. The beneficial effects of the pharmaceutical combination of the present invention can also be determined by other test models known as such to the person skilled in the pertinent art.

**Example A: Identify Synergistic Combinations based on the Loewe Additivity Model with high throughput screening**

The synergistic interaction of drug combinations were assessed based on the Loewe Additivity Model using Chalice software [CombinatoRx, Cambridge MA]). See, Lehár J, Krueger AS, Avery W, et al., 2009, in Synergistic drug combinations tend to improve therapeutically relevant selectivity, Nat Biotechnol. 27:659-66. The software compares the response (% inhibition or % reduction of cell viability) of drug treatment from a combination of two agents to the response of the agents acting alone, against the drug-with-itself dose-additive reference model (the Loewe Additivity Model). Deviations from dose additives can be assessed numerically with a "synergy score" which quantifies the overall strength of combination effect. A synergy score >0 indicates a synergistic combination. In order to ensure only strongly synergistic combinations were selected, the acceptance criteria were set at a higher level. Strongly synergistic combinations were defined as having both a synergy score >2, a synergy score that is twice as large as the background (non-synergy) model would predict, and a maximum efficacy of >100, a value equivalent to stasis, as determined from the growth inhibition calculation.

Twenty extensive characterized human neuroblastoma cell lines (Table 1) *infra* were treated with Compounds A1, A2, A3, and B, individually, and with the following combinations:

(1) Compounds A1 and B;
(2) Compounds A2 and B, and
(3) Compounds A3 and B. After treatment, cell viabilty (quantity of viable cells) for each test mixture was determined by the CellTiter-Glo® (CTG) luminescent cell viability assay (Promega) described in the Assay section, *infra.* Fifteen out of 20 of the cell lines generated high quality primary screening data; the other five cell lines either failed to grow or yielded data that were too noisy and therefore not included in the analysis. The response to treatment (% reduction of cell viability) was analyzed using Chalice software [CombinatoRx, Cambridge MA]). Data evaluation and graph generation were performed using Microsoft Excel software and Chalice software.

Synergy scores for the three tested combinations were tabulated in Table 2 and Table 3. Synergy was observed for the combination of A1 and B in LAN1 (F1174L), LAN5 (R1275Q), NB-1643 (R1275Q), NB-SD (F1174L), and NB-1691 (WT) cells. It is noted that this co-treatment was especially synergistic in LAN-5 cells. Synergy was observed for the combination of A2 and B in Kelly (F1174L), LAN-5 (R1275Q), SK-N-BE (2) (WT), and NB-1691 (WT). The self-combination of Compound A2 in the NB-1 (ALK-amplified) cell line was also identified as synergistic likely due to the potent single agent activity of this compound in this cell line. Synergy was not observed for the combination of A3 and B in any of the tested cell lines.

Table 2. Synergy Scores of Combination A1 x B in Human Neuroblastoma Cells

| Cell Line Name | ALK Status | Cmpd A1 x Cmpd B | | | Cmpd A1 Self Cross | | | Cmpd B Self Cross | | |
|---|---|---|---|---|---|---|---|---|---|---|
| | | Mean | Error | Max Combo Effect | Mean | Error | Max Combo Effect | Mean | Error | Max Combo Effect |
| CHP-134 | WT | 0.357 | 0.159 | 50.5 | 0.223 | 0.016 | 26.1 | 0.388 | 0.033 | 52.3 |
| IMR-5 | WT | 0.715 | 0.276 | 79.2 | 0.884 | 0.022 | 80.4 | 0.788 | 0.047 | 82.0 |
| KELLY | F1174L | 1.311 | 0.229 | 91.8 | 0.900 | 0.412 | 97.7 | 0.255 | 0.397 | 60.5 |
| LAN-1 | F1174L | **2.259** | **0.242** | **153.4** | 1.011 | 0.038 | 114.2 | 1.321 | 0.043 | 142.3 |
| LAN-5 | R1275Q | **4.073** | **0.711** | **115.8** | 1.731 | 0.044 | 166.1 | 1.161 | 0.038 | 99.2 |
| NB-1 | WT Amp | 1.421 | 0.579 | 172.8 | 1.607 | 0.054 | 181.9 | 1.429 | 0.036 | 111.0 |
| NB-1643 | R1275Q | **2.573** | **0.444** | **129.0** | 1.231 | 0.046 | 151.1 | 0.929 | 0.030 | 76.4 |
| NB-1691 | WT | **2.031** | **0.402** | **112.0** | 0.760 | 0.034 | 111.9 | 1.032 | 0.051 | 121.1 |
| NB-SD | F1174L | **2.499** | **0.652** | **128.2** | 0.908 | 0.039 | 141.5 | 1.476 | 0.034 | 98.6 |
| NGP | WT | 0.294 | 0.087 | 55.9 | 0.822 | 0.016 | 104.1 | 0.084 | 0.011 | 31.2 |
| NLF | WT | 0.202 | 0.070 | 41.3 | 0.152 | 0.082 | 27.1 | 0.122 | 0.124 | 43.9 |
| SH-SY5Y | F1174L | 1.701 | 0.379 | 92.9 | 0.638 | 0.451 | 91.5 | 0.811 | 0.813 | 86.8 |
| SK-N-AS | WT | 0.615 | 0.150 | 73.1 | 0.374 | 0.231 | 42.4 | 0.503 | 0.273 | 64.7 |
| SK-N-BE(2) | WT | 0.430 | 0.140 | 77.3 | 0.246 | 0.014 | 25.2 | 0.628 | 0.037 | 75.2 |
| SK-N-FI | WT | 1.267 | 0.193 | 70.6 | 1.135 | 0.022 | 59.7 | 0.435 | 0.036 | 60.3 |
| Mean | | 1.450 | | | 0.841 | | | 0.757 | | |
| Median | | 1.311 | | | 0.884 | | | 0.788 | | |

Values in ***BOLD*** were identified as synergistic interaction based on the combination of a Synergy score >2 and a Max Combo effect > 100..

Table 3. Synergy Scores of Combinations of A2 x B and A3 x B in Human Neuroblastoma Cells

| Cell Line Name | ALK Status | Cmpd A2 x Cmpd B | | | Cmpd A3 x Cmpd B | | | Cmpd A2 Self Cross | | | Cmpd A3 Self Cross | | | Cmpd B Self Cross | | |
|---|---|---|---|---|---|---|---|---|---|---|---|---|---|---|---|---|
| | | Mean | Error | Max Combo Effect | Mean | Error | Max Combo Effect | Mean | Error | Max Combo Effect | Mean | Error | Max Combo Effect | Mean | Error | Max Combo Effect |
| CHP-134 | WT | 0.17 | 0.43 | 121.50 | 0.12 | 0.08 | 26.50 | 0.10 | 0.13 | 180.10 | 0.02 | 0.01 | 10.05 | 0.35 | 0.11 | 48.76 |
| IMR-5 | WT | 0.44 | 0.23 | 182.35 | 0.25 | 0.23 | 64.48 | 0.23 | 0.32 | 179.71 | 0.73 | 0.02 | 99.95 | 1.04 | 0.32 | 89.02 |
| KELLY | F1174L | **3.08** | **0.10** | **180.45** | 0.33 | 0.18 | 76.93 | 0.64 | 0.11 | 191.61 | 0.47 | 0.04 | 134.74 | 0.19 | 0.10 | 27.66 |
| LAN-1 | F1174L | N/A | N/A | N/A | 0.59 | 0.17 | 69.42 | N/A | N/A | N/A | 0.63 | 0.14 | 77.73 | 0.25 | 0.20 | 62.78 |
| LAN-5 | R1275Q | **2.55** | **0.40** | **194.16** | N/A | N/A | N/A | 0.87 | 0.33 | 196.81 | N/A | N/A | N/A | 0.57 | 0.38 | 73.59 |
| NB-1 | WT_Amp | 1.93 | 0.15 | 192.87 | N/A | N/A | N/A | **2.97** | **0.07** | **193.34** | N/A | N/A | N/A | 0.34 | 0.16 | 40.57 |
| NB-1691 | WT | **2.62** | **0.17** | **168.26** | 0.45 | 0.20 | 72.66 | 0.97 | 0.34 | 196.56 | 0.21 | 0.02 | 36.53 | 0.38 | 0.25 | 91.57 |
| NB-SD | F1174L | 1.96 | 0.46 | 164.74 | 0.85 | 0.35 | 85.37 | 1.38 | 0.49 | 195.92 | 0.84 | 0.03 | 75.54 | 0.97 | 0.26 | 61.52 |
| NGP | WT | 0.88 | 0.14 | 135.01 | 0.00 | 0.00 | 0.00 | 0.84 | 0.09 | 184.04 | 0.01 | 0.02 | 11.39 | 0.01 | 0.01 | 17.05 |
| NLF | WT | 1.15 | 0.23 | 111.80 | 0.03 | 0.09 | 48.81 | 0.63 | 0.31 | 183.68 | 0.08 | 0.19 | 29.10 | 0.54 | 0.21 | 45.78 |
| SH-SY5Y | F1174L | N/A | N/A | N/A | 0.02 | 0.20 | 72.34 | N/A | N/A | N/A | 0.98 | 0.03 | 141.61 | 0.09 | 0.02 | 35.37 |
| SK-N-AS | WT | 1.59 | 0.14 | 79.57 | 0.03 | 0.16 | 46.47 | 0.20 | 0.07 | 61.99 | 0.19 | 0.01 | 55.78 | 0.48 | 0.10 | 42.50 |
| SK-N-BE(2) | WT | **2.95** | **0.49** | **141.96** | 0.04 | 0.08 | 38.40 | 0.47 | 0.20 | 177.26 | 0.02 | 0.00 | 10.76 | 1.41 | 0.50 | 93.36 |
| SK-N-FI | WT | 0.35 | 0.08 | 116.21 | 0.00 | 0.06 | 32.80 | 0.65 | 0.07 | 161.68 | 0.04 | 0.01 | 20.37 | 0.21 | 0.13 | 51.25 |

Values in ***BOLD*** were identified as synergistic interaction based on the combination of a Synergy score >2 and a Max Combo effect > 100.

The effect of drug treatment was demonstrated by Chalice matrices. Figure 2 shows the Chalice dose matrix and Loewe (ADD) excess inhibition for LAN-1 cells treated by a combination of Compounds A1 and B (top row), Compound A self-cross (middle row), and Compound B self-cross (bottom row). The % inhibition (reduction in cell viability) by the drug treatments were recorded in the block In the dose matrix (left); the single agent treatment at the far left column and the bottom row, and the combinations in the remaining 6x6 combination blocks. The differences between the data in the dose matrix and the expected inhibition value generated by the Loewe model were reported in the Loewe Excess matrix. In this Excess Inhibition matrix, synergy is defined as values >0; that is inhibition greater than what would be expected from a simple additive interaction. Antagonism is defined as values <0; that is inhibition greater than what would be expected from a simple additive interaction. The synergy score for the drug treatments were computed taken into account of the entire 6x6 combination blocks within the matrix. The resulted synergy scores in Lan-1 cells for Cpd. A1 x B, Cpd A1xA1 and Cpd. BxB, were 2.26, 1.01 and 1.32, respectively. The A1xB combination were synergistic in Lan-1 cells.

The statistical variation of the synergy scores tabulated in Tables 2 and 3 were graphically illustrated by a Box Plot (Figure 3). Only data from two of the three ALK inhibitors were included; as used in this plot, an ALK inhibitor refers to Compound A1 or Compound A2, and the CDK4/6 inhibitor refers to Compound B. In ALK disease cells, the ALK x CDK4/6 combinations were strongly synergetic with a synergy score of 2.26 and standard derivation of 0.9. The ALK x ALK and CDK4/6 X CDK4/6 combination is moderately sygergistic with synergy score at 1.011 (sd=0.4) and 1.16 (sd=0.43), respectively. In ALK normal cells, the combinations and the self-cross treatments were not sygeristic with synergistic score of about 0.5, but similarly large standard deviations.

For visual identification for strongly synergistic compositions, the data in Tables 2 and 3 were presented in scatter plots (Figures 4A-C) where maximum combination efficacy were plotted against the synergy scores. Only data from two of the three ALK inhibitors, compounds A1 and A2 were included. In a scatter plot, a vertical shift implies additive effect; a right shift implies synergistic interactions and a diagonal shift implies synergy with a boost in efficacy. Synergistic combination hits are those in the upper right quadrant where the synergy score is >2 and the maximum efficacy is >100. The two ALK inhibitors (Compounds A1 and A2) showed preferential single-agent efficacy for the ALK disease cell lines over the ALK normal cell lines (Figure 4A). The CDK inhibitor (Compound B) showed single-agent efficacy (>100) in two disease cell lines and one normal cell line (Figure 4B). The combination of ALK and CDK4/6 inhibitors resulted in an interaction leading both to synergy and increased efficacy in 7 out of 15 cell lines tested and is preferential in the ALK disease cell lines.

The results support the use of a combination of an ALK inhibitor and a CDK inhibitor for treatment of ALK positive cancers, particularly neuroblastoma.

**Example B: Determination of Combinatorial Drug Effects based on the Chou-Talalay Model**

Combinatorial drug effects of an ALK and a CDK4/6 inhibitors combination were quantified using the Chou-Talalay combination index method (Trends Pharmacol Sci 4, 450-454) using CalcuSyn v2 software (Biosoft, Cambridge, UK).

Four extensive characterized human neuroblastoma cell lines NB1643 (R1275Q), SHSY5Y (F1174L), NB1691 (WT), and EDC1 (WT) were selected for the study. The cell lines were dosed in triplicates in combination using the constant equipotent ratio where the combination partners, Compound A1 and Compound B were combined at 4x, 2x, 1x, ½ and ¼ of their individual $IC_{50}$ dose, and with each compound individually. The concentration dependence of the anti-proliferation effect for both combination partners, first alone and then in combination were measured using the xCELLigence system. CI for each of the treatments was computed by CalcuSyn v2 software (Biosoft, MO).

CI for ALK mutant cell line NB1643 (R1275Q) is reported in Table 5. It is understood that a CI of 0.9-1.1 indicates additive interaction, values below 0.9 indicate synergism, and values over 1.1 indicate antagonism. The data show the CI for all the tested combinations, with the exception of two, were less than 0.9; accordingly, the test combinations of Compound A and Compound B were synergistic.

Table 5. CI for NB1643 cells from the experimental values

| Cpd A1 (nM) | Cpd B (nM) | Fa | CI |
|---|---|---|---|
| 55 | 187.5 | 0.51 | 0.688 |
| 55 | 375 | 0.53 | 0.968 |
| 55 | 750 | 0.69 | 0.695 |
| 55 | 1500 | 0.78 | 0.710 |
| 55 | 3000 | 0.78 | 1.366 |

(continued)

| Cpd A1 (nM) | Cpd B (nM) | Fa | CI |
|---|---|---|---|
| 111 | 187.5 | 0.65 | 0.456 |
| 111 | 375 | 0.75 | 0.341 |
| 111 | 750 | 0.79 | 0.405 |
| 111 | 1500 | 0.84 | 0.462 |
| 111 | 3000 | 0.85 | 0.781 |
| 222 | 187.5 | 0.73 | 0.436 |
| 222 | 375 | 0.8 | 0.326 |
| 222 | 750 | 0.83 | 0.359 |
| 222 | 1500 | 0.86 | 0.429 |
| 222 | 3000 | 0.85 | 0.838 |
| 444 | 187.5 | 0.78 | 0.520 |
| 444 | 375 | 0.83 | 0.390 |
| 444 | 750 | 0.85 | 0.409 |
| 444 | 1500 | 0.9 | 0.321 |
| 444 | 3000 | 0.91 | 0.449 |
| 888 | 187.5 | 0.82 | 0.677 |
| 888 | 375 | 0.85 | 0.546 |
| 888 | 750 | 0.93 | 0.200 |
| 888 | 1500 | 0.89 | 0.508 |
| 888 | 3000 | 0.9 | 0.642 |

The combination drug effect of the A1xB combinations in NB-1643 cells were plotted in Figures 6A-D. Interpretation of these plots may be found on Figures 5A-5D, and in the Assay section *infra*. Each of the plots visually demonstrates that the combination of Compound A1 and B exhibited synergistic effect in NB-1643 cells. Particularly, the Fa-CI plots (Figures 6C and D) shows the CI were much less than 1 (additive) for all combinations tested. Additionally, the isobologram plot (Figure 6E) shows that the $ED_{90}$ and $ED_{75}$ doses for the co-treatments fell well below their respective isobolograms.

One of the many advantages of synergistic combination is that a smaller amount of drug can be used or the dosing less frequent to achieve the same efficacy with diminished side effect. The dose-reduction index (DRI, Chou and Chou, 1988) may be estimated from experimental values or from calculations. For NB1643 cells, the Dose-Reduction Index (DRI) from experimental values are reported in Table 6 and those from calculation are reported in Table 7.

Table 6. DRI from experimental values for Compound A1 and B Co-treatment on NB1643 Cells

| (DRI) | Drug alone | | Dose Reduction Index | |
|---|---|---|---|---|
| Fa | Cpd A1 (nM) | Cpd B (nM) | Cpd A1 | Cpd B |
| 0.51 | 182.6374 | 841.7031 | 3.321 | 4.530 |
| 0.75 | 801.9141 | 4759.6923 | 7.224 | 12.694 |
| 0.83 | 1583.9055 | 1.056e+004 | 7.135 | 14.084 |
| 0.9 | 3723.4705 | 2.874e+004 | 8.386 | 19.160 |
| 0.9 | 3723.4705 | 2.874e+004 | 4.193 | 9.580 |

Table 7. DRI from Calculations for Compound A1 and B Co-treatment on NB1643 Cells

| (DRI) | Drug alone | | Dose Reduction Index | |
|---|---|---|---|---|
| Fa | Cpd A1 (nM) | Cpd B (nM) | Cpd A1 | Cpd B |
| 0.020 | 0.7501 | 1.3507 | 3.099 | 1.652 |
| 0.050 | 2.8193 | 6.3668 | 3.455 | 2.310 |
| 0.100 | 8.0106 | 21.6280 | 3.764 | 3.008 |
| 0.150 | 15.2920 | 46.1139 | 3.969 | 3.543 |
| 0.200 | 24.8814 | 81.5448 | 4.130 | 4.007 |
| 0.250 | 37.1953 | 130.5781 | 4.269 | 4.436 |
| 0.300 | 52.8478 | 197.0133 | 4.394 | 4.849 |
| 0.350 | 72.7063 | 286.2422 | 4.510 | 5.256 |
| 0.400 | 97.9951 | 406.0079 | 4.622 | 5.669 |
| 0.450 | 130.4690 | 567.6688 | 4.732 | 6.095 |
| 0.500 | 172.7061 | 788.3600 | 4.842 | 6.543 |
| 0.550 | 228.6167 | 1094.8487 | 4.954 | 7.024 |
| 0.600 | 304.3766 | 1530.7866 | 5.072 | 7.552 |
| 0.650 | 410.2451 | 2171.2783 | 5.198 | 8.144 |
| 0.700 | 564.4020 | 3154.6674 | 5.336 | 8.829 |
| 0.750 | 801.9141 | 4759.6923 | 5.492 | 9.649 |
| 0.800 | 1198.7842 | 7621.7203 | 5.676 | 10.683 |
| 0.850 | 1950.5238 | 1.348e+004 | 5.907 | 12.083 |
| 0.900 | 3723.4705 | 2.874e+004 | 6.229 | 14.231 |
| 0.950 | 1.058e+004 | 9.762e+004 | 6.786 | 18.536 |
| 0.990 | 1.063e+005 | 1.455e+006 | 8.200 | 33.231 |

The effect of the combination of Compound A1 and B in SHSY5Y (F1174L) cells is reported in Table 8. The data show the combination was moderately synergistic at low to medium concentrations and antagonistic at high concentration.

Table 8. CI for SHSY5Y cells from the experimental values

| Cpd A1 (nM) | Cpd B (nM) | Fa | CI |
|---|---|---|---|
| 375 | 375 | 0.44 | 0.810 |
| 750 | 750 | 0.57 | 0.815 |
| 1500 | 1500 | 0.69 | 1.070 |
| 3000 | 3000 | 0.91 | 0.803 |
| 6000 | 6000 | 0.94 | 1.268 |

The effect of the combinations of Compound A1 and B in SHSY5Y (F1174L) cells are plotted in Figures 7A-F. The plots visually demonstrated that the combinations were slightly to moderately synergistic; the CI values in the Fa-CI plot (Figure 7C) was slightly below 1, and the $ED_{90}$ concentration was just above the $ED_{90}$ isobologram.

The effect of the combinations of Compound A1 and B in NB1691 (WT) cells are plotted in Figures 8A-F. The

Fa-CI plot (Figure 8C) shows the CI was below 1 when compound concentration was low and above 1 when compound concentration was high suggesting that the drug combination was synergistic at low concentration range and additive or slightly antagonistic at higher concentration range. This interpretation is supported by the isobologram plot (Figure 8E) showing that the $ED_{50}$ and $ED_{75}$ combinations were synergetic, but the $ED_{90}$ combination was antagonistic.

The effect of the combination of Compound A1 and B in NBEDC1 (WT) cells is reported in Table 9. The data show the combination was moderately synergistic to synergistic at low to medium concentrations and very strongly synergistic at high concentration.

Table 9. CI for NB-EBC1 cells from the experimental values

| Cpd A1 (nM) | Cpd B (nM) | Fa | CI |
|---|---|---|---|
| 400 | 325 | 0.48 | 0.535 |
| 800 | 650 | 0.66 | 0.699 |
| 1600 | 1300 | 0.85 | 0.781 |
| 3200 | 2600 | 0.95 | 0.799 |
| 6400 | 5200 | 1 | 0.002 |

The effect of the combinations of Compound A1 and B in NB EDC1 (WT) cells are plotted in Figure 9A-F. The Fa-CI plot (Figure 9C) shows the CI were all below 1 suggesting that the drug combinations were synergistic throughout the concentration range tested. This interpretation was supported by the isobologram plot (Figure 9E) showing that the $ED_{50}$, $ED_{75}$ and $ED_{90}$ combinations were all synergetic.

The data presented above demonstrated that the combination of an ALK inhibitor (Compound A1) and a CDK inhibitor (Compound B) was synergistic in neuroblastoma cells based on the Chou-Talalay combination index model. Synergy is present in both ALK positive cell lines and in wild type cell lines.

**Example** C: Effect of Co-treatment on **Cell Morphology** and **Cell** Death

Synergistic anti-proliferative effect of the combination of our invention was visualized by optical microscopy. Human neuroblastoma SH-SY5Y (F1174L) cells were treated with Compound A1 and Compound B, each at $IC_{50}$ concentration, and the combination of Compounds A1 and B, each compound at its $IC_{50}$ concentration. Seventy-two hours after treatment, the treated samples were compared to the untreated samples (vehicle) by optical microscopy and recorded in micrographs (Figures 10A to D). Compared to the untreated sample (Figure 10A), the sample treated by Compound A1 alone (Figure 10B) showed significant reduction in the number of intact cells. The sample that was treated with Compound B (Figure 10C) alone shows minimal effect in cell numbers and cell morphology. The sample treated by the combination of Compound A1 and Compound B (Figure 10D) shows almost no intact cells. These micrographs clearly demonstrated the synergy of the combination of the invention in enhancing cell death in comparison to treatments by the single compound alone.

**Example D: Effect of Co-treatment on Cell Death**

The combination of the invention exhibits synergistic effect in enhancing cell death, but not apoptosis. The effect of treatment with the combinations of the invention on cell viability and apoptosis were evaluated in three human neuroblastoma cell lines: NB1643 (R1275Q), SH-SY5Y (F1174L) and EBC1 (WT) using the ApoTox-Glo Triplex assay. For confirmation, the treatment effect on cell viability was also evaluated in NB1643 (R12750) using the CellTitre-Glo (CTG) Luminescent Cell Viability assay. The assays were described, *infra.*

Cell lines were dosed in triplicate with DMSO vehicle, Compound A1 and Compound B, individually, at the same doses used in combination therapy, and combinations of Compounds A1 and B at a constant equipotent ratio combination of ¼, ½, 1, 2, and 4 times the $IC_{50}$ value for each of the agents. $IC_{50}$ for Compound A1 and Compound B were previously determined as 222 nM and 749.5 nM, respectively. The test mixtures were evaluated by 72 hours after dosing, and the results were plotted in Figures 11A-C, 12A-C, 13A-C and 14A-C.

Figures 11A, 11B, and 11C show the effect of treatment on viability and apoptosis for NB1643 cells. Cell viability and apoptosis were represented as fractional change in fold against concentration of the compound(s). The data show that Compound A1 alone (Figure 11A) or the combination (Figure 11c) were effective in causing cell death and apoptosis, and Compound B alone was only slightly effective (Figure 11B). When compared the responses at specific dosages of Compound A1 alone and in combination (Figure 11C), the data show significant enhancement of cell death with the co-treatment, but same level of apoptosis was observed..

This finding of co-treatment enhanced cell death was separately confirmed by the CTG assay of treated NB1643 cells. At the specific dosage points, combination treatment (Figure 12C) significantly enhances cell death, when compared to single agent treatment (Figures 12A and B).

Figures 13A to C show the effect of treatment for SH-SY5Y (F1174L) cells. Again, comparing to the single agent treatments (Figure 13A and B), the combination treatment (Figure 13 C) shows synergistic effect on cell viability, but the same level of apoptosis at low compound concentration. It was noted that the cells were dying earlier at higher concentration, so the apoptosis was not detectable, and was not evaluated.

Figures 14A to C show the effect of drug treatment for EBC1 (WT) cells. The same level of cell death was observed when the cells were treated with Compound A1 alone (Figure 14 A) or with the co-treatment (Figure 14 C). The combination had no synergistic effect for EBC1 cells.

This study demonstrated that the combination of our invention, particularly, a combination of Compound A1 and Compound B, process synergistic anti-proliferative effect in ALK positive neuroblastoma cells. The combination of the invention would be useful in treating ALK positive proliferative diseases, particularly, ALK positive neuroblastoma.

### Example E: Co-treatment Greatly Reduces pALK and pRb Protein Expression

pALK and pRb are biomarkers for ALK and CDK activation, respectively. The retinoblastoma protein (Rb) is a tumor suppressor protein which prevents excessive cell growth by inhibiting cell cycle progression. Cell proliferation dependent on cdk4 or cdk6 activation through a variety of mechanisms should show an increase of phosphorylated Rb proteins (pRB); inhibition of CDK leads to decreases in pRb and cell cycle arrest. Inhibition of ALK reduces the expression of phosphorylation of ALK (pALK); decrease in pALK leads to decreased proliferation with an eventual endpoint of apoptosis.

Western blotting was used to assay the effect of the drug treatment on the amount of total and phosphorylated Rb protein and total and phosphorylated ALK in ALK+ and wide type neuroblastoma cells and correlated these data with compound doses in fraction of IC50. An ALK+ cell line, NB1643 (R1275Q) and a wide-type cell line EBC1 were selected for the study.

NB1643 (R1275Q) cells were treated with Compound A1 and Compound B individually and in combination at a constant equipotent ratio of 1/16, 1/8, 1/4 and 4 times of the $IC_{50}$ concentration of each of the compound. A sample treated with vehicle and no compound was prepared and served as control. Cell mixtures were analyzed 20 hours post treatment.

EBC1 (WT) cells were treated with Compound A1 and Compound B individually and in combination at a constant equipotent ratio of 1/4, 1/2, 1, and 4 times of the IC50 concentration of each of the compound. A sample with vehicle and no compound was prepared and served as control. The cell mixtures were analyzed 72 hours post treatment.

Figure 15 shows the total ALK (tALK) and pALK status of treated NB1643 cells. The data show that treatment by either agent alone or in combination have little effect on total ALK over the dosing range. Treatment by either agent alone or in combination reduced the amount of pALK protein starting at 1/16 time of the $IC_{50}$ doses; however, treatment by the combination produced a more pronounced reduction effect. It is further noted that the degree of reduction is dependent on the position of phosphorylation. pALK phosphorylated at the tyrosine 1604 codon shows larger reduction than pALK phosphorylated at the tyrosine 1278 codon.

Figure 16 shows the total Rb and pRb status of treated NB1643 cells. Treatment by either agent alone or the combination of the two agents reduced the expression of total Rb and pRb proteins. The reduction was greater from the combination treatment. The effect of treatment also dependent on the position of phosphorylation; the effect of treatment was substantially greater for pRB S795 than pRb S780.

Figure 17 shows the total and pALK status and the total and pRb status of treated EBC1 (WT) cells. The blot shows that the co-treatment was effective in reducing pALK and pRb protein expression.

The above studies conclusively showed that co-treatment reduced the expression of pALK and pRb proteins. The effect was enhanced with co-treatment when compared to treatment with single agent alone. Accordingly, the combinations of the invention exhibited synergy in ALK+ and wide-type cells.

### Example F: Co-treatment enhanced therapeutic effect against human neuroblastoma tumors in CB17 SCID mice

Enhancement of efficacy studies were conducted *in vivo* on CB17 SCID mice against human neuroblastoma SH-SY5Y (bearing an F1174L ALK mutation) xenografts. The mice were divided into four study groups:

1) a control group treated with solvent vehicle only;
2) a group treated with Compound A1 only at 50 mg/kg dose via p.o. gavage, OD (the dose previously shown to be ineffective in this mouse model);

3) a group treated with Compound B only at 250 mg/kg and reduced (in order to reduce toxicity) to 187.5 mg/kg starting on day 5. Treatment schedule was p.o. gavage, OD, and

4) a group treated with the combination of drugs (Compound A1 at 50 mg/kg and Compound B at 250 mg/kg and reduced to 187.5 mg/kg on day 5).

Result of this study is shown in Figures 18, 19A-D and 20.

In mice of Group 4, which were treated with a combination of Compound A1 and Compound B, the tumor volume showed substantial reduction relative to the other groups (Figure 18). When the tumor volume of each test mice in the test groups were plotted against time (Figures 19A to 19D), it is shown that the tumor volume decreased with time in the Group 4 (co-treatment) mice; while the tumor volume increased with time for all other Groups. Figure 20 shows the % of survival of the test mice with time. In Group 4, two of the mice died on day 7, and the rest survived through the treatment period. In Group 3 (treated with Compound B alone), one of the mice died on day 14 and the rest survived through the treatment period. The mice in Groups 1 and 2, the tumor grew too large, the mice were euthanized at week 1½ and 3 respectively.

The result demonstrated that combination therapy against human SY5Y NB xenografts achieved greater efficacy than the treatment with each drug alone. Impressively, treatment of SCID mice bearing the NB SY5Y tumor (which was previously shown to be not responsive to ALK inhibitor treatment alone) with a 50 mg/kg pharmacologically relevant dose of the combination of Compounds A1 and B led to shrinkage of the established tumor and achieved total tumor remission, while treatment with Compound A1 alone at 50 mg/kg resulted in only a slight tumor growth delay compared to vehicle control in this SY5Y xenograft model.

**Example G: Screening for Strongly Synergistic Interactions for Compounds A1 and B in Neuroblastoma Cells.**

To identify the strongly synergistic compositions in neuroblastoma cells lines, combinations of Compounds A1 and B were tested against a panel of 16 neuroblastoma cell lines, ten of which harbored either an activating ALK mutation or amplification (Table 10). The combinations were tested in duplicate using a 7 x 7 dose matrix block in 1536 well format with a cell proliferation readout as described in the Assay section *infra.* Compound A1 and B were combined with themselves to determine the effect of assay noise on the synergy assessment parameters of the expected dose-additive interaction. The number/viability of cells at the time of compound addition was likewise assessed and used to determine the maximum growth inhibition observed within the assay using the NCI method for calculation.

All synergy calculations were performed using CHALICE software package from Zalicus and potential synergistic interactions between compound combinations were assessed using the Excess Inhibition 2D matrix according to the Loewe additivity model and were reported as synergy score (Table 10) (Lehár et al). Strongly synergistic combinations were identified as having both (1) a synergy score greater than 2, a synergy score that is twice as large as the background (non-synergy) model would predict, and (2) a maximum efficacy of >100, a value roughly equivalent to stasis, as determined from the growth inhibition calculation. The drug effects for all assessed combinations were shown in scatter plots (Figure 21). It was observed that treatment with Compound A1 self-cross (top) resulted in preferential single agent efficacy for the ALK Disease subset of cell lines over the ALK WT cell lines. Treatment with Compound B self-cross (middle) showed no single agent efficacy in any of the cell lines tested. Co-treatment with Compounds A1 and B (bottom) resulted in an interaction leading both to synergy and increased efficacy in 3 of the 16 cell lines tested and was preferential in the ALK Disease cell lines.

Table 10. Synergy scores and maximum combination efficacy for Compound A1 and B Combination cross 16 neuroblastoma cell lines

| Cell Line | *ALK* | MYCN amplified | p53 | Synergy Score | Maximum Efficacy |
|---|---|---|---|---|---|
| NB1 | Amp *ALK*$^{del2-3}$ | Yes | WT | 1.22 | 174 |
| 415IMDM | F1174L | Yes | Unknown | 1.68 | 153 |
| KELLY | F1174L | Yes | WT | 1.06 | 93 |
| LAN1 | F1174L | Yes | Mut | 0.57 | 85 |
| NB-SD | F1174L | Yes | Mut | 1.76 | 97 |
| SHSY5Y | F1174L | No | WT | 1.16 | 93 |
| COGN426 | F1245C | Unknown | Unknown | 0.36 | 56 |
| CHP134 | F1245V | Yes | WT | 0.10 | 44 |
| **LAN5 *** | **R1275Q** | **Yes** | **WT** | **3.43** | **116** |
| **NB1643 *** | **R1275Q** | **Yes** | **WT** | **2.57** | **129** |

(continued)

| Cell Line | *ALK* | MYCN amplified | p53 | Synergy Score | Maximum Efficacy |
|---|---|---|---|---|---|
| IMR5 | WT | Yes | WT | 0.72 | 89 |
| **NB1691 *** | **WT** | **Yes** | **MDM2 Amp** | **2.08** | **118** |
| NLF | WT | Yes | Mut | 0.14 | 49 |
| SKNAS | WT | No | Mut | 0.36 | 68 |
| SKNBE(2) | WT | Yes | Mut | 0.78 | 83 |
| SKNFI | WT | No | Mut | 0.98 | 72 |

* synergetic interaction.

**Example H: Dose Effects of Co-treatment with an ALK Inhibitor and a CDK4/6 Inhibitor in Kelly Neuroblastoma Cells.**

The dose effects of co-treatment with an ALK inhibitor (Compound A1 or A2) and a CDK4/6 inhibitor (Compound B) in Kelly neuroblastoma cells were investigated. The assay was run as part of a larger screen. The Kelly cells were obtained from Novartis's cell library and were treated with combinations of Compounds A1 and B and Compounds A2 and B. The assay was as described in the Assay section infra with the exception that a 9 x 9 dose matrix was used instead. Combinations were tested in duplicate using a 9 x 9 dose matrix block. The single agents were dosed in the far left column and the bottom row, and the remaining 8x8 combination blocks were dosed with the compounds in a 3-fold serial dilution series where the top concentration of the stock solution was 1.67 mM, 5mM and 5mM for Compounds A1, A2 and B, respectively. Cell inhibition readout was as described in the Assay section *infra.* Data analyses were performed by Chalice software, and potential synergistic interactions between compound combinations were assessed according to the Loewe Additivity Model and are reported as synergy score. The number/viability of cells at the time of compound addition was likewise assessed and used to determine the maximum growth inhibition observed within the assay using the NCI method for calculation. The result is tabulated in Table 11 and graphically demonstrated in Figures 22A and 22B.

Table 11. Synergy scores and maximum combination efficacy for Compounds A1 and B Combinations and Compounds A2 and B in Kelly (ALK$^+$ F1174L) cells

| Combination | Synergy Score | Maximum Efficacy |
|---|---|---|
| A1 + B | 1.75 | 70.7 |
| A2 + B | 1.48 | 74.5 |

The combinations of either one of the ALK inhibitors with the CDK inhibitor were effective in inhibiting proliferation of Kelly cells, particularly at higher compound concentrations. The synergy scores were moderate, but the isobologram indicated a very strong interaction.

**Example I: Dose effects of co-treatment with an ALK inhibitors and a CDK4/6 inhibitor in Kelly and NB-1 neuroblastoma cells**.

The dose effects of co-treatment with an ALK inhibitor (Compound A1 or B) and a CDK4/6 inhibitor (Compound B) in Kelly (ALK+, Amp and F1174L) and NB-1 (ALK+, Amp) neuroblastoma cells were investigated. The assay was run to confirm the results of Example H above. Kelly cells and NB-1 cells were obtained from Novartis's cell library and were treated with combinations of Compounds A1 and B and Compounds A2 and B. In this experiment, combinations were tested in duplicate using a 9 x 9 dose matrix block where the combination blocks were dosed with a 3-fold serial dilution series. The top concentration of the stock solutions used on Kelly cells was 5mM for each of Compounds A1, A2 and B. The top concentration of the stock solution used on NB-1 cells was 0.56mM, 0.56mM, and 5mM for Compounds A1, A2 and B, respectively. Cell inhibition readout was as described in the Assay section *infra.* Data analyses were performed by Chalice software, and potential synergistic interactions between compound combinations were assessed according to the Loewe Additivity Model and reported as synergy score. The number/viability of cells at time of compound addition was likewise assessed and used to determine the maximum growth inhibition observed within the assay using the NCI method for calculation. Due to skipped wells during the compound transfer, results in the un-dosed blocks were not entered into the computation for synergy scores and maximum combination efficacy. The result is tabulated in Table 12 and the responses to treatment are graphically demonstrated in Figures 23A, 23B, 23C, 23D.

Table 12. Synergy scores and maximum combination efficacy for Compounds A1 and B Combinations and Compounds A2 and B in Kelly (ALK$^+$ F1174L) and NB-1 cells

| | Kelly | | NB-1 | |
|---|---|---|---|---|
| **Combination** | **Synergy Score** | **Maximum Efficacy** | **Synergy Score** | **Maximum Efficacy** |
| **A1 + B** | 2.51 | 165 | 0.174 | 98.5 |
| **A2 + B** | 2.29 | 115 | 0.194 | 123 |

The combinations of either one of the ALK inhibitors with the CDK inhibitor were effective in inhibiting proliferation of Kelly cells and the drug interaction were strongly synergistic.

The combinations of either one of the ALK inhibitors with the CDK inhibitor were ineffective in inhibiting proliferation of NB-1 cells and the combinations were not synergistic.

**Example J: Dose effects of co-treatment with ALK inhibitors and CDK4/6 inhibitors in Kelly, NB-1 and SH-SY5Y neuroblastoma cells.**

The experiment to determine the dose effects of co-treatment with an ALK inhibitor (Compound A1 or A2) and a CDK4/6 inhibitor (Compound B) in Kelly (ALK+, Amp and F1174L), NB-1 (ALK+, Amp) were repeated with different concentration of the drug compound; SH-SY5Y neuroblastoma cells were also included in the experiment. All three cell lines were obtained from Novartis's cell library or from the ATCC.

The cells were treated with combinations of Compounds A1 and B and Compounds A2 and Compound B. In this experiment, combinations were tested in duplicate using a 9 x 9 dose matrix block where the combination blocks were dosed with a 3-fold serial dilution series. The top concentration of the stock solution used on Kelly cells was 2.5mM for each of Compounds A1, A2 and B. The top concentration of the stock solution used on NB-1 cells was 0.28 mM, 0.28 mM, and 2.5 mM for Compounds A1, A2 and B, respectively. The top concentration of the stock solution used in SH-SY5Y cells was 2.5 mM for each of Compounds A1, A2 and B Cell inhibition readout was as described in the Assay section *infra.* Data analyses were performed by Chalice software, and potential synergistic interactions between compound combinations were assessed according to the Loewe additivity model and are reported as synergy score. The number/viability of cells at time of compound addition was likewise assessed and used to determine the maximum growth inhibition observed within the assay using the NCI method for calculation.

Data quality issues were observed. Single agent dose response for both A1 and A2 in Kelly cells were below expectation when compared to those of Example I. The lower dosing concentrations may have contributed to the low synergy score; but other factors, such as salting out of the compounds or cell line instability could play a role. Maximum combination efficacy for the combinations was not determined for this experiment. The synergy scores for Kelly and NB-1 cell lines are tabulated in Table 13 and the dose effects of the treatment are graphically demonstrated in Figures 24A to D. Data quality issues with the SH-SY5Y cell line were more serious, making interpretation of the data difficult (Figures 24E and 24F). Synergy score was not determined for this cell line.

Table 13. Synergy scores and maximum combination efficacy for Compounds A1 and B Combinations and Compounds A2 and B in Kelly (ALK$^+$ F1174L) and NB-1 cells

| | Kelly | | NB-1 | |
|---|---|---|---|---|
| **Combination** | **Synergy Score** | **Maximum Efficacy** | **Synergy Score** | **Maximum Efficacy** |
| A1 + B | 0.82 | ND | 0.142 | ND |
| A2 + B | 1.52 | ND | 0.0.601 | ND |
| ND means not determined | | | | |

Synergy scores for the combinations of A1 x B and A2 x B were low to moderate, below the criteria for strongly synergistic combination (synergy score >2). The combinations of either one of the ALK inhibitors with the CDK inhibitor were not synergistic in NB-1 cells. It should be understood that the data could be unreliable due to the observed data issues.

**ASSAYS**

**Preparation of the ALK and CDK Inhibitors**

The compounds disclosed herein may be synthesized via routine chemistry by one skilled in the art.

Compound A1, 5-chloro-N2-(2-isopropoxy-5-methyl-4-(piperidin-4-yl)phenyl)-N4-[2-(propane-2-sulfonyl)-phenyl]-pyrimidine-2,4-diamine, is specifically disclosed as Example 66 of WO2010/020675, and was prepared by the synthetic procedure described therein.

Compound A2, N6-(2-isopropoxy-5-methyl-4-(1-methylpiperidin-4-yl)phenyl)-N4-(2-(isopropylsulfonyl)phenyl)-1H-pyrazolo[3,4-d]pyrimidine-4,6-diamine, is specifically disclosed as Example 231 of WO2010/020675, and was prepared by the synthetic procedure described therein.

Compound A3 (reference), commonly known as crizotinib, trade name XALKORI®, is marketed by Pfizer Corp. and is commercially available.

Compound B, 7-cyclopentyl-N,N-dimethyl-2-((5-(piperazin-1-yl)pyridin-2-yl)amino)-7H-pyrrolo[2,3-d]pyrimidine-6-carboxamide, is disclosed as Example 74 of WO2010/020675, and was prepared by the synthetic procedure described therein.

**Cell Lines and Cell Cultures**

The cell lines utilized in this work were human neuroblastoma-derived and were obtained from Novartis internal cell library, ATCC and/or from Children's Oncology Group Reference Laboratories in The Children's Hospital of Philadelphia (CHoP). The CHoP cell lines were routinely tested for mycoplasma infection as well as genotyped (AmpFLSTR identifier kit, Life Technologies) to ensure integrity and to guard against cross-contamination. In addition, the cell lines have had genome-wide DNA copy number status determined on the Illumina HH550 SNP chip, and genome-wide exon-level gene expression determined on the Illumina expression chip. The cell lines may be maintained according to recommended media conditions known in the art (e.g., Thiele, C. J. Neuroblastoma: in (Ed.) Masters, J. Human Cell Culture. Lancaster, UK: Kluwer Academic Publishers. 1998, Vol 1, p. 21-53.) Particularly, the cells may be maintained in RPMI-1640 media with 10% fetal bovine serum with 1% penicillin/streptomycin, and 1% L-glutamine at 37°C and 5% $CO_2$. Alternately, the cells may be stored frozen and reconstituted prior to use.

The cell lines were chosen to be equally representative of the ALK target status in primary tissues: ALK mutation positive, ALK mutation negative but genomic amplification and overexpression of wild type ALK, and ALK mutation negative and normal copy number. The cell lines that were ALK mutation positive represent three unique mutations in the anaplastic lymphoma kinase (ALK) tyrosine kinase domain. Exome sequencing, with Sanger sequencing, confirmed that the mutations were in the ALK tyrosine kinase domain. Further, the cell lines were characterized for their MycN, TP53, ALK, TrkA and TrkB status, and the results are summarized in Table 1 below.

Table 1: Human Neuroblastoma Cell Lines and Characterization

| Cell Line Name | MYCN | TP53 | ALK | Trk Status |
|---|---|---|---|---|
| 415IMDM | Amp | Unknown | F1174L | |
| CHLA | | Mutated | F1245V | |
| CHP-134 | Amp | WT | WT | TrkA-, TrkB+ |
| COG-N- 426 | | Unknown | F1245C | |
| IMR5 | Amp | WT | WT | TrkA+, TrkB- |
| Kelly | Amp | WT | F1174L | |
| LAN1 | Amp | Mutated | F1174L | |
| LAN5 | Amp | WT | R1275Q | |
| NB1 | Amp | WT | WT Amp | |
| NB-1643het | Amp | WT | R1275Q | TrkA-, TrkB+ |
| NB-1691 | Amp | MDM2 Amp | WT | |
| NB-1771 | | | pALK+ | |
| NB-EBC1 | Not Amp | WT | WT | |
| NB-SD | Amp | Mut | F1174L | |
| NGP | Amp | MDM2 Amp | WT | TrkA-, TrkB- |
| NLF | Amp | Mut | WT | TrkA-, TrkB- |
| SK-N-AS | Not Amp | Mut | WT | TrkA+, TrkB- |

(continued)

| Cell Line Name | MYCN | TP53 | ALK | Trk Status |
|---|---|---|---|---|
| SK-N-BE2C | Amp | Mut | WT | |
| SK-N-FI | Not Amp | Mut | WT | |
| SH-SY-5Y | Not Amp | WT | F1174L | TrkA-, TrkB- |

It is understood that there are other neuroblastoma cell lines that are suitable for testing with the combinations of the present invention. Information on these cell lines may be obtained from: Thiele CJ.; Neuroblastoma: In (Ed.) Masters, J. Human Cell Culture. Lancaster, UK: Kluwer Academic Publishers. 1998, Vol 1, p 21-53.

**Cell Viability Assay**

Cell viability were determined by measuring cellular ATP content using the CellTiter-Glo® (CTG) luminescent cell viability assay (Promega). CTG reagent was added to cells that have been treated with the test compound, and the resulted luminescence were read by plate reader (e.g., Viewlux, Perkin Elmer). Reduced and enhanced luminescent signal values (responses) were calculated relative to untreated (control) cells, and the calculated signal value proportional to the cell viability.

**Identify synergistic therapeutic combinations in a high-through screen based on the Loewe Additivity Model**

Synergistic combinations were identified based on the Loewe Addivity Model. To measure the effects of drug combinations on the cell viability, cells from the 20 cell lines listed in Table 1 above were seeded into 1536-well assay plates at a density 300 cells per well in a 7μL final volume and incubated at 37°C overnight in a GNF Systems incubator with 95% RH and 5% $CO_2$.

A six-point dose response curve for the test compounds were prepared in a 384 well ECHO compatible source plate (Labcyte P-05525) with 3-fold serial dilution series. For example, for a six-point dose response curve and a top compound concentration of 5 mM, the concentration of the compounds in the source well were 5mM, 1.67mM, 0.56mM, 0.19mM, 0.06mM and 0.02mM.

Approximately 18 hours after plating, compound combinations were generated on the fly by transferring 7.5nL of compound from the pre-diluted source plates using the Labcyte ECHO555 integrated onto the ACP-1 system with replicate plates per cell line; the final DMSO concentration per well was 0.2%. It is noted that the total volume in the wells was 7μL.

To evaluate the anti-proliferative activity of all the combinations in a non-bias way, as well as to identify synergistic effect at all possible concentration, the dosing was in a 7x7 matrix (9x9 in early experiments) well (block) which utilized all possible permutations of the six (or 8) point serially-diluted test agents. The single agent curves were created by dosing the two agents individually in the first six wells of the first column (left hand) and the first row (bottom) of the combination block; each well received 7.5 nL of the test compound and 7.5 nL of DMSO, with progressively lower compound concentration towards the lower left hand corner. The well at the intersection of the first column and first row which received no compound was dosed with 2x 7.5nL of DMSO and served as control. The combination curves were created by dosing 7.5nL each of the two compounds in each well across their entire dosage range, and again with progressively lower compound concentration towards the lower left hand corner.

Following compound addition, the plates were returned to the incubator for 120 hours. The effects of the combinations on cellular viability were assessed with the addition of Cell Titer Glo (Promega G7573) using one of the GNF Bottle Valve dispensers on the ACP-1 system; plates were then incubated at room temperature for ten minutes and read on the integrated Perkin Elmer Viewlux (2 second exposure, 2x bin, high sensitivity). The raw data was normalized using the DMSO-treated cell control well within each plate. The number/viability of cells at time of compound addition was likewise assessed and used to determine the maximum growth inhibition observed within the assay using the NCI method for calculation. See, Boyd, M. R.; Paull, K. D.; Rubinstein, L. R. In Cytotoxic Anticancer Drugs: Models and Concepts for Drug Discovery and Development; Vleriote, F. A.; Corbett, T. H.; Baker, L. H., Eds.; Kluwer Academic: Hingham, MA, 1992; pp 11-34, and Monks, A.; Scudiero, D. A.; Skehan, P.; Shoemaker, R. H.; Paull, K. D.; Vistica, D. T.; Hose, C.; Langley, J.; Cronice, P.; Vaigro-Wolf, M.; Gray-Goodrich, M.; Campbell, H.; Mayo, M. R. JNCI, J. Natl. Cancer Inst. 1991, 83, 757-766.

All synergy calculations were performed using CHALICE software from Zalicus. See, Lehár J, Krueger AS, Avery W, et al., 2009, in Synergistic drug combinations tend to improve therapeutically relevant selectivity, Nat Biotechnol. 27:659-66. Potential synergistic interactions between compound combinations were assessed using the Excess Inhibition

2D matrix according to the Loewe Additivity Model and are reported as synergy score.

Compounds were combined with themselves to determine the effect of assay noise on the synergy assessment parameters of the expected dose-additive interaction. Synergistic combination hits (strongly synergistic) were identified as having both a synergy score >2, a synergy score that is twice as large as the background model (non-synergy) would predict, and a maximum efficacy of >100, a value equivalent to stasis, as determined from the growth inhibition calculation.

Synergistic interactive can be visually assessed from the 2D matrix output of the CHALICE software. Figure 1 shows the 2D matrix plots of a hypothetical growth inhibition experiment. The dose matrix plot (left) is the Chalice representation of the experimental data where the single agent dose response curves are shown on the far left column and the bottom row with the upper right corner of the combination block depicting the highest concentration of each agent. The Loewe Excess Inhibition plot (right) represents the comparison of the experimental data above to the Loewe model generated from the single agent curves. The dose additive model calculates an expected inhibition value for each block in the combination matrix. Synergy is defined as values >0 in the Excess Inhibition plot; that is inhibition greater than what would be expected from a simple additive interaction. Antagonism is defined as values <0; that is inhibition less than what would be expected from a simple additive interaction.

Other common alternative for visual presentation of the data from drug combination studies includes block plots (Figure 3) and scatter plots (Figures 4A, 4B and 4C). A box plot was used to compare synergy scores of the treatment regimens. Scatter plots were used to visualize the trends of interactions between the compounds and to identify the strongly synergistic interactions.

**Determination of Combinational Drug Effects Based on the Chou-Talalay Combination Index Theorem**

Cell Culture

The neuroblastoma cell lines used in this experiment were described in the cell culture section above and in Table 1. Particularly, the cells were maintained in the cells maintained in RPMI-1640 media with 10% fetal bovine serum with 1% penicillin/streptomycin, and 1% L-glutamine at 37°C and 5% $CO_2$.

In vitro growth $IC_{50}$ with Compound A1 and Compound B monotherapy

*In vitro* inhibitory activity was determined in five (5) neuroblastoma cell lines with a 96-well Real-Time Cell Electronic Sensing xCELLigence system (ACEA, San Diego, CA) which measures a "cell index". Cell index is derived from alterations in electrical impedance as cells interact with the biocompatible microelectrode surface in each well to measure substrate adherent proliferation. Depending on the growth kinetics, the following cell densities were plated per well: NB1643: 20,000; SHSY5Y: 6,000; SKBE2C:10,000; NBEBC1: 11,000; NB1691: 30,000. After 24 hours, the plated cells were treated in triplicate with the test compound, each dose as indicated or with DMSO vehicle control. Compound A1 was dosed at 1 nM to 10,000 nM per well, while Compound B was dosed at 0.6 nM to 6,000 nM or 1 nM to 10,000 nM per well. At 72 hours after drug exposure, the cell index was recorded.

The $IC_{50}$ was calculated using GraphPad Prism 5.0 four parameter variable slope fitting. The $IC_{50}$ of Compound A1 and Compound B in selected cell lines are summarized in Table 4 below. These values were used in dosing of the combination study which follows.

*In vitro* drug combination studies

Drug combination effects and quantification of synergy were determined using the Chou-Talalay combination index method (Trends Pharmacol Sci 4, 450-454) and CalcuSyn v2 software (Biosoft, MO) in four neuroblastoma cell lines. Cells were plated and *in vitro* proliferation was measured using the xCELLigence system as described above. Cells were dosed in triplicates, with constant equipotent drug combinations, where the two agents, in e.g., Compound A1 and Compound B, were combined at 4x, 2x, 1x, ½ and ¼ of their individual $IC_{50}$ dose, and with each agent individually.

The anti-proliferation potency of each individual agent was estimated by the median-effect dose, $D_m$. $D_m$ is the compound concentration that results in the median effect defined as the x-intercept on a "median-effect plot" where x = log (D) and y = log ($f_a/f_u$) according to the following definitions:

D: dose of drug;

$F_a$: fraction affected is defined as the fraction of cells affected by the given concentration of compounds alone or in combination. $F_a$ = 0 is determined based on DMSO control by the dose, and $F_a$ =1 is a full response (no viable cells left) and.

$F_u$: fraction unaffected by dose where fu = 1 - fa.

The $D_m$ of Compound A1 and Compound B in selected cell lines are summarized in Table 4 below.

Table 4: Summary of $IC_{50}$ and Dm for Compounds A1 and B in Selected Neuroblastoma Cells

| NB Cell Line Name | ALK Status | Cpd A1 $IC_{50}$ (nM) | Cpd A1 Dm (nM) | Cpd B $IC_{50}$ (nM) | Cpd B Dm (nM) |
|---|---|---|---|---|---|
| NB1643het | R1275Q | 222 | 172.7 | 749.5 | 788.4 |
| SHSY5Y | F1174L | 963.15 | 1500 | 111 | 1500 |
| NBEBC1 | WT | 1924.7 | 1662 | 328 | 1258.7 |
| NB1691 | WT | 1933 | 761.6 | 314.8 | 2647.3 |
| SKBE2C | WT | 602.7 | 349.5 | 145.8 | 446 |
| SKNAS | WT | 2153 | | 4724.5 | |
| SKNFI | WT | 3475 | | >6uM | |

The effect of combination drug effects was determined utilizing the combination index (CI) as defined by Chou according to the following equation:

$$CI = (D)_1 / (D_x)_1 + (D)_2 / (D_x)_2$$

where $(D_x)_1$ and $(D_x)_2$ are the concentrations of compounds $D_1$ and $D_2$ needed to produce a given level of anti-proliferative effect when used individually, whereas $(D)_1$ and $(D)_2$ are their concentrations that produce the same anti-proliferative effect when used in combination. The combination index is a quantitative measure of drug interaction defined as an additive effect (CI =1), antagonism (CI >1), or synergy (CI <1). Typically, a CI range, as used herein, is used to assess synergy. A combination index of 0.9-1.1 indicates additive interaction, values below 0.9 indicate synergism, and values over 1.1 indicate antagonism. The follow is a description of the CI ranges:

<0.1 +++++ Very strong synergism
0.1-0.3 ++++ Strong synergism
0.3-0.7 +++ Synergism
0.7-0.85 ++ Moderate synergism
0.85-0.90 + Slight synergism
0.90-1.10 ± Nearly additive
1.10-1.20 - Slight antagonism
1.20-1.45 -- Moderate antagonism

The combination index was used to assess the Dose-Reduction Index (DRI) (Chou and Chou, 1988), where:

$$CI = (D)_1 / (D_x)_1 + (D)_2 / D_x)_2 = 1 / (DRI)_1 + 1 / (DRI)_2$$

The DRI estimates how much the dose of each drug can be reduced when synergistic drugs are given in combination, while still achieving the same effect size as each drug administered individually.

The drug combination effect may be demonstrated graphically. Typical examples of drug combination plots based on the Chou and Talalay combination index theorem include (a) the "Fa - CI plot", (b) the classic isobologram; (c) the normalized isobologram which are for combinations at different combination ratios, and (d) the Fa-PRI plot (Chou and Martin, 2005. The interpretation of the various plots are summarized in Figures 5A-D. For assessing synergistic effect, the Fa-CI plot and the isobologram plot are more relevant.

***In vitro* viability and Apoptosis by Caspase-Glo 3/7 Assay**

In vitro cell viability and Caspase-Glo 3/7 were simultaneously assayed using the ApoTox-Glo Triplex Assay (Promega, CA) in three neuroblastoma cell lines. At 24 hours, cells were treated in triplicate with DMSO vehicle, Compound A1 or Compound B individually at the same doses used in combination therapy, or with a constant equipotent ratio combination at the doses indicated. After 72 hours of drug exposure, the fluorogenic substrate GF-AFC was added. The test mixture was incubated for 10 minutes and AFC fluorescence was measured at 380-400nm excitation and 505

nm emission. GF-AFC fluoresces following cleavage by a protease that is active when intracellular and inactive upon loss of cell membrane integrity, and thereby GF-AFC fluorescence is correlated with cell viability.

Following measurement of live cell fluorescence, a luminogenic Caspase-Glo 3/7 substrate and luciferase was added to the same wells. Luminescence was measured after a 30 minutes incubation period. Luciferin is released following cleavage of the substrate by caspase-3/7, and thereby the luminescent signal is proportional to caspase activity.

**Western blots**

Each cell line was grown to 70 to 80% confidence, and treated with Compound A1, Compound B, or in combination at an equipotent ratio for 20 hours or 72 hours as indicated. Washed twice with ice-cold phosphate buffered saline and whole-cell protein lysates were analyzed as described (Mosse, et al. Identification of ALK as a major familial neuroblastoma predisposition gent, 2008, Nature Vol 455) by immunoblotting with antibodies to: ALK, 1:1000; pALK $Tyr^{1604}$, 1:1000; pALK $Tyr^{1278}$, 1:2000; RB, 1:2000; $pRB^{S780}$, 1:2000; $pRB^{S795}$, 1:2000; Cyclin D1, 1:1000; Cyclin D3, 1:1000 (Cell Signaling); CDK4, 1:2000; and CDK6, 1:3000; (Santa Cruz).

**In vivo tumor growth inhibition**

CB17 scid female mice (Taconic Farms) were used to propagate subcutaneously implanted neuroblastoma tumors. Tumor diameters were measured twice per week with electronic calipers, and tumor volumes were calculated with the spheroid formula, (p/6)xd3, where d represents mean diameter. Once tumor volume exceeded 200 $mm^3$, mice were randomized (n = 10 per arm) to receive vehicle, Compound A1 (50 mg/kg per dose), Compound B (150 mg/kg per dose), or combined Compound A1 (50 mg/kg per dose) and Compound B (150 mg/kg per dose) daily by oral gavage for 7 weeks. Mice were euthanized when tumor volume exceeded 3000 $mm^3$ or at the study conclusion at 7 weeks. A mixed-effects linear model was used to assess tumor volume over time between treatment and vehicle groups, controlling for tumor size at enrollment. Event-free survival probabilities were estimated using the Kaplan-Meier method and survival curves were compared using the log-rank test (SAS 9.3 and Stata 12.1). An event was defined as time to tumor volume $\geq$ 3000 $mm^3$, and tumor volumes after week 7 were censored. Mice were maintained under protocols and conditions approved by our institutional animal care and use committee.

**Claims**

**1.** A pharmaceutical combination comprising, separately or together,

(a) a first agent which is

(i) Compound A1, described by Formula A1 below:

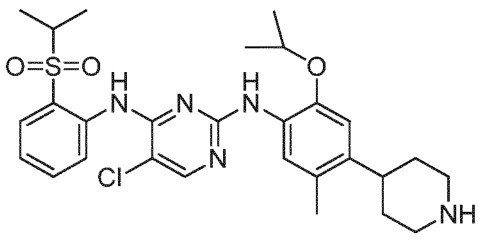

(A1),

or a pharmaceutically acceptable salt thereof,
or
(ii) Compound A2, described by Formula A2 below:

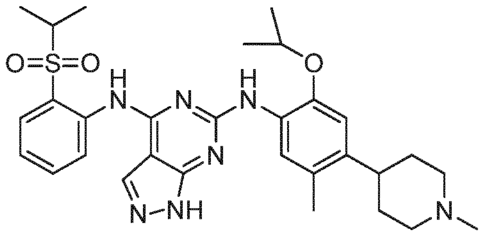

(A2),

or a pharmaceutically acceptable salt thereof,

and (b) a second agent which is
Compound B, described by Formula B below:

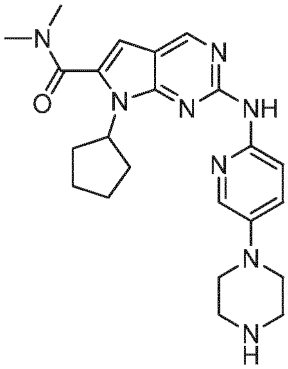

(B),

or a pharmaceutically acceptable salt thereof,
for use in the treatment of neuroblastoma.

2. The pharmaceutical combination for use according to Claim 1, wherein the first agent is Compound A1, or a pharmaceutically acceptable salt thereof,
and the second agent is Compound B, or a pharmaceutically acceptable salt thereof.

3. The pharmaceutical combination for use according to Claim 1, wherein the first agent is Compound A2, or a pharmaceutically acceptable salt thereof,
and the second agent is Compound B, or a pharmaceutically acceptable salt thereof.

4. A pharmaceutical composition comprising a pharmaceutical combination defined in any one of Claims 1 to 3, and at least one excipient for use in the treatment of neuroblastoma.

5. The combination for use according to any one of Claims 1 to 3, or the pharmaceutical composition for use according to claim 4, wherein the neuroblastoma is an ALK positive cancer.

6. The combination for use according to claim 5, or the pharmaceutical composition for use according to claim 5, wherein the cancer is dependent on a mutation of the ALK gene.

7. The combination for use according to claim 5, or the pharmaceutical composition for use according to claim 5, wherein the cancer is dependent on an amplification of the ALK gene.

8. The combination for use according to claim 6, wherein the mutation of the ALK gene is R1275Q.

**Patentansprüche**

1. Pharmazeutische Kombination, umfassend separat oder gemeinsam

(a) ein erstes Agens, bei dem es sich um

(i) Verbindung A1 gemäß Formel A1 unten:

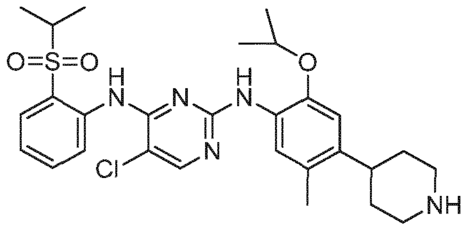

(A1)

oder ein pharmazeutisch annehmbares Salz davon
oder
(ii) Verbindung A2 gemäß Formel A2 unten:

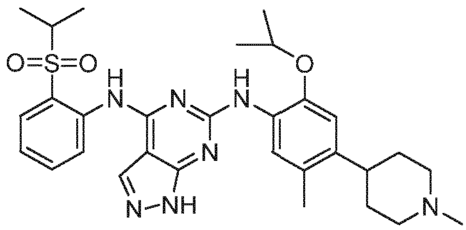

(A2)

oder ein pharmazeutisch annehmbares Salz davon handelt,

und (b) ein zweites Agens, bei dem es sich um
Verbindung B gemäß Formel B unten:

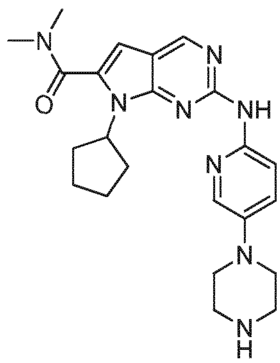

(B)

oder ein pharmazeutisch annehmbares Salz davon handelt,
zur Verwendung in der Behandlung von Neuroblastom.

2. Pharmazeutische Kombination zur Verwendung nach Anspruch 1, wobei es sich bei dem ersten Agens um Verbindung A1 oder ein pharmazeutisch annehmbares Salz davon handelt und bei dem zweiten Agens um Verbindung B oder ein pharmazeutisch annehmbares Salz davon handelt.

3. Pharmazeutische Kombination zur Verwendung nach Anspruch 1, wobei es sich bei dem ersten Agens um Verbindung A2 oder ein pharmazeutisch annehmbares Salz davon handelt und bei dem zweiten Agens um Verbindung B oder ein pharmazeutisch annehmbares Salz davon handelt.

4. Pharmazeutische Zusammensetzung, umfassend eine pharmazeutische Kombination wie in einem der Ansprüche 1 bis 3 definiert sowie mindestens einen Grundstoff zur Verwendung in der Behandlung von Neuroblastom.

5. Kombination zur Verwendung nach einem der Ansprüche 1 bis 3 oder pharmazeutische Zusammensetzung zur Verwendung nach Anspruch 4, wobei es sich bei dem Neuroblastom um einen ALK-positiven Krebs handelt.

6. Kombination zur Verwendung nach Anspruch 5 oder pharmazeutische Zusammensetzung zur Verwendung nach Anspruch 5, wobei der Krebs von einer Mutation des ALK-Gens abhängig ist.

7. Kombination zur Verwendung nach Anspruch 5 oder pharmazeutische Zusammensetzung zur Verwendung nach Anspruch 5, wobei der Krebs von einer Amplifikation des ALK-Gens abhängig ist.

8. Kombination zur Verwendung nach Anspruch 6, wobei es sich bei der Mutation des ALK-Gens um R1275Q handelt.

## Revendications

1. Combinaison pharmaceutique comprenant, séparément ou conjointement,

(a) un premier agent qui est

(i) le composé A1, décrit par la formule A1 ci-dessous :

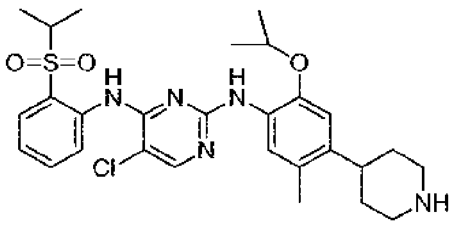

(A1),

ou un sel pharmaceutiquement acceptable de celui-ci,
ou
(ii) le composé A2, décrit par la formule A2 ci-dessous :

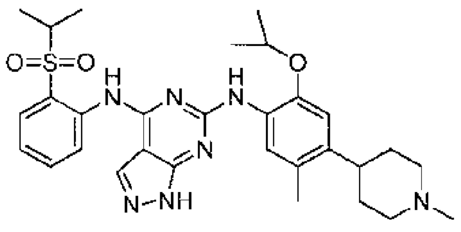

(A2),

ou un sel pharmaceutiquement acceptable de celui-ci,

et (b) un deuxième agent qui est le composé B, décrit par la formule B ci-dessous :

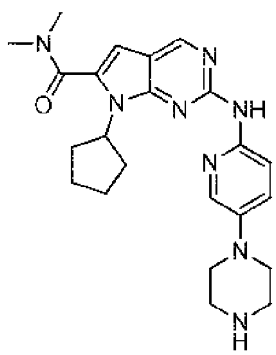

(B),

ou un sel pharmaceutiquement acceptable de celui-ci,
pour utilisation dans le traitement d'un neuroblastome.

2. Combinaison pharmaceutique pour utilisation selon la revendication 1, dans laquelle le premier agent est le composé A1, ou un sel pharmaceutiquement acceptable de celui-ci,
et le deuxième agent est le composé B, ou un sel pharmaceutiquement acceptable de celui-ci.

3. Combinaison pharmaceutique pour utilisation selon la revendication 1, dans laquelle le premier agent est le composé A2, ou un sel pharmaceutiquement acceptable de celui-ci,
et le deuxième agent est le composé B, ou un sel pharmaceutiquement acceptable de celui-ci.

4. Composition pharmaceutique comprenant une combinaison pharmaceutique définie dans l'une quelconque des revendications 1 à 3, et au moins un excipient pour utilisation dans le traitement d'un neuroblastome.

5. Combinaison pour utilisation selon l'une quelconque des revendications 1 à 3, ou composition pharmaceutique pour utilisation selon la revendication 4, le neuroblastome étant un cancer positif pour ALK.

6. Combinaison pour utilisation selon la revendication 5, ou composition pharmaceutique pour utilisation selon la revendication 5, le cancer étant dépendant d'une mutation du gène ALK.

7. Combinaison pour utilisation selon la revendication 5, ou composition pharmaceutique pour utilisation selon la revendication 5, le cancer étant dépendant de l'amplification du gène ALK.

8. Combinaison pour utilisation selon la revendication 6, la mutation du gène ALK étant R1275Q.

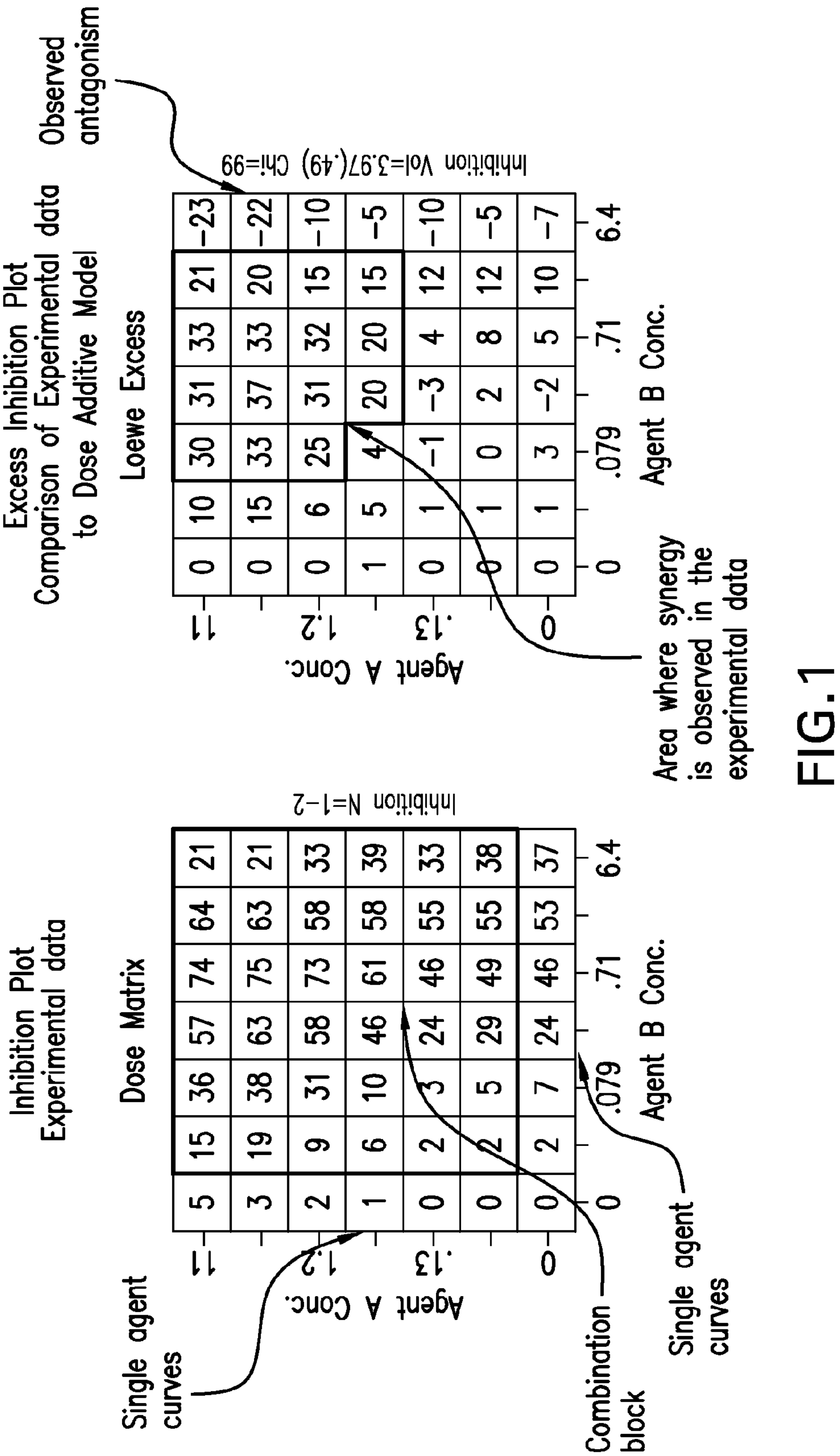
Inhibition Plot
Experimental data
Dose Matrix
Single agent curves
Combination block
Single agent curves
Agent A Conc.
11
1.2
.13
0
Agent B Conc.
0
.079
.71
6.4
5 15 36 57 74 64 21
3 19 38 63 75 63 21
2 9 31 58 73 58 33
1 6 10 46 61 58 39
0 2 3 24 46 55 33
0 2 5 29 49 55 38
0 2 7 24 46 53 37
Inhibition N=1-2
Excess Inhibition Plot
Comparison of Experimental data
to Dose Additive Model
Loewe Excess
Observed antagonism
Area where synergy is observed in the experimental data
0 10 30 31 33 21 −23
0 15 33 37 33 20 −22
0 6 25 31 32 15 −10
1 5 4 20 20 15 −5
0 1 −1 −3 4 12 −10
0 1 0 2 8 12 −5
0 1 3 −2 5 10 −7
Inhibition Vol=3.97(.49) Chi=99

FIG.1

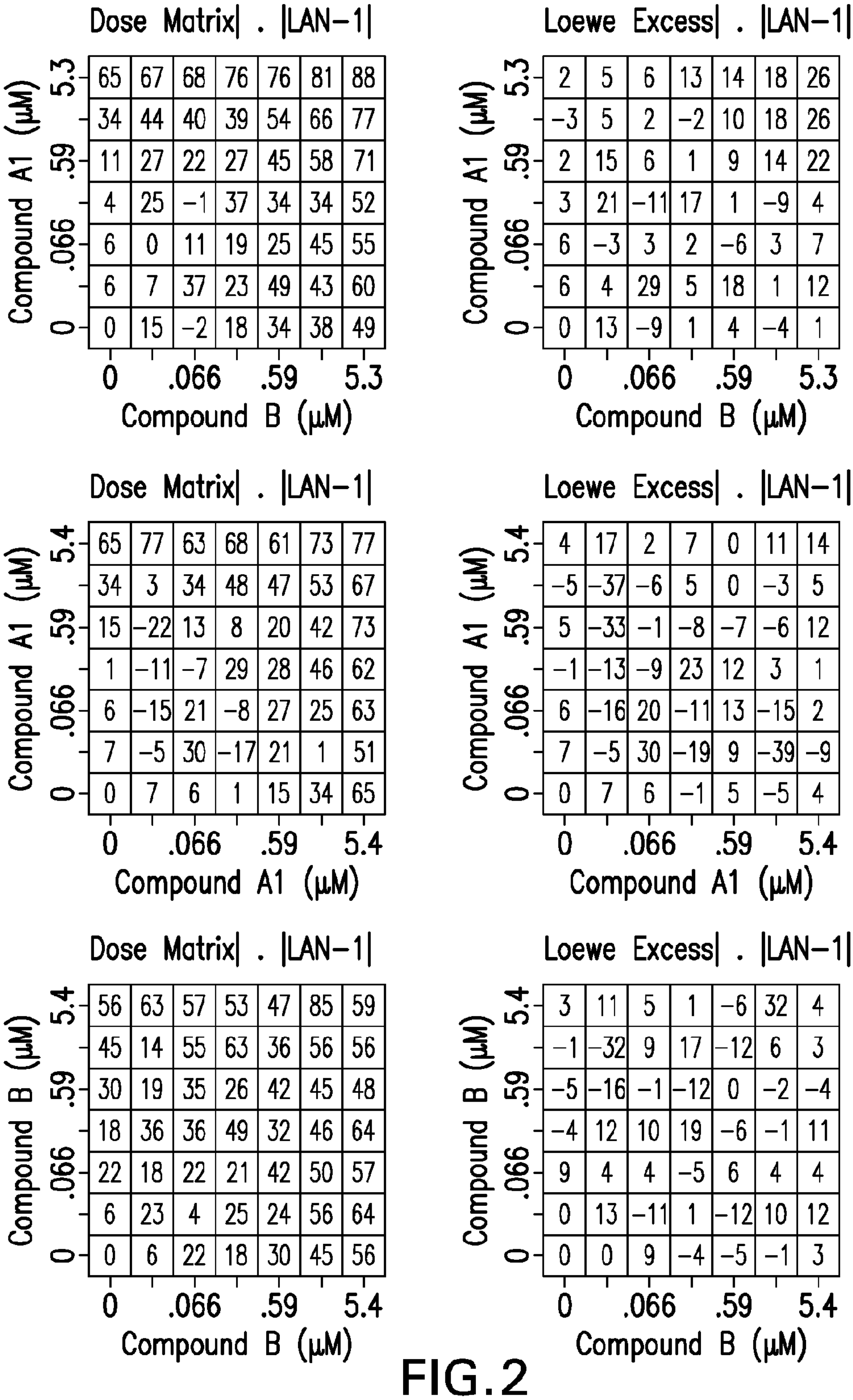
Dose Matrix| . |LAN-1|
65 67 68 76 76 81 88
34 44 40 39 54 66 77
11 27 22 27 45 58 71
4 25 -1 37 34 34 52
6 0 11 19 25 45 55
6 7 37 23 49 43 60
0 15 -2 18 34 38 49
Compound A1 (μM)
0 .066 .59 5.3
Compound B (μM)
Loewe Excess| . |LAN-1|
2 5 6 13 14 18 26
-3 5 2 -2 10 18 26
2 15 6 1 9 14 22
3 21 -11 17 1 -9 4
6 -3 3 2 -6 3 7
6 4 29 5 18 1 12
0 13 -9 1 4 -4 1
Compound A1 (μM)
0 .066 .59 5.3
Compound B (μM)
Dose Matrix| . |LAN-1|
65 77 63 68 61 73 77
34 3 34 48 47 53 67
15 -22 13 8 20 42 73
1 -11 -7 29 28 46 62
6 -15 21 -8 27 25 63
7 -5 30 -17 21 1 51
0 7 6 1 15 34 65
Compound A1 (μM)
0 .066 .59 5.4
Compound A1 (μM)
Loewe Excess| . |LAN-1|
4 17 2 7 0 11 14
-5 -37 -6 5 0 -3 5
5 -33 -1 -8 -7 -6 12
-1 -13 -9 23 12 3 1
6 -16 20 -11 13 -15 2
7 -5 30 -19 9 -39 -9
0 7 6 -1 5 -5 4
Compound A1 (μM)
0 .066 .59 5.4
Compound A1 (μM)
Dose Matrix| . |LAN-1|
56 63 57 53 47 85 59
45 14 55 63 36 56 56
30 19 35 26 42 45 48
18 36 36 49 32 46 64
22 18 22 21 42 50 57
6 23 4 25 24 56 64
0 6 22 18 30 45 56
Compound B (μM)
0 .066 .59 5.4
Compound B (μM)
Loewe Excess| . |LAN-1|
3 11 5 1 -6 32 4
-1 -32 9 17 -12 6 3
-5 -16 -1 -12 0 -2 -4
-4 12 10 19 -6 -1 11
9 4 4 -5 6 4 4
0 13 -11 1 -12 10 12
0 0 9 -4 -5 -1 3
Compound B (μM)
0 .066 .59 5.4
Compound B (μM)

FIG.2

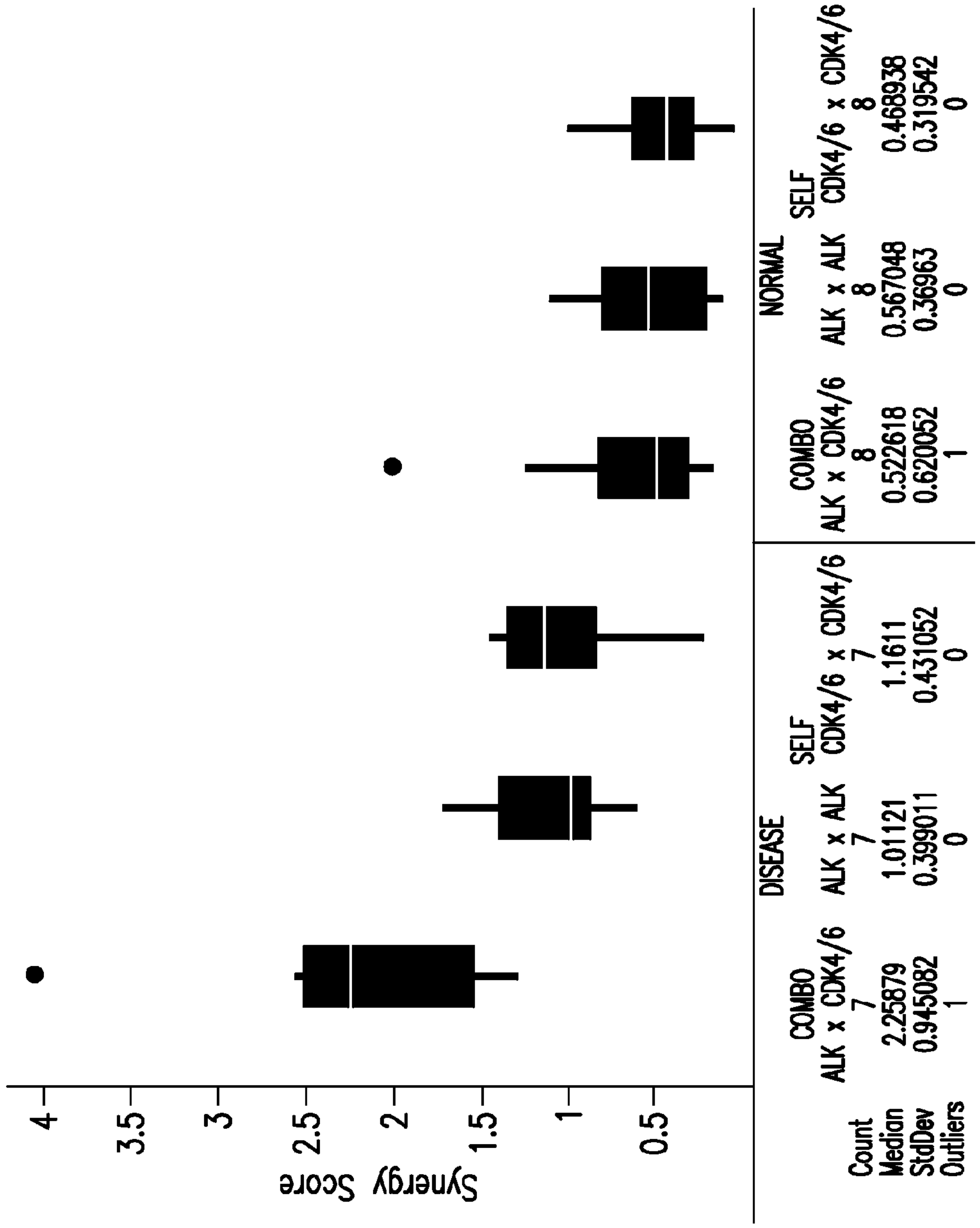
Synergy Score
4
3.5
3
2.5
2
1.5
1
0.5
DISEASE
NORMAL
COMBO
SELF
COMBO
SELF
ALK x CDK4/6
ALK x ALK
CDK4/6 x CDK4/6
ALK x CDK4/6
ALK x ALK
CDK4/6 x CDK4/6
Count
Median
StdDev
Outliers
7
2.25879
0.945082
1
7
1.01121
0.399011
0
7
1.1611
0.431052
0
8
0.522618
0.620052
1
8
0.567048
0.36963
0
8
0.468938
0.319542
0

FIG.3

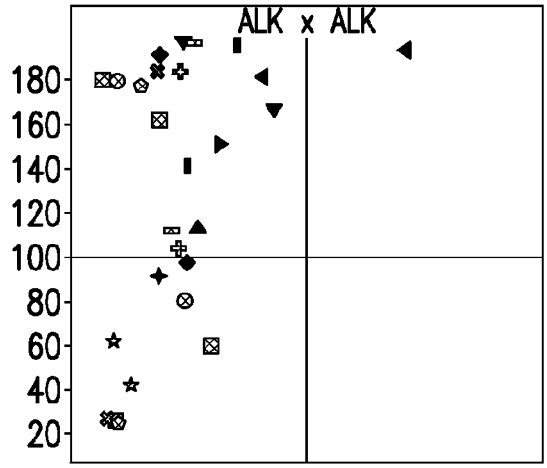


FIG.4A

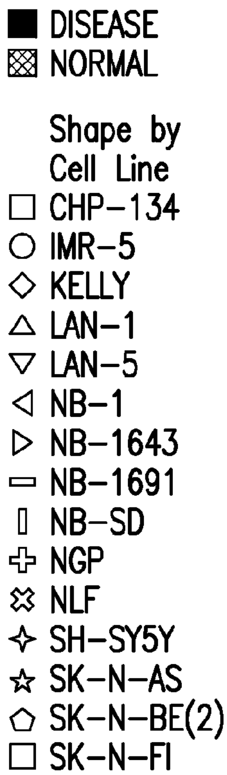


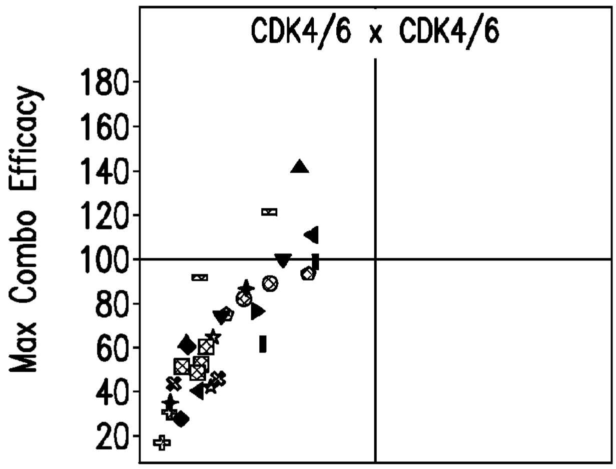


FIG.4B

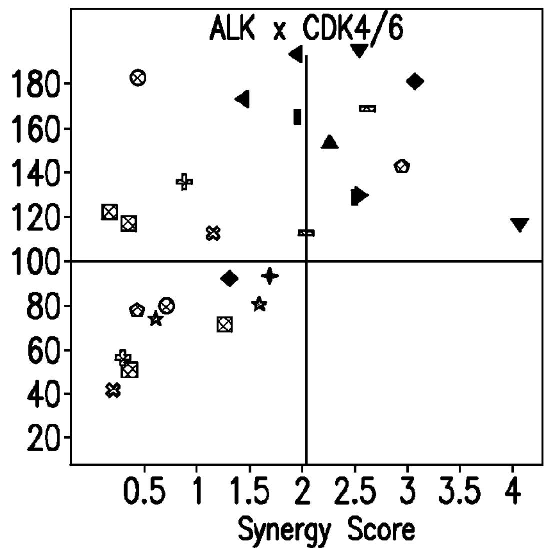


FIG.4C

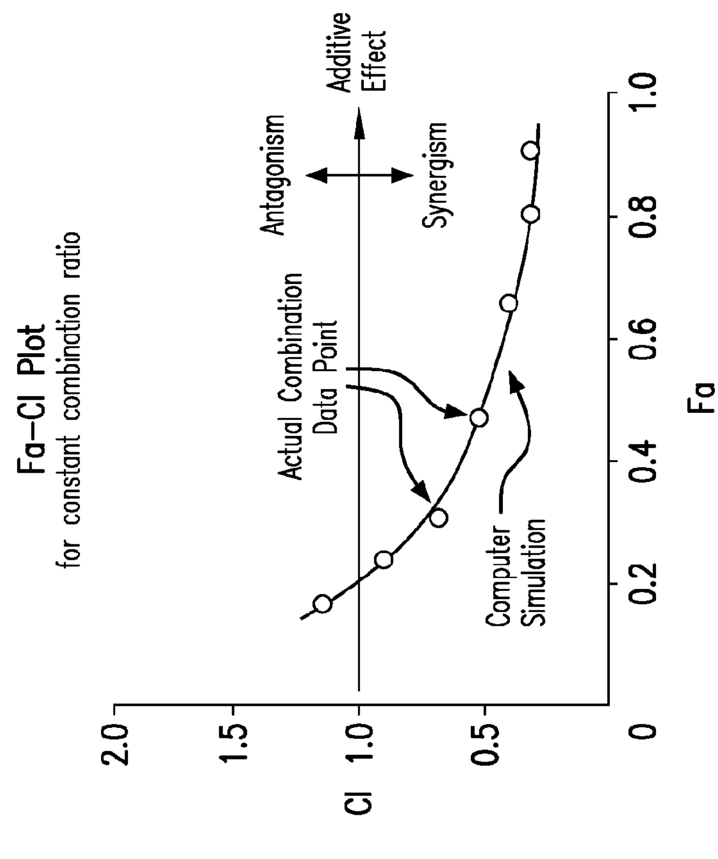
Fa–CI Plot
for constant combination ratio
CI
2.0
1.5
1.0
0.5
0
0.2
0.4
0.6
0.8
1.0
Fa
Antagonism
Synergism
Additive Effect
Actual Combination Data Point
Computer Simulation

FIG.5A

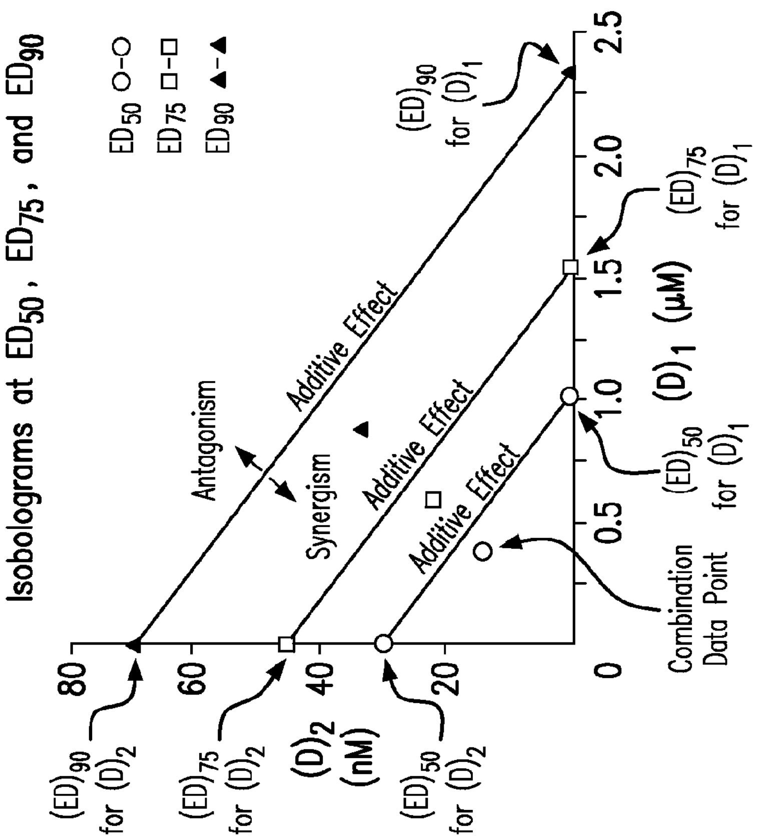
Isobolograms at $ED_{50}$, $ED_{75}$, and $ED_{90}$
$ED_{50}$ ○–○
$ED_{75}$ □–□
$ED_{90}$ ▲–▲
$(D)_2$ (nM)
80
60
40
20
0
0.5
1.0
1.5
2.0
2.5
$(D)_1$ (μM)
$(ED)_{90}$ for $(D)_2$
$(ED)_{75}$ for $(D)_2$
$(ED)_{50}$ for $(D)_2$
$(ED)_{50}$ for $(D)_1$
$(ED)_{75}$ for $(D)_1$
$(ED)_{90}$ for $(D)_1$
Antagonism
Synergism
Additive Effect
Additive Effect
Additive Effect
Combination Data Point

FIG.5B

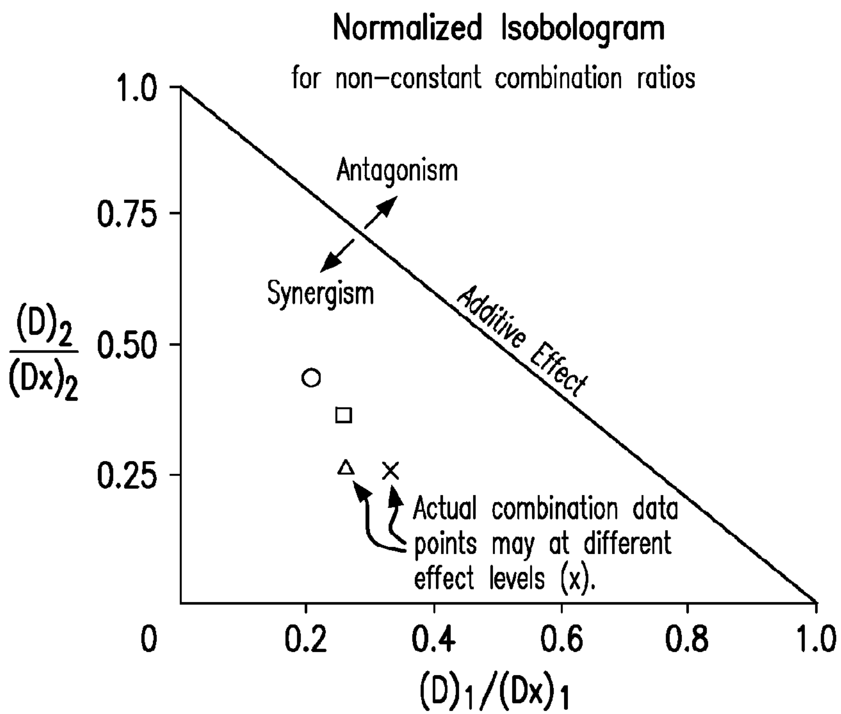
Normalized Isobologram
for non-constant combination ratios
1.0
0.75
0.50
0.25
0
$\frac{(D)_2}{(Dx)_2}$
Antagonism
Synergism
Additive Effect
Actual combination data
points may at different
effect levels (x).
0.2
0.4
0.6
0.8
1.0
$(D)_1/(Dx)_1$

FIG.5C

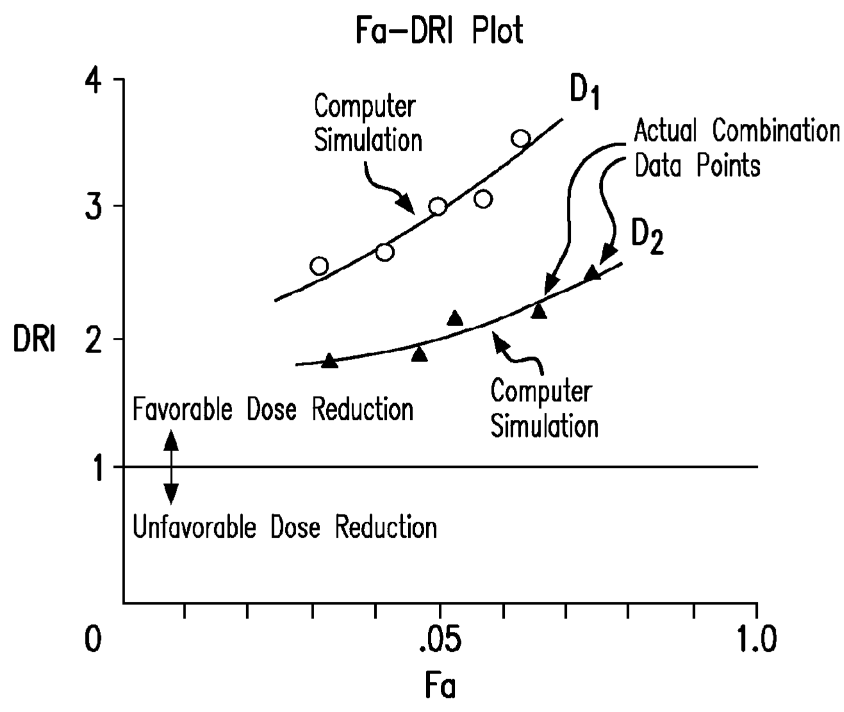
Fa–DRI Plot
4
3
2
1
0
DRI
Computer
Simulation
$D_1$
Actual Combination
Data Points
$D_2$
Computer
Simulation
Favorable Dose Reduction
Unfavorable Dose Reduction
.05
1.0
Fa

FIG.5D

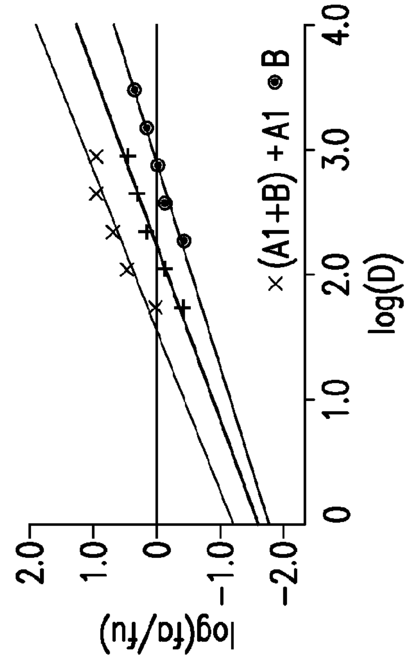


FIG.6A

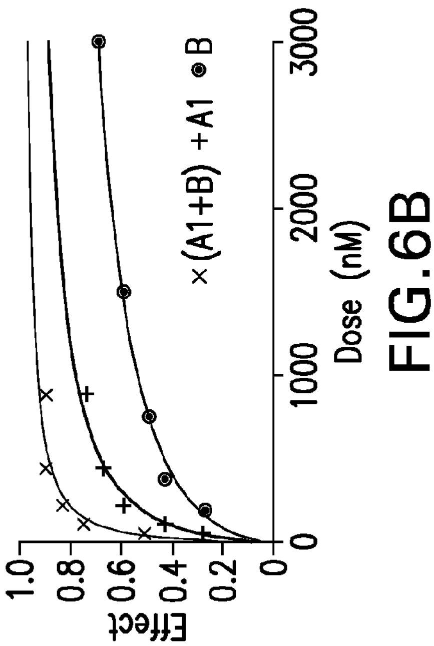


FIG.6B

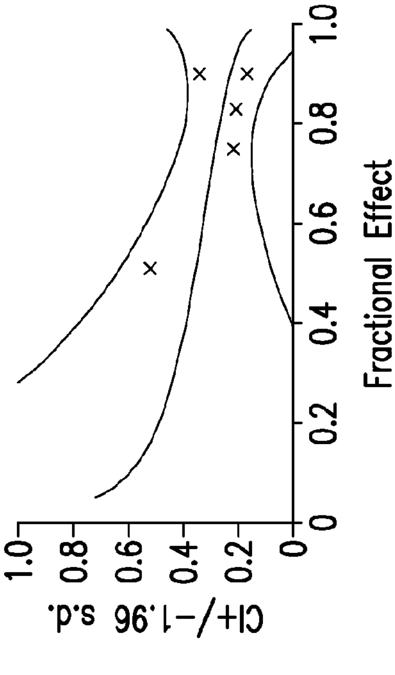


FIG.6C

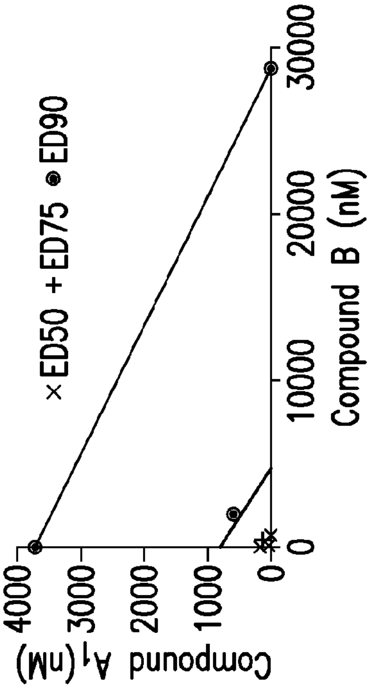


FIG.6E

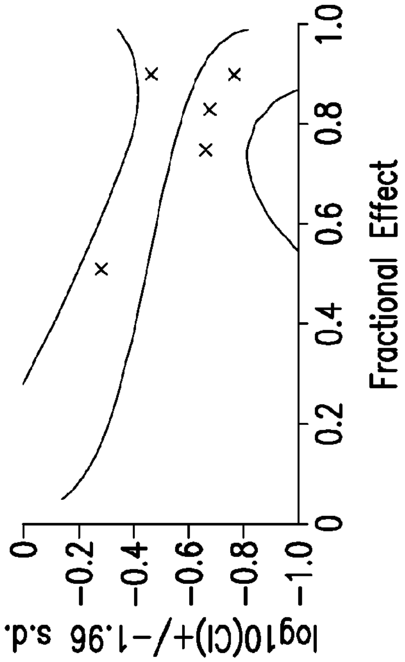


FIG.6D

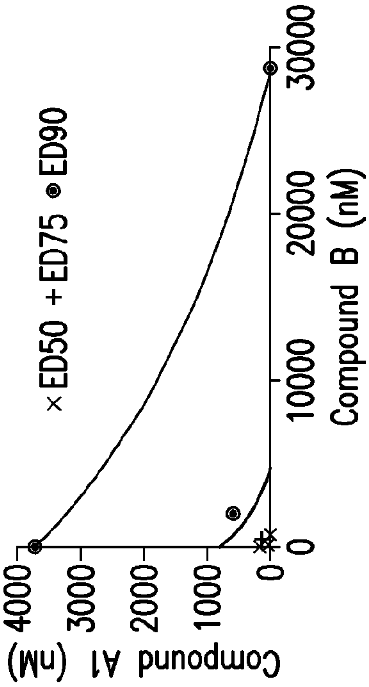


FIG.6F

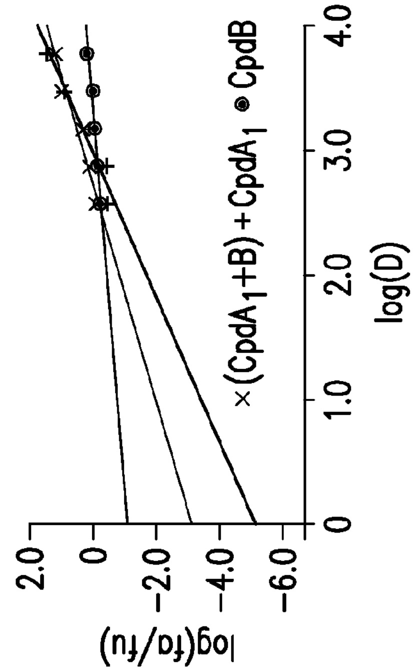


FIG. 7A

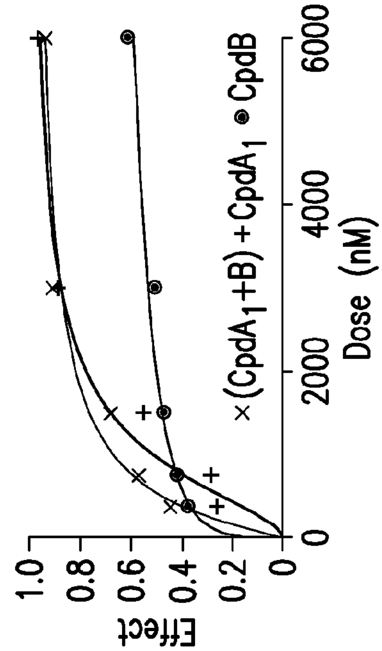


FIG. 7B

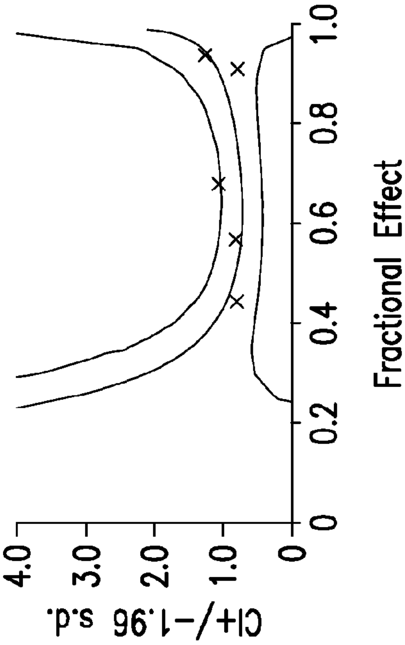


FIG. 7C

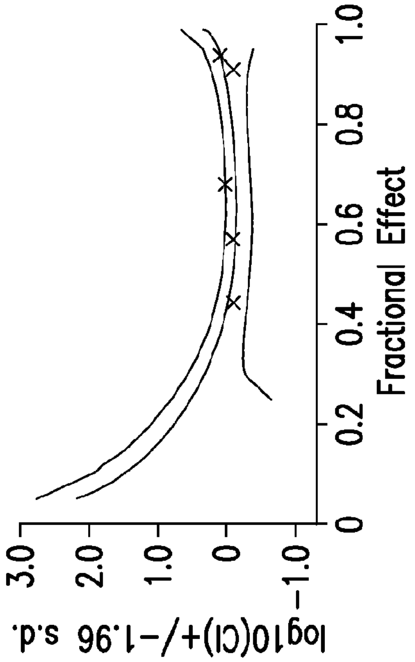


FIG.7D

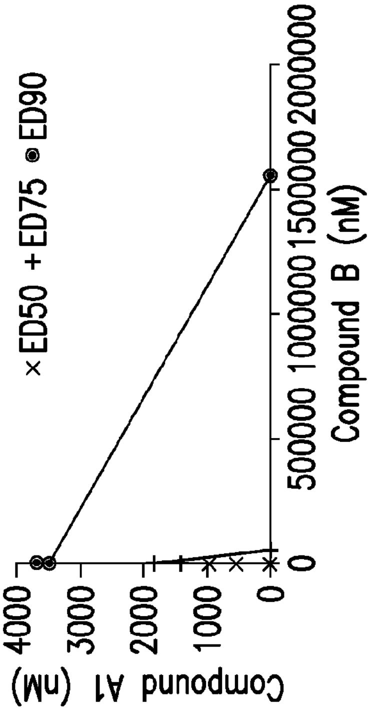


FIG.7E

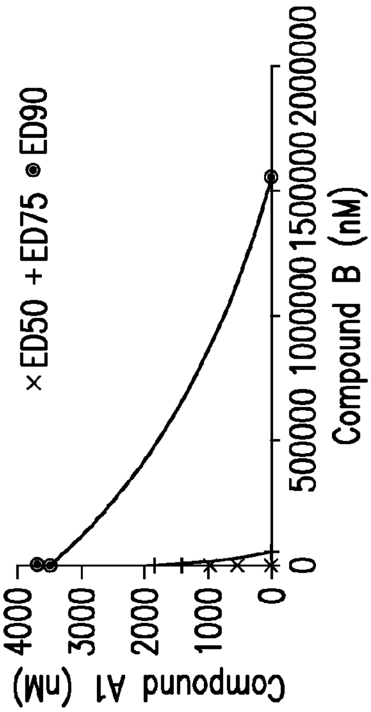


FIG.7F

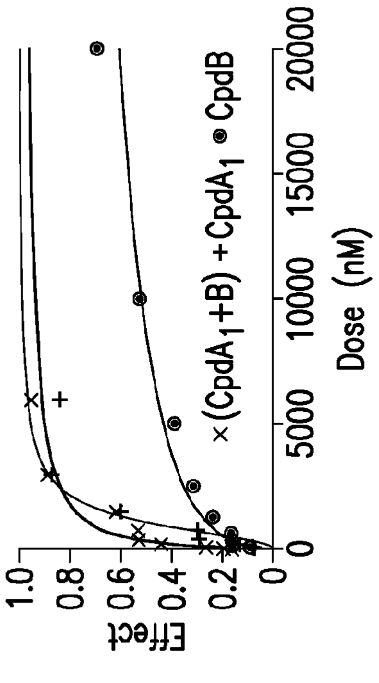


FIG.8B

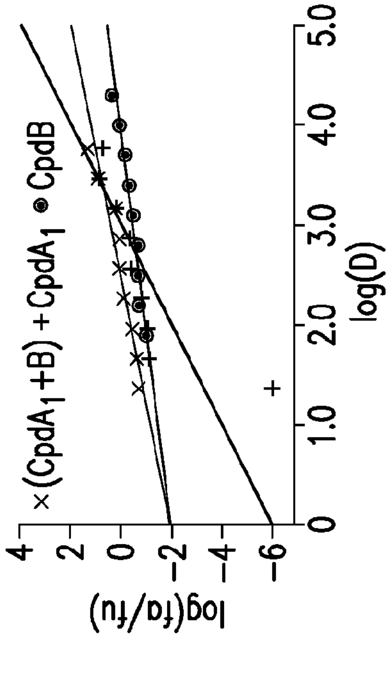


FIG.8A

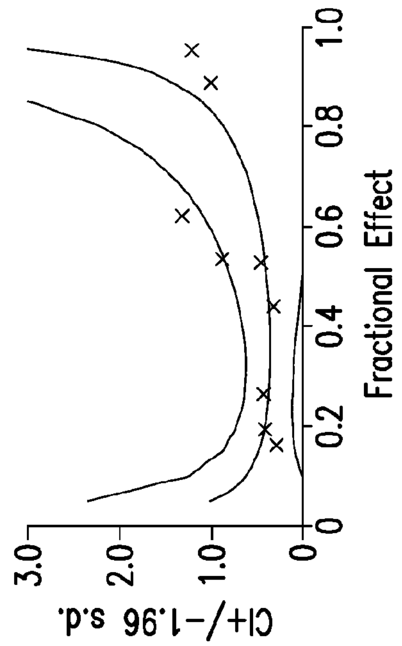


FIG.8C

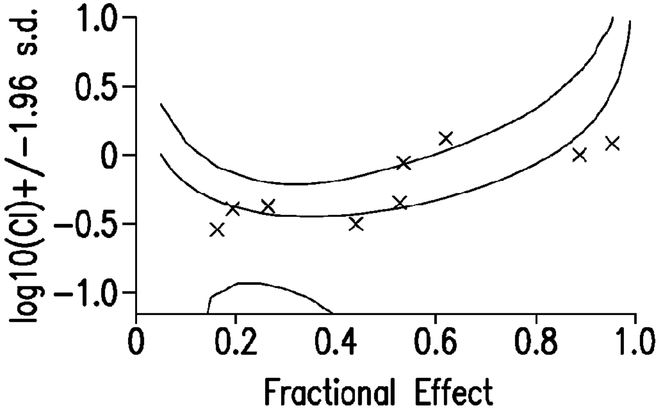


FIG.8D

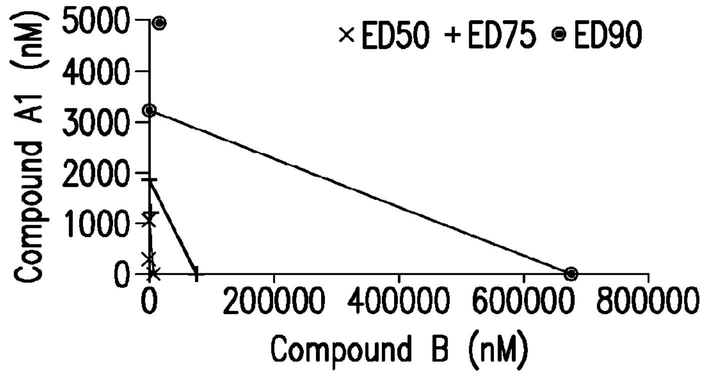


FIG.8E

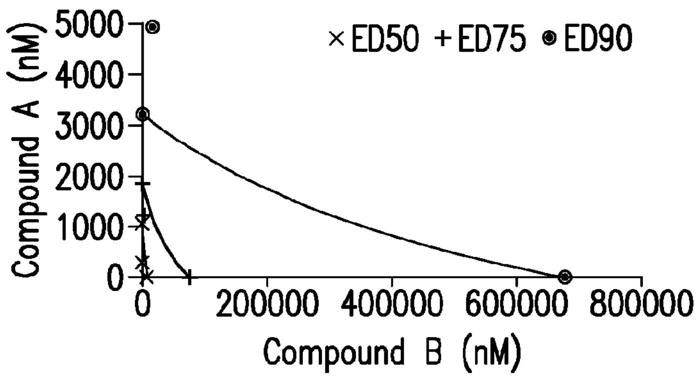


FIG.8F

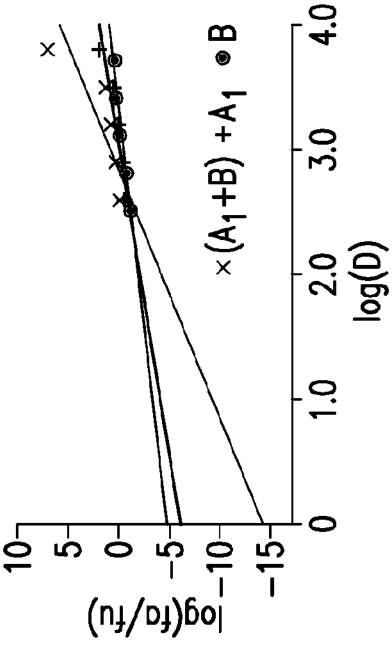


FIG.9A

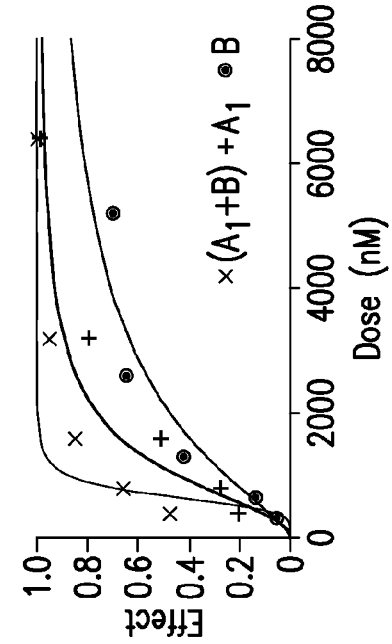


FIG.9B

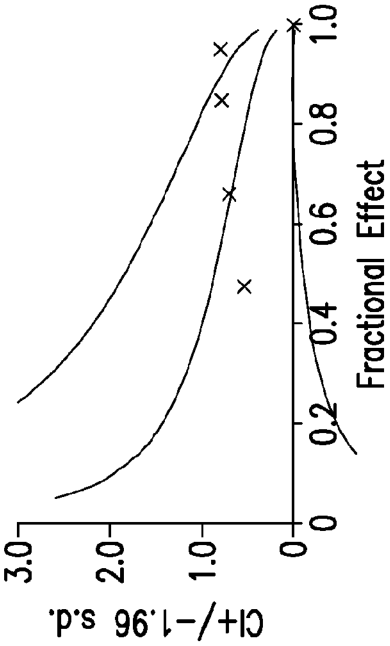


FIG.9C

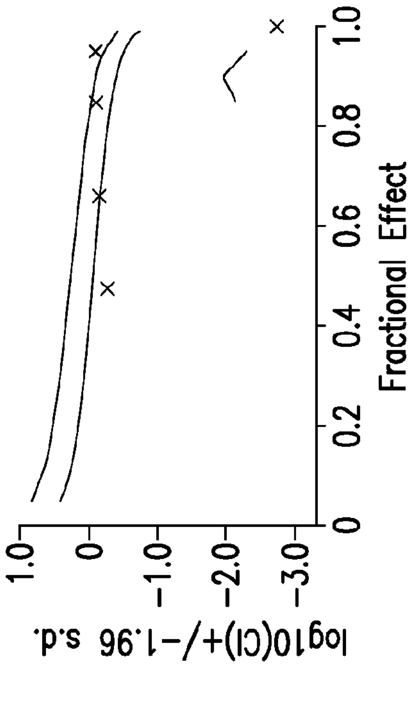


FIG.9D

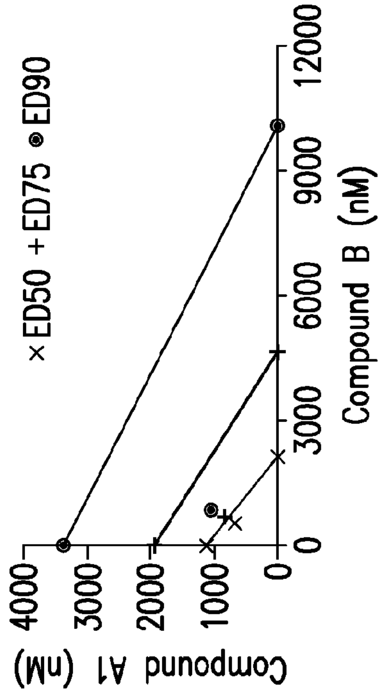


FIG.9E

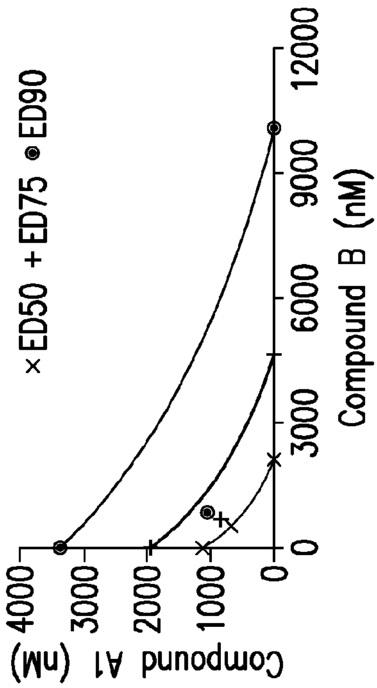


FIG.9F

Vehicle

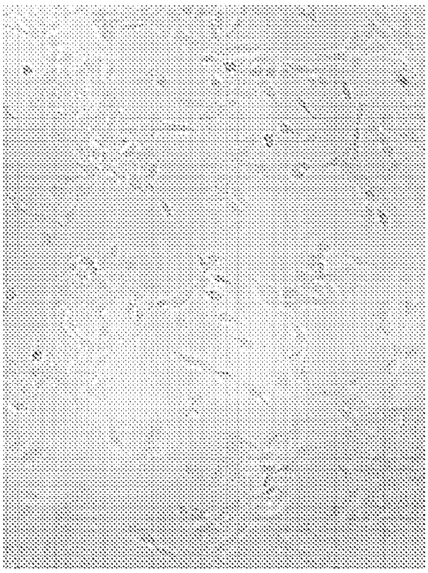

FIG.10A

Cmpd A1 IC50

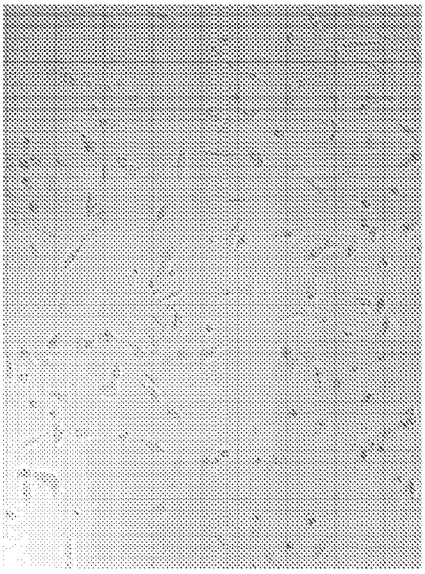

FIG.10B

Cmpd B IC50

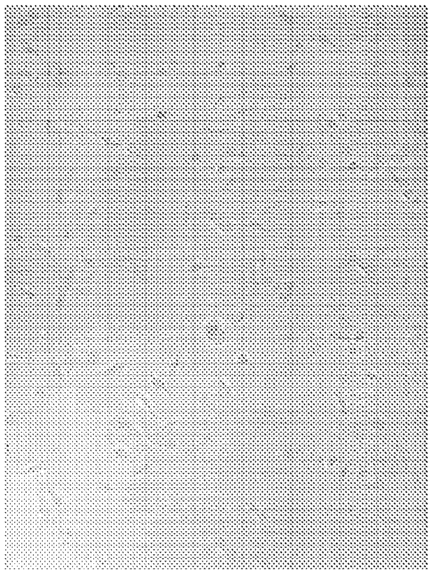

FIG.10C

Cmpd A1 IC50+Cmpd B IC50

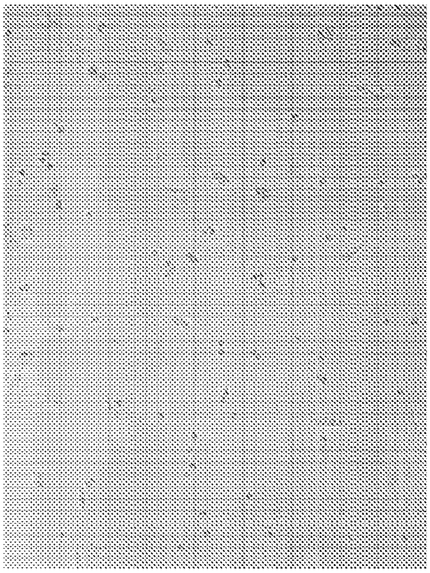

FIG.10D

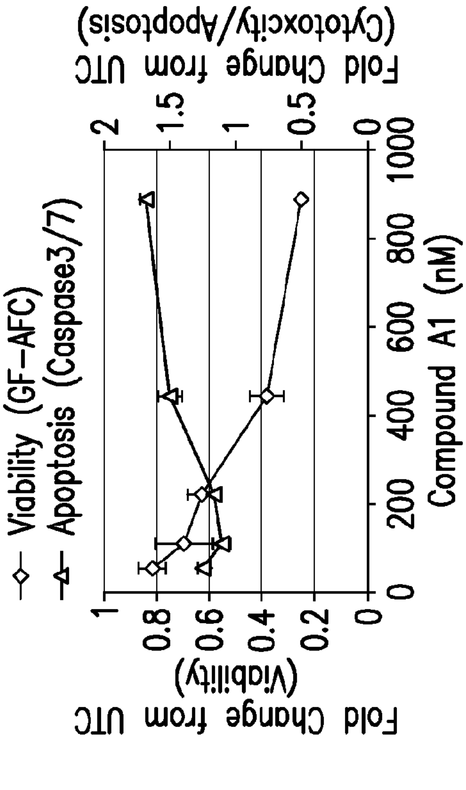


FIG.11A

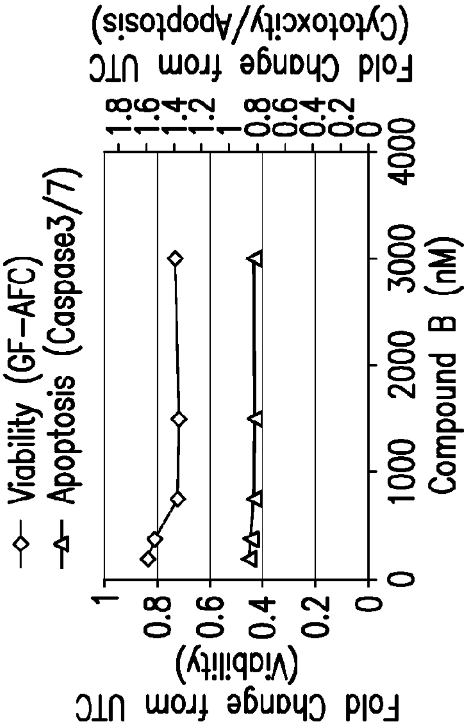


FIG.11B

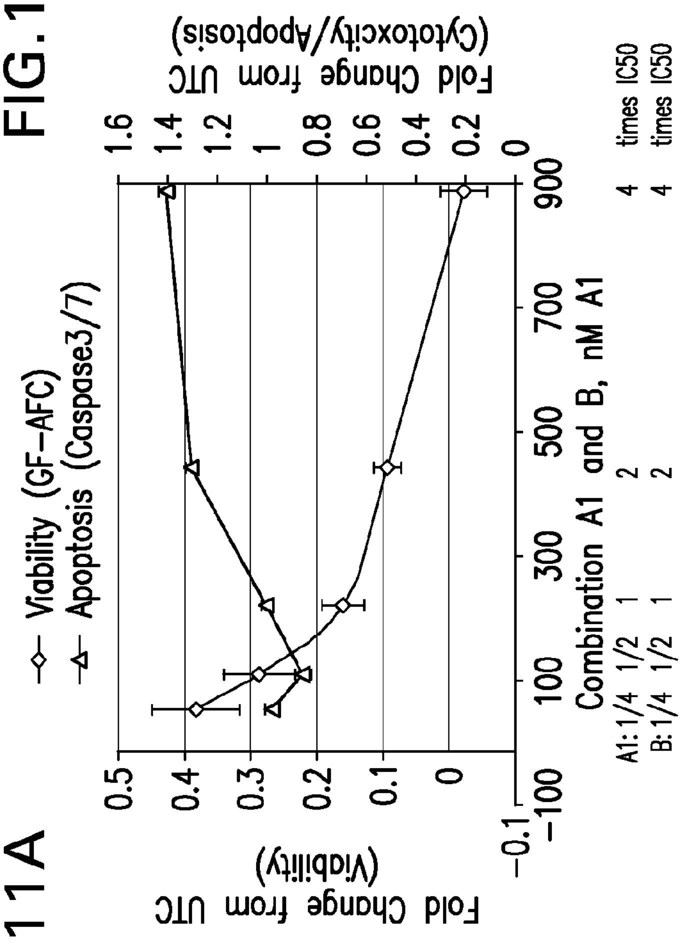


FIG.11C

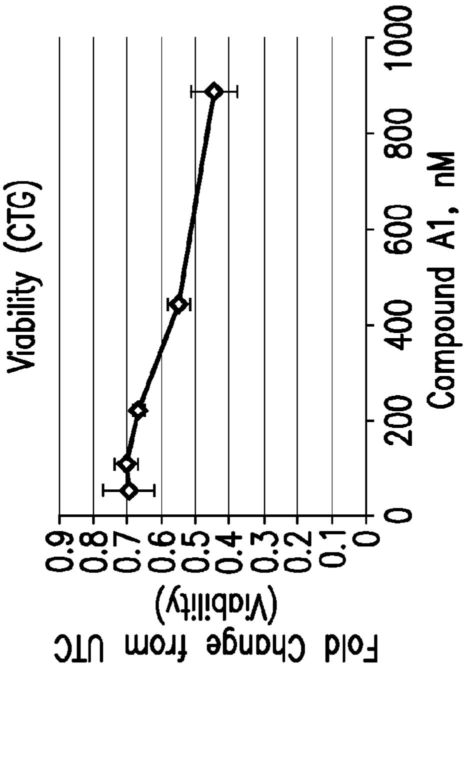


FIG.12A

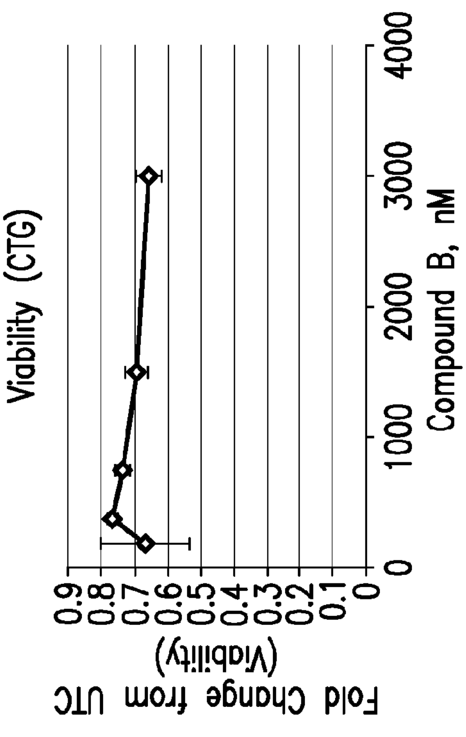


FIG.12B

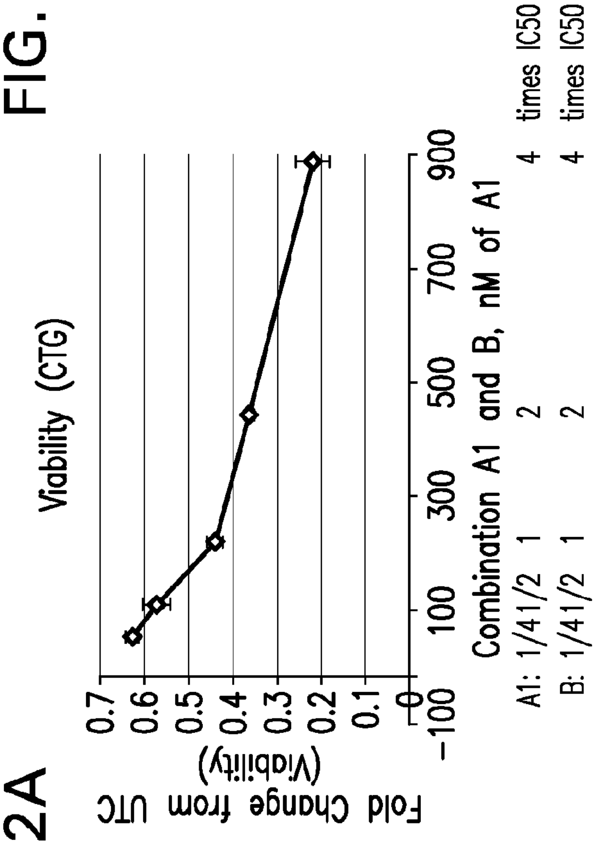


FIG.12C

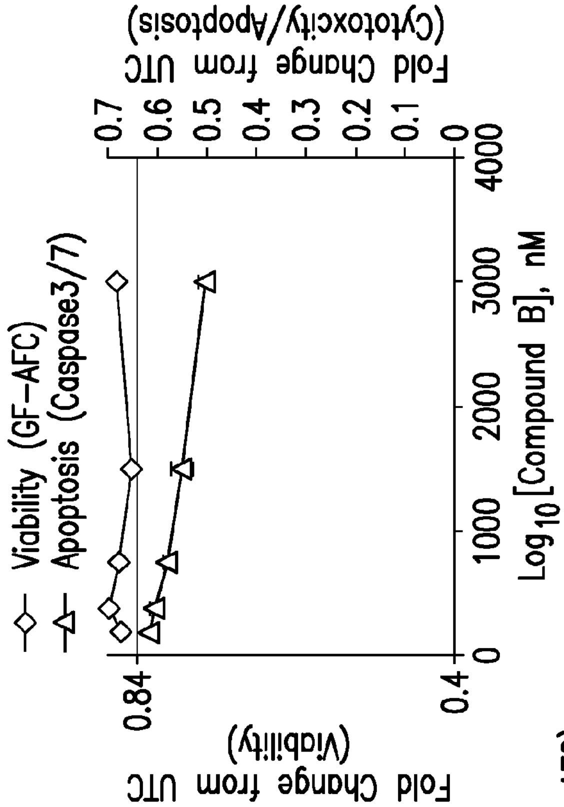


FIG.13B

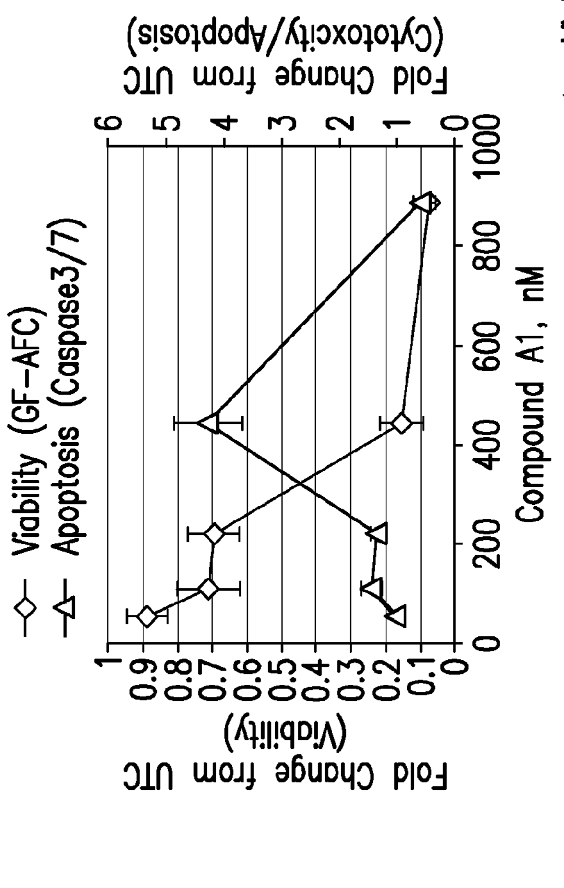


FIG.13A

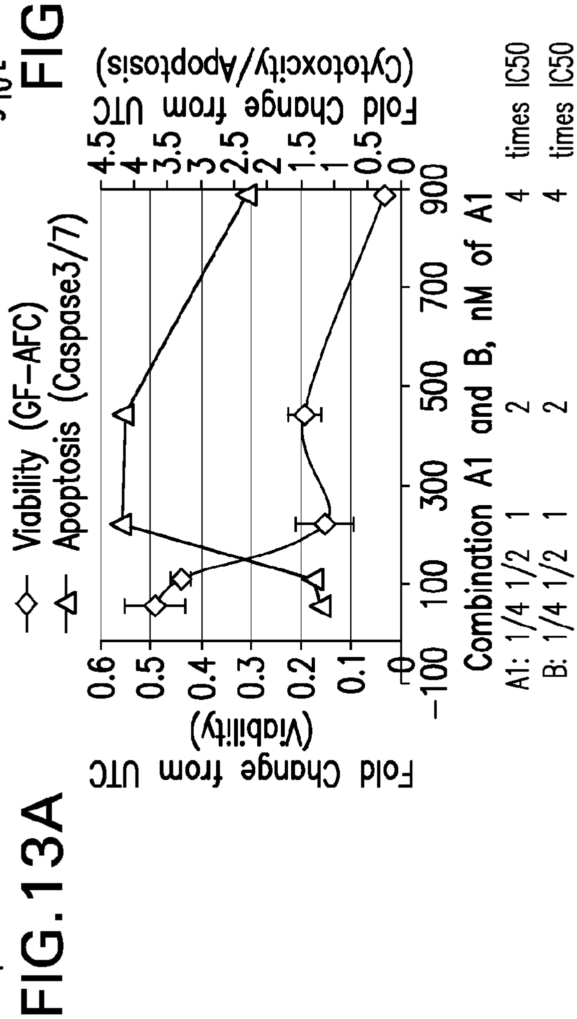


FIG.13C

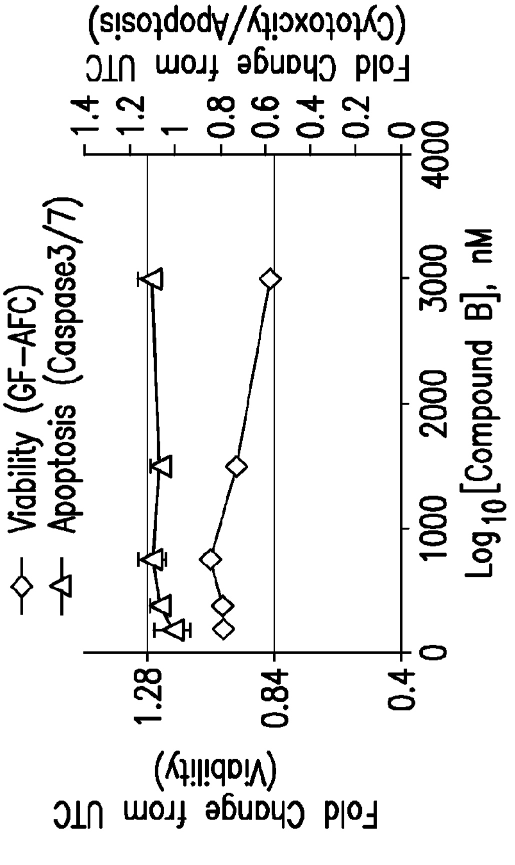


FIG.14B

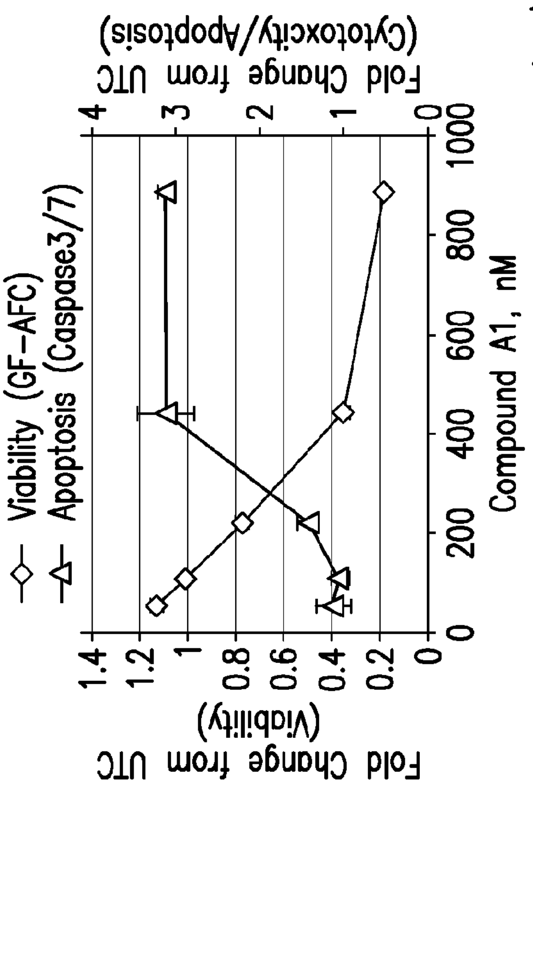


FIG.14A

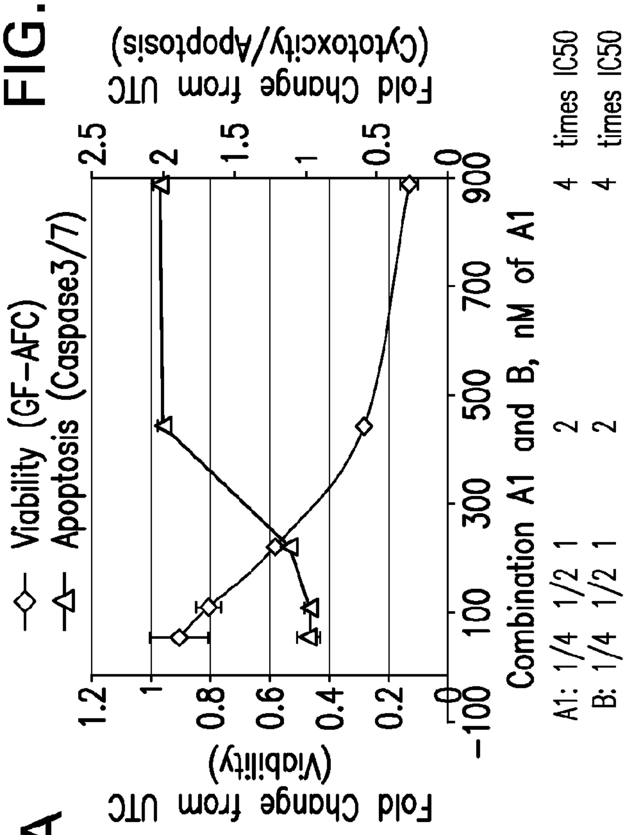


FIG.14C

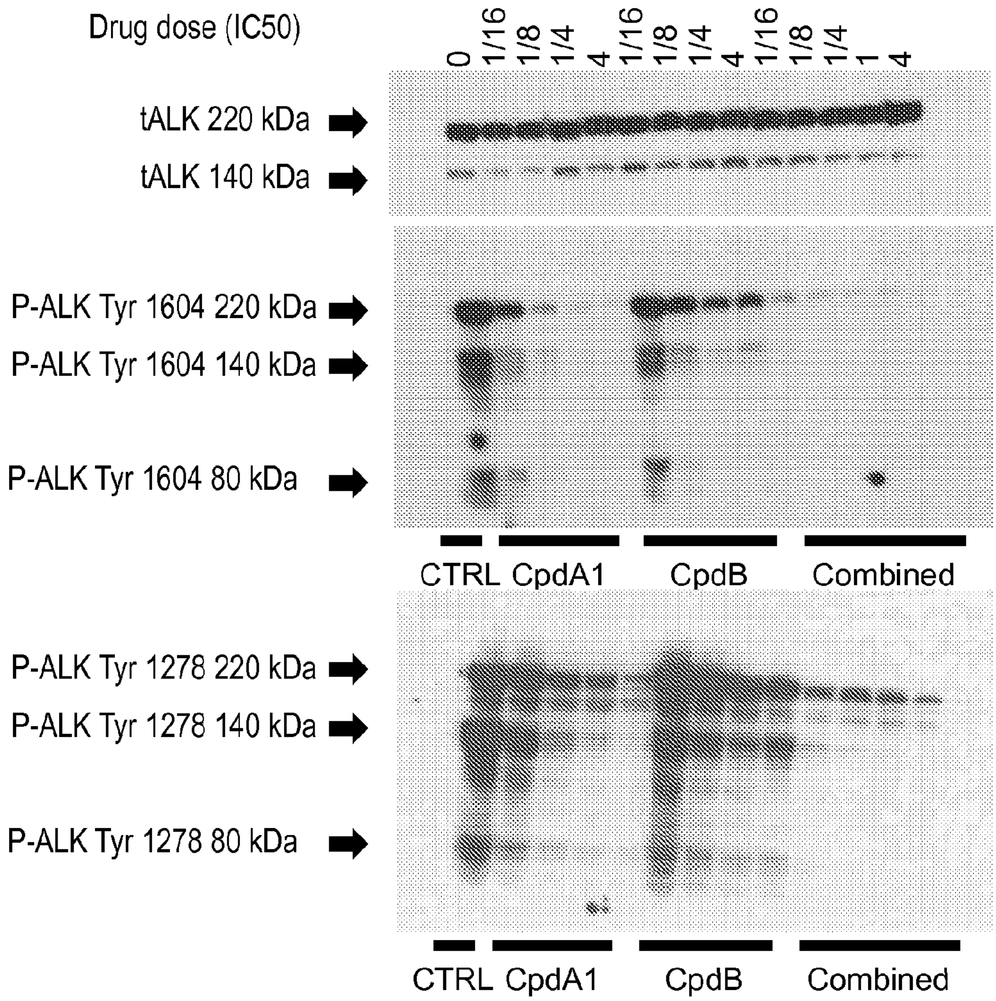
Drug dose (IC50)
0
1/16
1/8
1/4
4
1/16
1/8
1/4
4
1/16
1/8
1/4
1
4
tALK 220 kDa
tALK 140 kDa
P-ALK Tyr 1604 220 kDa
P-ALK Tyr 1604 140 kDa
P-ALK Tyr 1604 80 kDa
CTRL CpdA1 CpdB Combined
P-ALK Tyr 1278 220 kDa
P-ALK Tyr 1278 140 kDa
P-ALK Tyr 1278 80 kDa
CTRL CpdA1 CpdB Combined

FIG.15

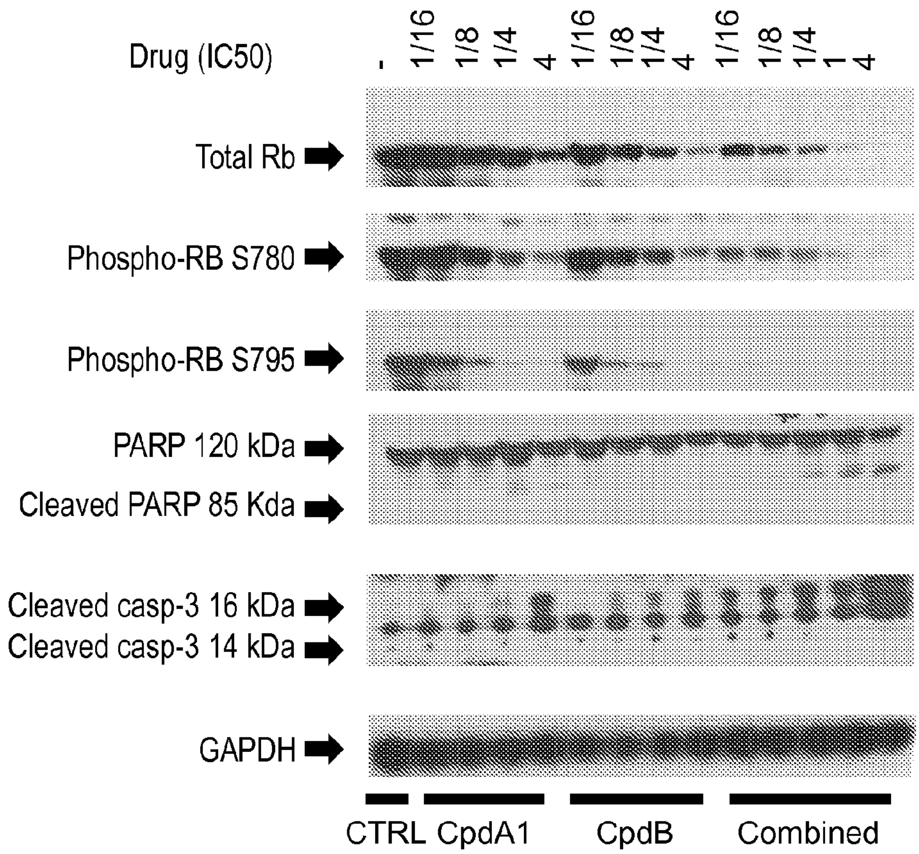
Drug (IC50)
- 1/16 1/8 1/4 4 1/16 1/8 1/4 4 1/16 1/8 1/4 1 4
Total Rb
Phospho-RB S780
Phospho-RB S795
PARP 120 kDa
Cleaved PARP 85 Kda
Cleaved casp-3 16 kDa
Cleaved casp-3 14 kDa
GAPDH
CTRL CpdA1 CpdB Combined

FIG.16

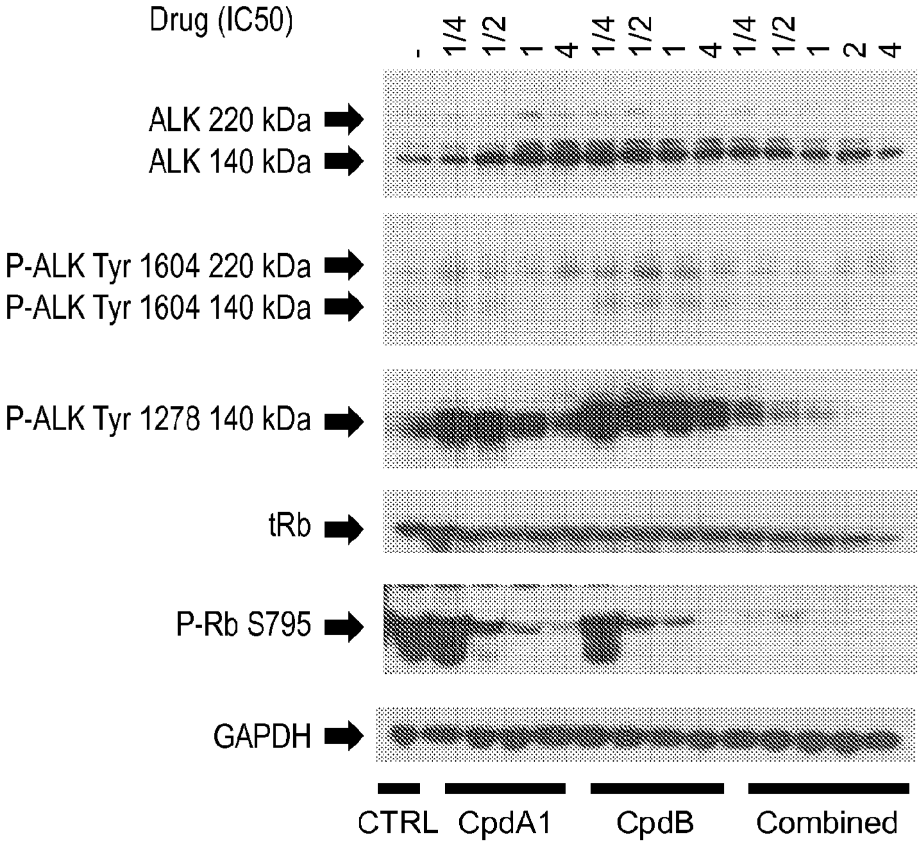
Drug (IC50)
- 1/4 1/2 1 4 1/4 1/2 1 4 1/4 1/2 1 2 4
ALK 220 kDa
ALK 140 kDa
P-ALK Tyr 1604 220 kDa
P-ALK Tyr 1604 140 kDa
P-ALK Tyr 1278 140 kDa
tRb
P-Rb S795
GAPDH
CTRL
CpdA1
CpdB
Combined

FIG.17

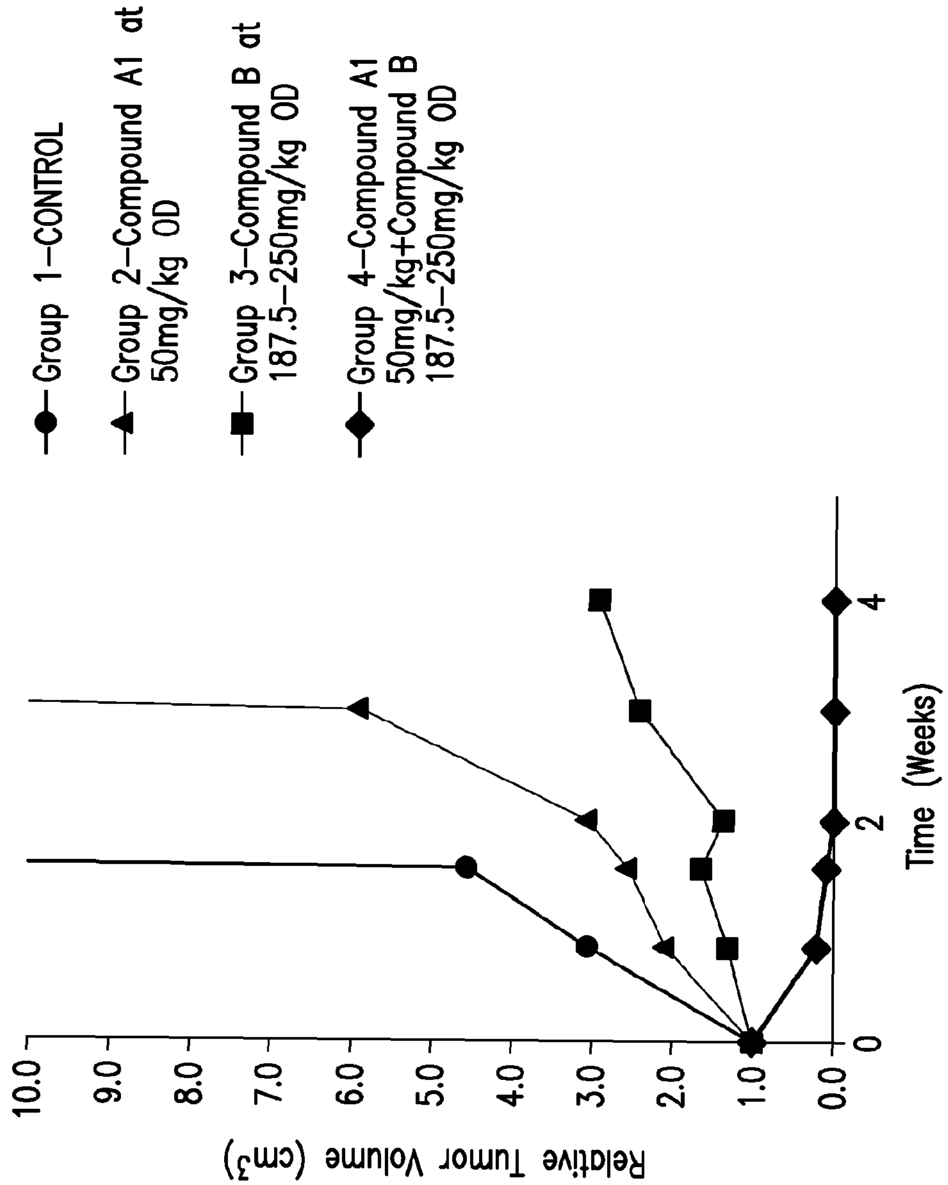
Group 1–CONTROL
Group 2–Compound A1 at 50mg/kg OD
Group 3–Compound B at 187.5–250mg/kg OD
Group 4–Compound A1 50mg/kg+Compound B 187.5–250mg/kg OD
Relative Tumor Volume (cm3)
10.0
9.0
8.0
7.0
6.0
5.0
4.0
3.0
2.0
1.0
0.0
0
2
4
Time (Weeks)

FIG.18

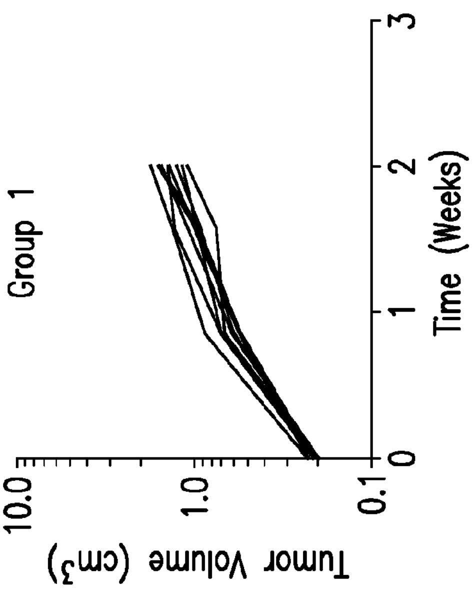


FIG.19A

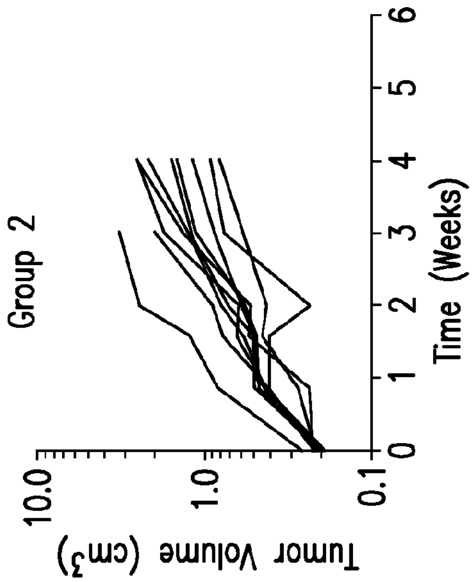


FIG.19B

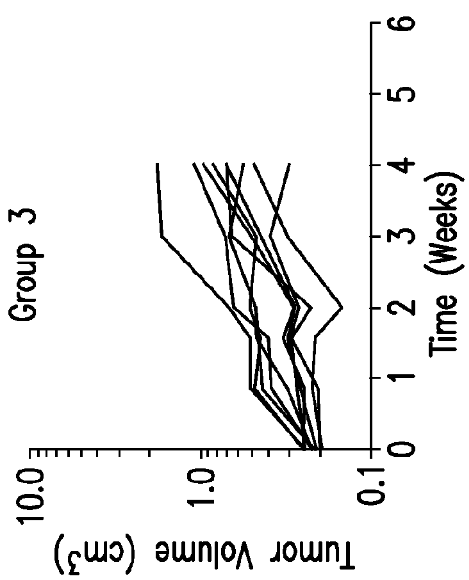


FIG.19C

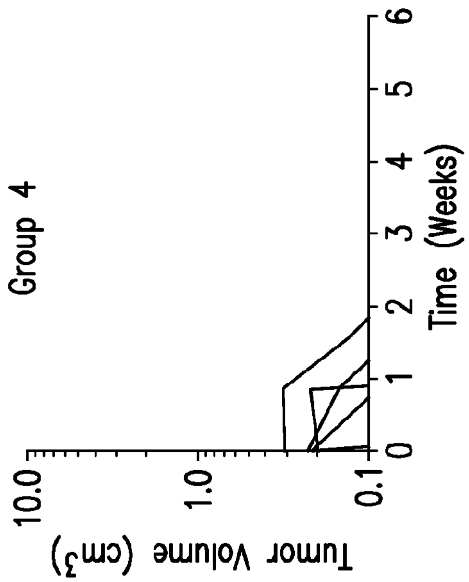


FIG.19D

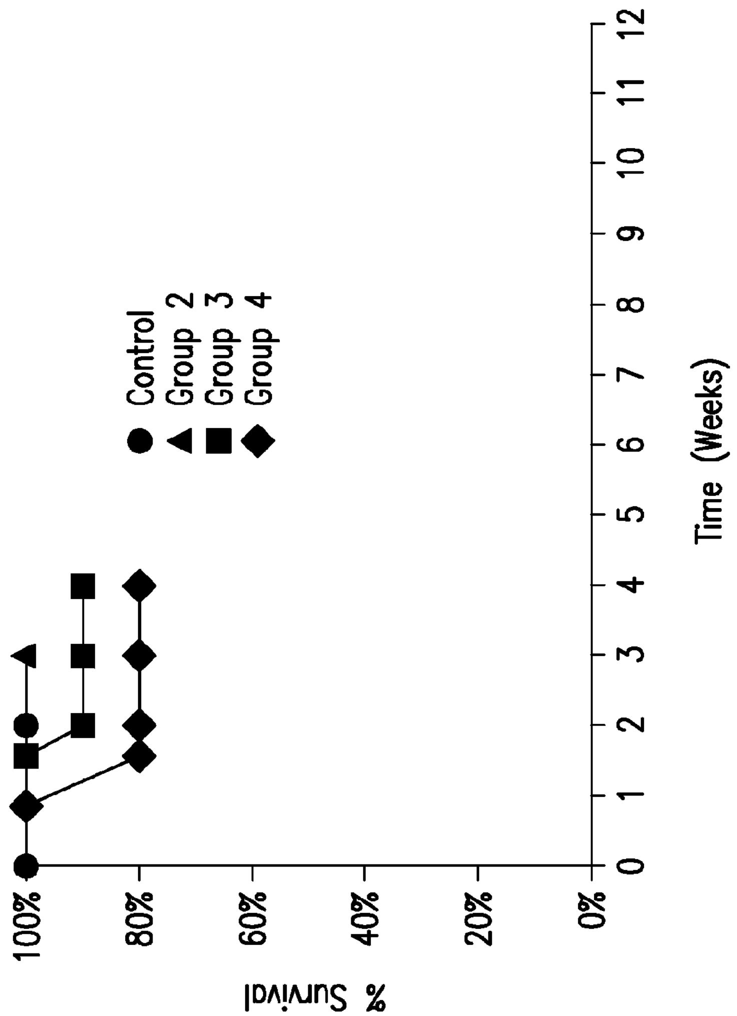

% Survival
100%
80%
60%
40%
20%
0%
0
1
2
3
4
5
6
7
8
9
10
11
12
Time (Weeks)
Control
Group 2
Group 3
Group 4


FIG.20

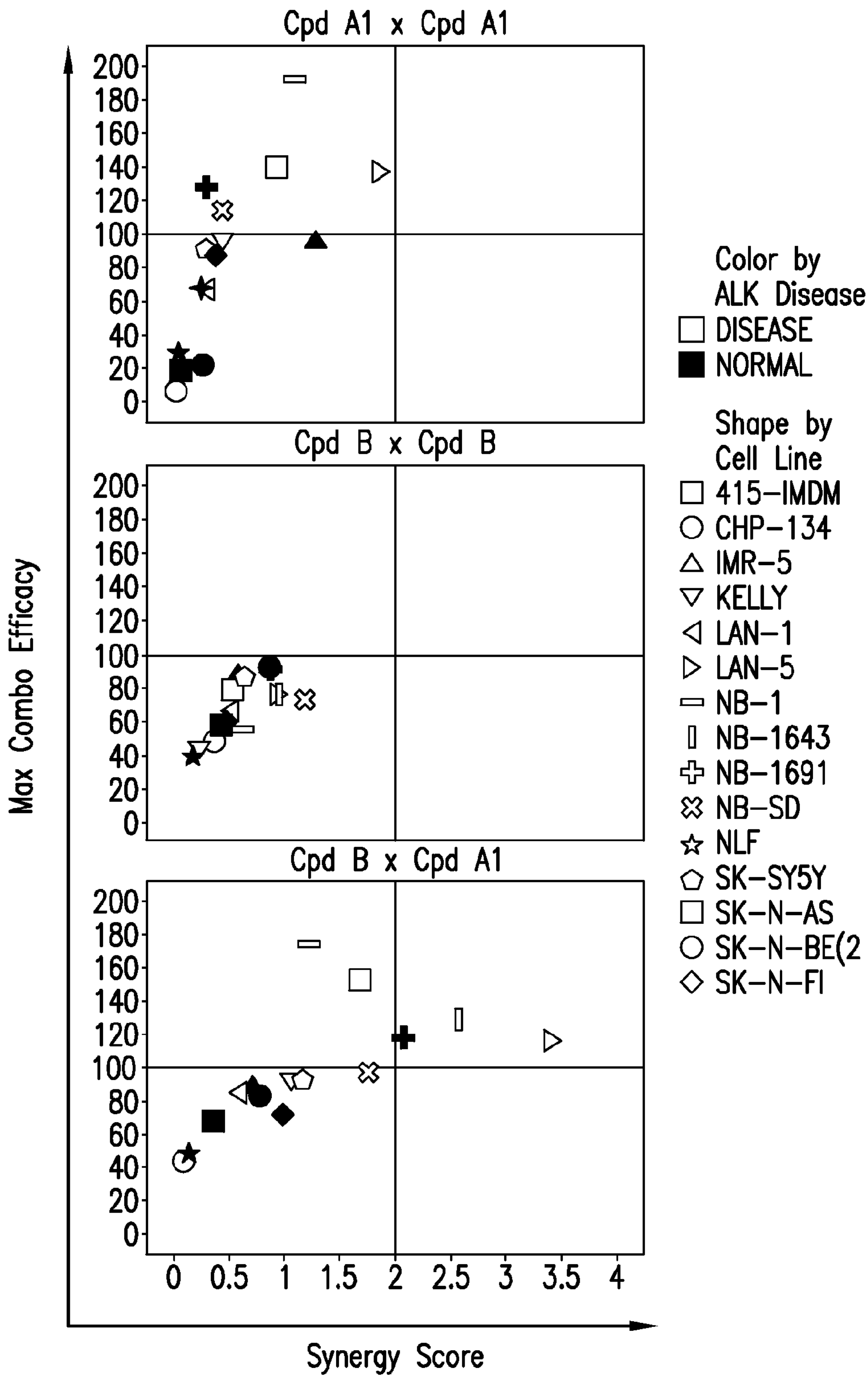

Cpd A1 x Cpd A1
Cpd B x Cpd B
Cpd B x Cpd A1
Max Combo Efficacy
Synergy Score
200
180
160
140
120
100
80
60
40
20
0
0
0.5
1
1.5
2
2.5
3
3.5
4
Color by ALK Disease
DISEASE
NORMAL
Shape by Cell Line
415-IMDM
CHP-134
IMR-5
KELLY
LAN-1
LAN-5
NB-1
NB-1643
NB-1691
NB-SD
NLF
SK-SY5Y
SK-N-AS
SK-N-BE(2
SK-N-FI


FIG.21

Inhibition (%)

| Compound A1 (μM) \ Compound B (μM) | 0 | | .0074 | | .066 | | .6 | | 5.4 |
|---|---|---|---|---|---|---|---|---|---|
| 1.8 | 40 | 32 | 40 | 41 | 43 | 47 | 51 | 50 | 54 |
| | 17 | 23 | 27 | 35 | 30 | 44 | 51 | 54 | 52 |
| .2 | 10 | −5 | 21 | 13 | 28 | 29 | 38 | 44 | 48 |
| | −6 | −19 | 0 | 9 | −1 | 25 | 34 | 41 | 47 |
| .022 | −9 | −22 | −11 | −9 | 1 | 9 | 26 | 27 | 34 |
| | −7 | −5 | −14 | 9 | 9 | 12 | 31 | 37 | 38 |
| .0025 | −18 | −22 | −4 | −2 | −5 | 15 | 37 | 39 | 24 |
| | −8 | 12 | −20 | 17 | −3 | 21 | 19 | 36 | 52 |
| 0 | −7 | −18 | −8 | 6 | 23 | 11 | 29 | 35 | 31 |

N=1−2

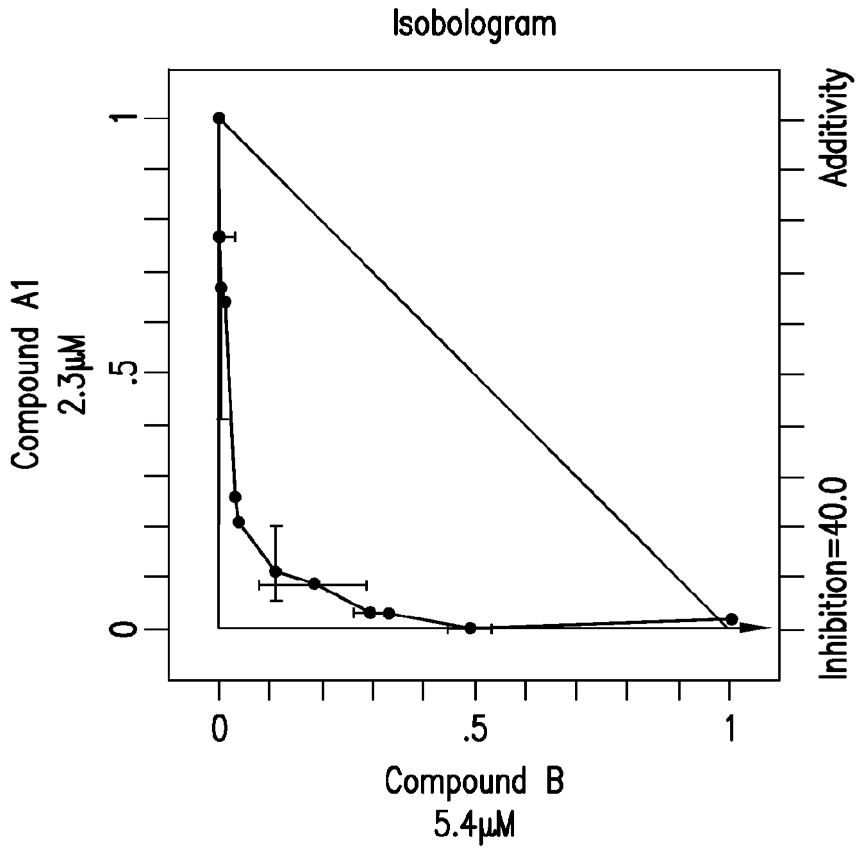


FIG.22A

Inhibition (%)

| Compound B (µM) \ Compound A2 (µM) | 0 | | .0074 | | .066 | | .6 | | 5.4 |
|---|---|---|---|---|---|---|---|---|---|
| 5.4 | 31 | 25 | 45 | 29 | 46 | 42 | 49 | 54 | 56 |
| | 36 | 35 | 25 | 33 | 34 | 35 | 52 | 50 | 59 |
| .6 | 23 | 19 | 27 | 23 | 29 | 37 | 45 | 50 | 58 |
| | 22 | 14 | 20 | 20 | 33 | 31 | 35 | 41 | 50 |
| .066 | −1 | 10 | 3 | 13 | 8 | 13 | 27 | 33 | 53 |
| | 2 | 3 | −12 | −1 | 6 | 10 | 13 | 27 | 46 |
| .0074 | −13 | 1 | −20 | 9 | −7 | 20 | 25 | 23 | 46 |
| | −10 | −8 | −3 | −13 | −13 | 13 | 17 | 36 | 40 |
| 0 | −5 | −10 | 0 | −4 | −4 | 5 | 27 | 28 | 47 |

N=2

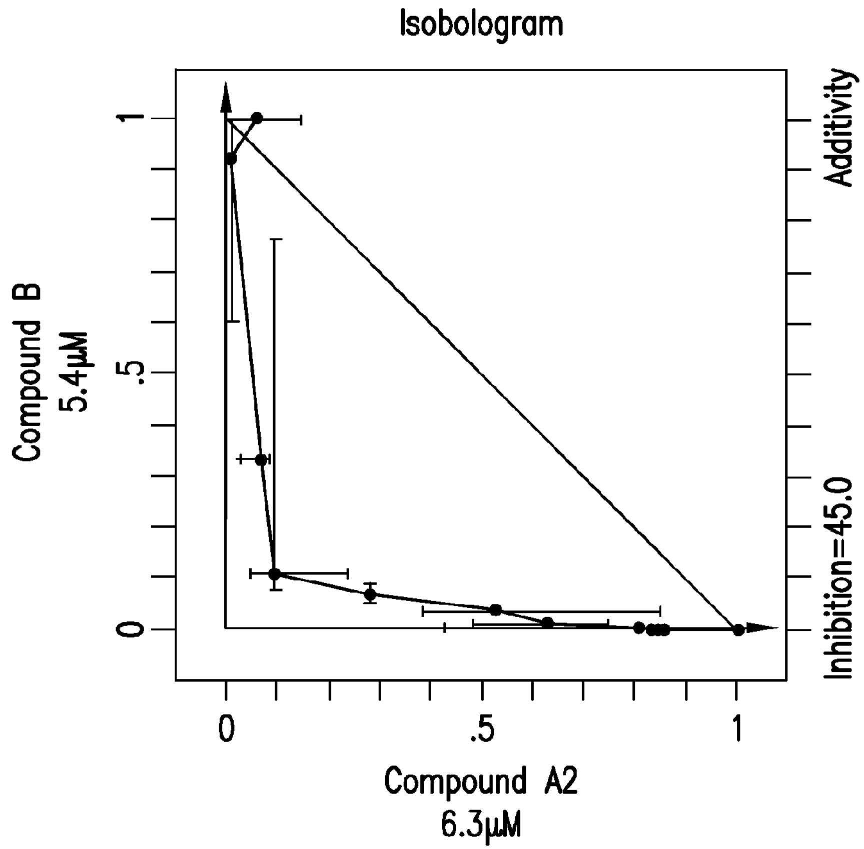


FIG.22B

Dose Matrix|KELLY

Inhibition (%)

| Compound B (µM) \ Compound A1 (µM) | 0 | 0.002 | 0.007 | 0.022 | 0.066 | 0.199 | 0.596 | 1.786 | 5.358 |
|---|---|---|---|---|---|---|---|---|---|
| 5.358 | 50 | 52 | 53 | 56 | 58 | 63 | 66 | 76 | 90 |
| 1.786 | 45 | 51 | 53 | 55 | 54 | 60 | 69 | 74 | 93 |
| 0.596 | 37 | 45 | 39 | 48 | 48 | 58 | 69 | 72 | 93 |
| 0.199 | 18 | 23 | 20 | 19 | 29 | 45 | 56 | 65 | 92 |
| 0.066 | 3 | 5 | 9 | 4 | 12 | 26 | 42 | 57 | 90 |
| 0.022 | −0 | −5 | −10 | −11 | 3 | 12 | 34 | 52 | 91 |
| 0.007 | −2 | 3 | 11 | −2 | −5 | 18 | 35 | 55 | 89 |
| 0.002 | 1 | 10 | 5 | 0 | 7 | 23 | 37 | 54 | 91 |
| 0 | − | 1 | −5 | 13 | 10 | 23 | 33 | 47 | 92 |

Loewe Excess Matrix|KELLY

Inhibition (%)

| Compound B (µM) \ Compound A1 (µM) | 0 | 0.002 | 0.007 | 0.022 | 0.066 | 0.199 | 0.596 | 1.786 | 5.358 |
|---|---|---|---|---|---|---|---|---|---|
| 5.358 | 1 | 3 | 3 | 6 | 9 | 14 | 17 | 23 | −0 |
| 1.786 | −2 | 4 | 6 | 8 | 7 | 13 | 21 | 21 | 3 |
| 0.596 | 0 | 8 | 3 | 11 | 11 | 18 | 24 | 19 | 3 |
| 0.199 | 1 | 6 | 2 | 0 | 7 | 18 | 18 | 12 | 1 |
| 0.066 | −1 | −0 | 2 | −6 | −2 | 5 | 8 | 4 | −1 |
| 0.022 | −1 | −7 | −14 | −18 | −8 | −7 | 2 | −2 | 1 |
| 0.007 | −3 | 1 | 7 | −9 | −15 | −0 | 3 | 2 | −1 |
| 0.002 | 1 | 8 | 2 | −6 | −3 | 5 | 6 | 1 | 0 |
| 0 | − | −1 | −9 | 7 | −1 | 5 | 1 | −6 | 2 |

FIG.23A

Dose Matrix|KELLY

Inhibition (%)

| Compound B (μM) \ Compound A2 (μM) | 0 | 0.002 | 0.007 | 0.022 | 0.066 | 0.199 | 0.596 | 1.786 | 5.358 |
|---|---|---|---|---|---|---|---|---|---|
| 5.358 | 52 | 55 | 56 | 57 | 62 | 61 | 65 | – | 77 |
| 1.786 | 47 | 46 | 57 | 58 | 58 | 53 | 69 | – | 78 |
| 0.596 | 43 | 40 | 40 | 45 | 49 | 54 | 58 | – | 76 |
| 0.199 | 19 | 31 | 27 | 30 | 35 | 39 | 47 | – | 73 |
| 0.066 | 11 | 8 | 6 | 22 | 22 | 21 | 36 | – | 69 |
| 0.022 | 4 | 6 | 3 | 14 | 9 | 21 | 25 | – | 64 |
| 0.007 | 7 | 11 | 12 | –3 | 6 | 22 | 26 | – | 57 |
| 0.002 | –8 | 7 | –8 | 5 | 13 | 20 | 32 | – | 59 |
| 0 | – | –6 | 1 | –4 | 2 | 19 | 20 | 42 | 64 |

Loewe Excess Matrix|KELLY

Inhibition (%)

| Compound B (μM) \ Compound A2 (μM) | 0 | 0.002 | 0.007 | 0.022 | 0.066 | 0.199 | 0.596 | 1.786 | 5.358 |
|---|---|---|---|---|---|---|---|---|---|
| 5.358 | 0 | 3 | 5 | 6 | 10 | 9 | 14 | – | 13 |
| 1.786 | –1 | –2 | 9 | 10 | 10 | 4 | 19 | – | 13 |
| 0.596 | 4 | 0 | 1 | 6 | 9 | 14 | 15 | – | 12 |
| 0.199 | –4 | 8 | 3 | 6 | 10 | 12 | 13 | – | 9 |
| 0.066 | 2 | –1 | –4 | 11 | 10 | 3 | 9 | – | 5 |
| 0.022 | 1 | 3 | –1 | 9 | 2 | 8 | 1 | – | –0 |
| 0.007 | 6 | 10 | 11 | –6 | 1 | 11 | 2 | – | –7 |
| 0.002 | –8 | 7 | –9 | 3 | 8 | 9 | 9 | – | –5 |
| 0 | – | –6 | 0 | –6 | –3 | 8 | –3 | –0 | 0 |

FIG.23B

Dose Matrix|NB-1

Inhibition (%)

| Compound B (µM) \ Compound A1 (µM) | 0 | 2.72E-4 | 8.17E-4 | 0.002 | 0.007 | 0.022 | 0.066 | 0.199 | 0.596 |
|---|---|---|---|---|---|---|---|---|---|
| 5.358 | 9 | 10 | 11 | – | – | – | – | – | – |
| 1.786 | 13 | 14 | 8 | – | – | – | – | – | – |
| 0.596 | 13 | 17 | 14 | – | – | – | – | – | – |
| 0.199 | 11 | 7 | −2 | 6 | −3 | 12 | 21 | 70 | 84 |
| 0.066 | 9 | 3 | −0 | 4 | 3 | −6 | 15 | 71 | 86 |
| 0.022 | 8 | 3 | 2 | −1 | 0 | 5 | 31 | 73 | 85 |
| 0.007 | 4 | 4 | −1 | 5 | −4 | −1 | 27 | 73 | 85 |
| 0.002 | 13 | 13 | 16 | 5 | 5 | 5 | 20 | 74 | 86 |
| 0 | – | 11 | 11 | 9 | 3 | 5 | 27 | 75 | 84 |

Loewe Excess Matrix|NB-1

Inhibition (%)

| Compound B (µM) \ Compound A1 (µM) | 0 | 2.72E-4 | 8.17E-4 | 0.002 | 0.007 | 0.022 | 0.066 | 0.199 | 0.596 |
|---|---|---|---|---|---|---|---|---|---|
| 5.358 | −1 | −1 | 1 | – | – | – | – | – | – |
| 1.786 | 3 | 3 | −2 | – | – | – | – | – | – |
| 0.596 | 3 | 7 | 3 | – | – | – | – | – | – |
| 0.199 | 1 | −3 | −12 | −5 | −13 | 1 | −7 | −4 | −0 |
| 0.066 | −1 | −7 | −10 | −7 | −7 | −16 | −13 | −3 | 2 |
| 0.022 | −2 | −7 | −8 | −10 | −9 | −5 | 3 | −1 | 1 |
| 0.007 | −6 | −6 | −11 | −5 | −14 | −11 | −0 | −0 | 1 |
| 0.002 | 4 | 4 | 7 | −4 | −4 | −4 | −8 | 0 | 2 |
| 0 | – | 11 | 11 | 9 | 3 | 2 | −1 | 1 | −0 |

FIG.23C

Dose Matrix|NB-1

Inhibition (%)

| Compound B (µM) \ Compound A2 (µM) | 0 | 2.72E−4 | 8.17E−4 | 0.002 | 0.007 | 0.022 | 0.066 | 0.199 | 0.596 |
|---|---|---|---|---|---|---|---|---|---|
| 5.358 | 5 | 4 | 11 | 10 | 11 | 21 | 55 | 73 | 84 |
| 1.786 | 5 | − | − | − | − | − | − | − | − |
| 0.596 | 4 | 10 | 7 | 10 | 11 | 22 | 49 | 71 | 84 |
| 0.199 | −6 | 0 | −4 | −2 | −2 | −7 | 33 | 71 | 81 |
| 0.066 | −3 | 2 | 2 | −7 | −0 | −4 | 38 | 69 | 79 |
| 0.022 | 3 | 4 | −4 | 1 | −2 | −3 | 36 | 66 | 82 |
| 0.007 | −0 | −3 | −0 | 2 | 3 | −0 | 31 | 65 | 81 |
| 0.002 | −1 | −5 | 3 | −5 | −3 | −4 | 28 | 67 | 79 |
| 0 | − | −0 | −1 | −10 | −8 | −4 | 29 | 68 | 80 |

Loewe Excess Matrix|NB-1

Inhibition (%)

| Compound B (µM) \ Compound A2 (µM) | 0 | 2.72E−4 | 8.17E−4 | 0.002 | 0.007 | 0.022 | 0.066 | 0.199 | 0.596 |
|---|---|---|---|---|---|---|---|---|---|
| 5.358 | 0 | −1 | 6 | 5 | 6 | 16 | 28 | 3 | 5 |
| 1.786 | −0 | − | − | − | − | − | − | − | − |
| 0.596 | 0 | 6 | 2 | 5 | 6 | 17 | 22 | 1 | 5 |
| 0.199 | −6 | 0 | −4 | −2 | −3 | −11 | 6 | 1 | 2 |
| 0.066 | −3 | 2 | 2 | −7 | −1 | −7 | 12 | −1 | 0 |
| 0.022 | 3 | 4 | −4 | 1 | −3 | −6 | 9 | −4 | 3 |
| 0.007 | −0 | −3 | −0 | 2 | 2 | −3 | 4 | −5 | 3 |
| 0.002 | −1 | −5 | 3 | −5 | −3 | −7 | 1 | −3 | 0 |
| 0 | − | −0 | −1 | −10 | −8 | −6 | 2 | −3 | 1 |

FIG.23D

Dose Matrix|KELLY

Inhibition (%)

| Compound B (μM) \ Compound A1 (μM) | 0 | 0.001 | 0.004 | 0.011 | 0.033 | 0.099 | 0.298 | 0.893 | 2.679 |
|---|---|---|---|---|---|---|---|---|---|
| 2.679 | 51 | 53 | 53 | 49 | 51 | 45 | 52 | 52 | 68 |
| 0.893 | 41 | 39 | 43 | 37 | 42 | 43 | 43 | 53 | 68 |
| 0.298 | 30 | 25 | 31 | 31 | 32 | 34 | 30 | 37 | 61 |
| 0.099 | 26 | 25 | 26 | 36 | 21 | 30 | 24 | 32 | 61 |
| 0.033 | 9 | 22 | 7 | 19 | 13 | 20 | 13 | 24 | 51 |
| 0.011 | 4 | 14 | 5 | 12 | 13 | 13 | 25 | 6 | 56 |
| 0.004 | 1 | 7 | 8 | 8 | −7 | 4 | 10 | 9 | 55 |
| 0.001 | 9 | 15 | 5 | 2 | 8 | 3 | 3 | 16 | 58 |
| 0 | – | 3 | 4 | 5 | 2 | −6 | 9 | 9 | 54 |

Loewe Excess Matrix|KELLY

Inhibition (%)

| Compound B (μM) \ Compound A1 (μM) | 0 | 0.001 | 0.004 | 0.011 | 0.033 | 0.099 | 0.298 | 0.893 | 2.679 |
|---|---|---|---|---|---|---|---|---|---|
| 2.679 | 0 | 2 | 2 | −2 | −0 | −6 | −1 | −2 | 14 |
| 0.893 | −1 | −3 | 1 | −5 | 0 | 1 | −0 | 6 | 13 |
| 0.298 | −1 | −6 | −0 | 0 | 1 | 2 | −3 | 0 | 6 |
| 0.099 | 5 | 5 | 5 | 16 | 1 | 10 | 2 | 4 | 6 |
| 0.033 | −3 | 9 | −5 | 7 | 1 | 7 | −1 | 4 | −3 |
| 0.011 | −2 | 7 | −2 | 5 | 6 | 5 | 16 | −9 | 2 |
| 0.004 | −3 | 3 | 5 | 4 | −10 | −0 | 4 | −4 | 1 |
| 0.001 | 8 | 13 | 3 | 0 | 6 | 1 | −1 | 5 | 3 |
| 0 | – | 3 | 4 | 5 | 2 | −6 | 7 | −2 | −0 |

FIG.24A

Dose Matrix|KELLY

Inhibition (%)

| Compound B (µM) \ Compound A2 (µM) | 0 | 0.001 | 0.004 | 0.011 | 0.033 | 0.099 | 0.298 | 0.893 | 2.679 |
|---|---|---|---|---|---|---|---|---|---|
| 2.679 | 48 | 54 | 46 | 51 | 53 | 49 | 57 | 60 | 68 |
| 0.893 | 36 | 39 | 36 | 39 | 38 | 45 | 41 | 49 | 66 |
| 0.298 | 26 | 32 | 34 | 35 | 21 | 30 | 35 | 44 | 52 |
| 0.099 | 22 | 31 | 18 | 23 | 23 | 27 | 24 | 41 | 47 |
| 0.033 | 18 | 21 | 12 | 11 | 14 | 21 | 14 | 26 | 43 |
| 0.011 | 5 | 8 | 2 | 7 | −2 | 6 | 9 | 23 | 40 |
| 0.004 | 8 | 4 | 4 | −1 | 6 | −2 | 19 | 15 | 28 |
| 0.001 | 6 | −0 | 10 | 5 | −4 | 7 | −2 | 21 | 34 |
| 0 | – | 3 | −2 | 5 | 3 | 7 | 7 | 8 | 28 |

Loewe Excess Matrix|KELLY

Inhibition (%)

| Compound B (µM) \ Compound A2 (µM) | 0 | 0.001 | 0.004 | 0.011 | 0.033 | 0.099 | 0.298 | 0.893 | 2.679 |
|---|---|---|---|---|---|---|---|---|---|
| 2.679 | 1 | 7 | −2 | 4 | 6 | 1 | 10 | 13 | 21 |
| 0.893 | −3 | 1 | −2 | −0 | −1 | 6 | 2 | 9 | 23 |
| 0.298 | −4 | 3 | 5 | 6 | −8 | 0 | 5 | 12 | 15 |
| 0.099 | 2 | 10 | −3 | 2 | 2 | 6 | 2 | 17 | 15 |
| 0.033 | 4 | 7 | −1 | −3 | 0 | 7 | −2 | 7 | 13 |
| 0.011 | −3 | −1 | −7 | −2 | −11 | −4 | −2 | 7 | 12 |
| 0.004 | 3 | −1 | −1 | −6 | 1 | −8 | 11 | 1 | −0 |
| 0.001 | 3 | −3 | 7 | 2 | −8 | 2 | −9 | 8 | 7 |
| 0 | – | 3 | −2 | 5 | 1 | 5 | 0 | −5 | 1 |

FIG.24B

Dose Matrix|NB-1

Inhibition (%)

| Compound B (μM) \ Compound A1 (μM) | 0 | 1.36E-4 | 4.08E-4 | 0.001 | 0.004 | 0.011 | 0.033 | 0.099 | 0.298 |
|---|---|---|---|---|---|---|---|---|---|
| 2.679 | 6 | 6 | −4 | 1 | −1 | 4 | 9 | 27 | 41 |
| 0.893 | −2 | −3 | 5 | 2 | 1 | 6 | 8 | 19 | 24 |
| 0.298 | 6 | −1 | 4 | 3 | 3 | 9 | 8 | 17 | 25 |
| 0.099 | 14 | 12 | 10 | 10 | 5 | 11 | 14 | 22 | 31 |
| 0.033 | 2 | 4 | 3 | 8 | 0 | 3 | 7 | 17 | 28 |
| 0.011 | 10 | 5 | 9 | 7 | 2 | 7 | 10 | 13 | 34 |
| 0.004 | 10 | 9 | 9 | 2 | 4 | 3 | 11 | 24 | 32 |
| 0.001 | 14 | 14 | 9 | 10 | 6 | 11 | 13 | 22 | 39 |
| 0 | – | 10 | 1 | 6 | −0 | 6 | 12 | 17 | 37 |

Loewe Excess Matrix|NB-1

Inhibition (%)

| Compound B (μM) \ Compound A1 (μM) | 0 | 1.36E-4 | 4.08E-4 | 0.001 | 0.004 | 0.011 | 0.033 | 0.099 | 0.298 |
|---|---|---|---|---|---|---|---|---|---|
| 2.679 | −2 | −2 | −11 | −6 | −9 | −3 | −2 | 7 | 4 |
| 0.893 | −9 | −10 | −2 | −5 | −7 | −2 | −3 | −1 | −12 |
| 0.298 | −1 | −8 | −3 | −4 | −5 | 2 | −3 | −3 | −11 |
| 0.099 | 7 | 4 | 3 | 3 | −2 | 4 | 3 | 2 | −5 |
| 0.033 | −5 | −3 | −4 | 1 | −7 | −4 | −4 | −3 | −8 |
| 0.011 | 3 | −2 | 1 | −1 | −5 | −0 | −1 | −7 | −2 |
| 0.004 | 3 | 2 | 1 | −5 | −3 | −4 | 0 | 4 | −5 |
| 0.001 | 6 | 6 | 2 | 3 | −1 | 4 | 3 | 2 | 3 |
| 0 | – | 10 | −0 | 5 | −3 | 0 | 1 | −3 | 1 |

FIG.24C

**Dose Matrix|NB-1**

Inhibition (%)

| Compound B (μM) \ Compound A2 (μM) | 0 | 1.36E−4 | 4.08E−4 | 0.001 | 0.004 | 0.011 | 0.033 | 0.099 | 0.298 |
|---|---|---|---|---|---|---|---|---|---|
| 2.679 | 3 | 9 | −0 | 10 | 11 | 19 | 27 | 59 | 67 |
| 0.893 | 2 | −4 | −1 | −2 | 5 | 10 | 16 | 53 | 68 |
| 0.298 | −4 | −4 | −9 | 4 | 6 | 8 | 20 | 52 | 63 |
| 0.099 | −2 | −2 | −4 | −4 | 5 | 1 | 18 | 51 | 63 |
| 0.033 | −5 | −2 | −7 | −1 | 4 | 5 | 10 | 49 | 62 |
| 0.011 | 0 | −4 | −6 | 1 | 0 | 9 | 5 | 51 | 64 |
| 0.004 | 3 | −1 | 2 | −3 | 1 | 9 | 8 | 49 | 64 |
| 0.001 | −4 | −7 | −4 | −1 | 1 | 6 | 11 | 47 | 63 |
| 0 | − | −8 | −5 | 2 | 2 | 4 | 7 | 49 | 61 |

**Loewe Excess Matrix|NB-1**

Inhibition (%)

| Compound B (μM) \ Compound A2 (μM) | 0 | 1.36E−4 | 4.08E−4 | 0.001 | 0.004 | 0.011 | 0.033 | 0.099 | 0.298 |
|---|---|---|---|---|---|---|---|---|---|
| 2.679 | 3 | 9 | −0 | 9 | 11 | 19 | 20 | 10 | 6 |
| 0.893 | 2 | −4 | −1 | −2 | 5 | 10 | 9 | 4 | 7 |
| 0.298 | −4 | −4 | −9 | 4 | 6 | 8 | 13 | 2 | 2 |
| 0.099 | −2 | −2 | −4 | −4 | 5 | 1 | 11 | 2 | 2 |
| 0.033 | −5 | −2 | −7 | −2 | 4 | 5 | 3 | 0 | 1 |
| 0.011 | 0 | −4 | −6 | 1 | −0 | 9 | −2 | 2 | 2 |
| 0.004 | 3 | −1 | 2 | −3 | 1 | 8 | 0 | −0 | 2 |
| 0.001 | −4 | −7 | −4 | −1 | 1 | 6 | 4 | −3 | 2 |
| 0 | − | −8 | −5 | 2 | 2 | 4 | −0 | 0 | −0 |

FIG.24D

Dose Matrix|SH-SY5Y

Inhibition (%)

| Compound B (μM) \ Compound A1 (μM) | 0 | 0.001 | 0.004 | 0.011 | 0.033 | 0.099 | 0.298 | 0.893 | 2.679 |
|---|---|---|---|---|---|---|---|---|---|
| 2.679 | 28 | 31 | 27 | 13 | 18 | 10 | 15 | 17 | 52 |
| 0.893 | −5 | 15 | −4 | −5 | 6 | 19 | 3 | 6 | 28 |
| 0.298 | −3 | −7 | −11 | 6 | −13 | 4 | 12 | 5 | 35 |
| 0.099 | 2 | −2 | −16 | −13 | 10 | 16 | −10 | 1 | 39 |
| 0.033 | 5 | −12 | −2 | −5 | −5 | −20 | −10 | −10 | 27 |
| 0.011 | −29 | −10 | 6 | −11 | −10 | −27 | 2 | −14 | 21 |
| 0.004 | −2 | 8 | −35 | −18 | −1 | −6 | −4 | −0 | 37 |
| 0.001 | −1 | −20 | −11 | 10 | −4 | 12 | 3 | 10 | 42 |
| 0 | – | 3 | −1 | 6 | 3 | 0 | 9 | −6 | 48 |

Loewe Excess Matrix|SH-SY5Y

Inhibition (%)

| Compound B (μM) \ Compound A1 (μM) | 0 | 0.001 | 0.004 | 0.011 | 0.033 | 0.099 | 0.298 | 0.893 | 2.679 |
|---|---|---|---|---|---|---|---|---|---|
| 2.679 | 0 | 2 | −1 | −15 | −11 | −19 | −13 | −12 | 4 |
| 0.893 | −5 | 15 | −4 | −5 | 6 | 19 | 3 | −0 | −20 |
| 0.298 | −3 | −7 | −11 | 6 | −13 | 4 | 12 | 5 | −13 |
| 0.099 | 2 | −2 | −16 | −13 | 10 | 16 | −10 | 1 | −9 |
| 0.033 | 5 | −12 | −2 | −5 | −5 | −20 | −10 | −10 | −21 |
| 0.011 | −29 | −10 | 6 | −11 | −10 | −27 | 2 | −14 | −27 |
| 0.004 | −2 | 8 | −35 | −18 | −1 | −6 | −4 | −0 | −12 |
| 0.001 | −1 | −20 | −11 | 10 | −4 | 12 | 3 | 10 | −6 |
| 0 | – | 3 | −1 | 6 | 3 | 0 | 9 | −6 | −0 |

FIG.24E

Dose Matrix|SH-SY5Y

| Compound B (μM) \ Compound A2 (μM) | 0 | 0.001 | 0.004 | 0.011 | 0.033 | 0.099 | 0.298 | 0.893 | 2.679 |
|---|---|---|---|---|---|---|---|---|---|
| 2.679 | 21 | 12 | −0 | 10 | −5 | 20 | 28 | 34 | 42 |
| 0.893 | −12 | −13 | 1 | 1 | 16 | −3 | 1 | 17 | 44 |
| 0.298 | −1 | −15 | −15 | −2 | 2 | 8 | 4 | 32 | 32 |
| 0.099 | −11 | −13 | −21 | −27 | 16 | −22 | −9 | 22 | 16 |
| 0.033 | −7 | −4 | −14 | −7 | −12 | −8 | 0 | 11 | 20 |
| 0.011 | −11 | −11 | −14 | −16 | 9 | −5 | 11 | 12 | 10 |
| 0.004 | −16 | −17 | −11 | −41 | −34 | 13 | 9 | −10 | 15 |
| 0.001 | −34 | −38 | −27 | −19 | −24 | −1 | −10 | −0 | 25 |
| 0 | − | −16 | −22 | −4 | 0 | −5 | 8 | 15 | 13 |

Inhibition (%)

Loewe Excess Matrix|SH-SY5Y

| Compound B (μM) \ Compound A2 (μM) | 0 | 0.001 | 0.004 | 0.011 | 0.033 | 0.099 | 0.298 | 0.893 | 2.679 |
|---|---|---|---|---|---|---|---|---|---|
| 2.679 | −0 | −9 | −21 | −11 | −26 | −1 | 7 | 13 | 21 |
| 0.893 | −12 | −13 | 1 | 1 | 16 | −4 | −13 | 3 | 31 |
| 0.298 | −1 | −15 | −15 | −2 | 2 | 8 | −9 | 19 | 19 |
| 0.099 | −11 | −13 | −21 | −27 | 16 | −22 | −19 | 9 | 2 |
| 0.033 | −7 | −4 | −14 | −7 | −12 | −8 | −8 | −3 | 6 |
| 0.011 | −11 | −11 | −14 | −16 | 9 | −5 | 3 | −2 | −3 |
| 0.004 | −16 | −17 | −11 | −41 | −34 | 13 | 1 | −23 | 1 |
| 0.001 | −34 | −38 | −27 | −19 | −24 | −1 | −17 | −14 | 11 |
| 0 | − | −16 | −22 | −4 | 0 | −5 | 0 | 1 | −1 |

Inhibition (%)

FIG.24F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008073687 A1
- WO 2007140222 A
- WO 2010020675 A
- WO 2011101409 A


### Non-patent literature cited in the description


- **FISCHER, P. M.** *Curr. Opin. Drug Discovery Dev.,* 2001, vol. 4, 623-634
- **BONVINI et al.** *Haematologica,* 2009, vol. 84 (7), 944-955
- **LEHÁR J ; KRUEGER AS ; AVERY W et al.** Synergistic drug combinations tend to improve therapeutically relevant selectivity. *Nat Biotechnol.,* 2009, vol. 27, 659-66
- *Trends Pharmacol Sci,* vol. 4, 450-454
- Neuroblastoma. **THIELE, C. J.** J. Human Cell Culture. Kluwer Academic Publishers, 1998, vol. 1, 21-53
- Neuroblastoma. **THIELE CJ.** J. Human Cell Culture. Kluwer Academic Publishers, 1998, vol. 1, 21-53
- **BOYD, M. R. ; PAULL, K. D. ; RUBINSTEIN, L. R.** In Cytotoxic Anticancer Drugs: Models and Concepts for Drug Discovery and Development. Kluwer Academic: Hingham, 1992, 11-34
- **MONKS, A. ; SCUDIERO, D. A. ; SKEHAN, P. ; SHOEMAKER, R. H. ; PAULL, K. D. ; VISTICA, D. T. ; HOSE, C. ; LANGLEY, J. ; CRONICE, P. ; VAIGRO-WOLF, M.** *JNCI, J. Natl. Cancer Inst.,* 1991, vol. 83, 757-766
- **MOSSE et al.** Identification of ALK as a major familial neuroblastoma predisposition gent. *Nature,* 2008, vol. 455